# EUROPEAN PATENT APPLICATION

(11) **EP 3 401 315 A1**
(43) Date of publication of application: **14.11.2018**
(21) Application number: 16883420.8
(22) Date of filing: 22.12.2016
(51) Int. Cl.: C07D 413/12, C07D 417/14, C07D 471/08, C07D 413/14, A61P 1/16, A61P 31/20, A61P 35/00, A61P 29/00

(54) **FXR RECEPTOR MODULATOR, PREPARATION METHOD THEREFOR, AND USES THEREOF**

(30) Priority: 06.01.2016 CN 201610011327
(71) Applicant: Guangzhou Henovcom Bioscience Co. Ltd., Guangzhou, Guangdong 510530 (CN)
(72) Inventor: ZHANG, Jiancun, Guangzhou Guangdong 510535 (CN); ZOU, Qingan, Guangzhou Guangdong 510535 (CN); CHEN, Yanwei, Guangzhou Guangdong 510535 (CN)
(74) Representative: HGF Limited
(86) International application number: PCT/CN2016/111516
(87) International publication number: WO 2017/118294

(57) **Abstract**

The present disclosure disclosed a modulator of FXR receptor and preparation and use thereof, which relates to the technical filed of medicinal chemistry. The present disclosure provides a modulator of FXR receptor having a structural formula I or a pharmaceutically acceptable salt, stereoisomer, solvate or prodrug thereof, which can combine with FXR receptor (that is NR1H4) and be acted as a FXR agonist or a partial agonist for preventing and treating the disease mediated by FXR, such as chronic intrahepatic or extrahepatic cholestasis, hepatic fibrosis caused by chronic cholestasis or acute intrahepatic cholestasis, chronic hepatitis B, gallstone, hepatic carcinoma, colon cancer or intestinal inflammatory disease, etc. Specifically, for some chemical compounds, their EC₅₀ for FXR agonist activity reach below 100nM, which show an excellent FXR agonist activity and an excellent prospect to provide a new pharmaceutical selection in clinical treatment for the disease mediated by FXR.

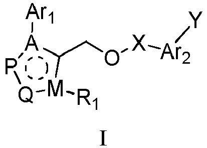

## Description

### Technical field

The present disclosure relates to the technical field of medicinal chemistry, in particular to a modulator of FXR receptor and preparation method and use thereof.

### Background of Invention

Multi-cellular organisms are dependent on advanced mechanisms for information transfer between cells and chambers. The transmitted information may be highly complex and can result in the alterations of genetic programs involved in cellular differentiation, proliferation or reproduction, etc. The signals (or hormones) for transmitting information generally refer to molecules with low molecular weight, such as peptides, fatty acids or cholesterol derivates and so on.

Many of these signals can take effects by ultimately altering the transcription of specific genes. A group of well-studied proteins that regulate the cells in response to various signals is known as nuclear receptor from transcription factor family, hereinafter commonly referred to as "NR". Members of the "NR" include steroid hormone receptors, vitamin D receptors, ecdysone receptors, cis-form and trans-form retinoic acid receptors, thyroid hormones receptors, bile acid receptors, cholesterol-derivative receptors, fatty acid receptors (and other peroxisome proliferator-activated receptors); and the so-called orphan receptors, which is a protein that has a similar structure to other identified receptors of NR group, but their ligands have not yet been identified. The orphan receptor may indicate an unknown signal pathway in the cell, or may be a nuclear receptor which can take effects without ligand activation. It has been found by CD. Mangelsdorf et al.,that transcriptional activation with some of these orphan receptors may occur in the absence of exogenous ligands and/or through signal transduction pathways stemed from cell surface. (The Nuclear receptor superfamily: the second decade", Cell 1995, 83(6), 835-839; R Evans "The nuclear receptor superfamily: arosetta stoneforphysiology", Mol. Endocrinol. 2005, 19(6), 1429-1438).

In general, there are three functional domains defined in the NR, N-terminal domain which has some certain regulatory function. DNA-binding domain(hereinafter referred to as "DBD") generally contains two zinc fingers and identifies specific hormone response elements(hereinafter referred to as HRE) of the promoter in the responding genes, wherein the amino acid residues in "DBD" can specifically bind to DNA sequence (M.Schena, "Mammalian glucocorticoid receptor derivatives enhance transcription in yeast", Science 1988, 241(4868), 965-967). Ligand binding domain (hereinafter referred to as "LBD") is located at C-terminal domain of the NR. It has been shown that the LBD can interfere with the interaction between DBD and HRE in the absence of hormone. It seems that hormone binding leads to distinct conformation of the NR, and therefore initiates such interference (A .Brzozowski et al., "Molecular basis of agonism and antagonism in the oestrogen receptor", Nature 1997, 389(6652), 753-758).

For example, steroid hormone receptors of the NR generally have effects on growth and function of specific cells by binding to cellular receptors and forming nuclear receptor-ligand complex. Subsequently, the nuclear receptor-ligand complex is interacted with hormone response elements (HRE) of the control domain in the specific gene, to alter specific genetic expression (A. Aranda, A. Pascual, "Nuclear hormone receptors and gene expression", Physiol. Rev. 2001, 81(3) 1269-1304).

Farnesoid X receptor α(hereinafter when it referred to a nuclear receptor in humans, called NR1H4) is a second type nuclear receptor of the prototype NR, and FXR in a form of heterodimer and Farnesoid X receptor together bind to promoter domain of a target gene to activate the gene(B. Forman et al., "Identification of a nuclear receptor that is activated by famesol metabolites", Cell 1995, 81(5, 687-693). Bile acids have been identified as the corresponding physiological ligands of the NR1H4 (D. Parks et al., "Bile acids: naturalligands for an orphan nuclear receptor", Science 1999, 284(5418), 1365-1368; M. Makishima et al., "Identification of a nuclear receptor for bile acids", Science 1999, 284(5418), 1362-1365), in which chenodeoxycholic acid (CDCA) has the best activity and is involved in some genetic expressions that regulates bile acid balance in body. Farnesol and derivatives thereof are together called as farnesoate, which is initially described to activate the mouse of ortholog, when bile acid concentrations are high enough, while they do not activate the receptors of humans or mouse. FXR (that is NR1H4 receptor) can be expressed in liver, intestine, colon, ovary, adrenal gland and kidney. In addition to regulating genetic expression in cells, FXR also seems to be involved in paracrine and endocrine pathways (J. Hol et al., "Definition of a novel growth factor-dependent signal cascade for the suppression of bile acid biosynthesis", Genes Dev. 2003, 17(13), 1581-91; T. Inagaki, etc., "Fibroblast growth factor 15 functions as an enterohepatic signal to regulate bile acid homeostasis", Cell Metab. 2005, 2(4), 217-225).

It has been reported in an article that FXR, through up-regulating lysosomal fate/survival factor Taco-2 in macrophages, directly affects the survival of organisms that are infected by bacterium or protozoan parasites after it is activated (P. Anand et al., "Downregulation of TACO gene transcription restricts mycobacterial entry/survival within human macrophages", FEMS Microbiol. Lett. 2005, 250(1), 137-144), which provides a basis for FXR to act as a target drug for treating intracellular bacterial or parasitic infections such as tuberculosis, leprosy, leishmaniasis, American trypanosomiasis (Chagas Disease), etc.

### SUMMARY OF INVENTION

It is therefore an objective of the present disclosure to provide a modulator of FXR receptor, which can combine with FXR receptor (that is NR1H4) and be served as an agonist or partial agonist of the FXR receptor.

In order to achieve the purposes, the present disclosure takes the following technical solution: A modulator of FXR receptor having a structural formula I a pharmaceutically acceptable salt, stereoisomer, solvate or prodrug thereof is provided: 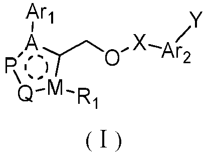 wherein:
A is selected from C or N;
P is selected from C, N or O;
Q is selected from C, N or O;
M is selected from C or N;
R₁ is selected from hydrogen, 1 to 5 R₂ substituted or unsubstituted C₁∼C₆ alkyl, 1 to 5 R₂ substituted or unsubstituted C₂∼C₆ alkenyl, 1 to 5 R₂ substituted or unsubstituted C2∼C6 alkynyl, 1 to 5 R₂ substituted or unsubstituted C₃∼C₆ cycloalkyl, 1∼5 R₂ substituted or unsubstituted heterocyclyl, 1 to 5 R₂ subastituted or unsubstituted aryl, or 1 to 5 R₂ substituted or unsubstituted heteroaryl;
Ar₁ is selected from 1 to 5 R₆ substituted or unsubstitutedl C₅∼C₁₀ aryl, or 1 to 5 R₆ substituted or unsubstituted C₅∼C₁₀ heteroaryl;
X is selected from one of the following groups: 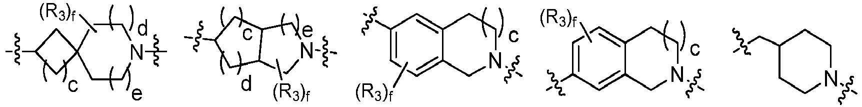 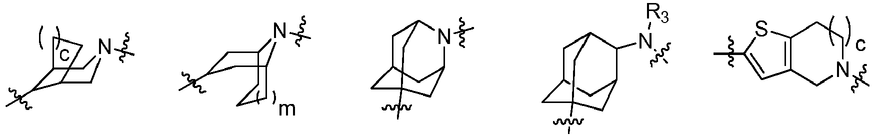 wherein:
   c is selected from 0, 1, 2, or 3;
   d is selected from 0, 1, 2, or 3;
   e is selected from 0, 1, 2, or 3;
   f is selected from 0, 1, 2, or 3;
   m is selected from 1 or 2;
   each R₃ is independently selected from hydrogen, halogen, 1 to 5 R₇ substituted or unsubstituted C₁∼C₃ alkyl, 1 to 5 R₇ substituted or unsubstituted C₁∼C₃ alkoxy, or 1 to 5 R₇ substituted or unsubstituted C₃∼C₆ cycloalkyl; and two independent substituents R₃ together with the atoms to which they attached can form a 3 to 6-membered carbocyclic, aryl, heteroaryl or heterocyclic ring;
   Ar₂ is selected from 1 to 3 R₈ substituted or unsubstituted phenyl, 1 to 3 R₈ substituted or unsubstituted naphthyl, or 1 to 3 R₈ substituted or unsubstituted monocyclic or bicyclic C₅∼C₁₀ heteroaryl;
   R₈ is selected from hydrogen, halogen, C₁∼C₄ alkyl, C₁∼C₄ alkoxy, C₃∼C₅ cycloalkyl, C₂∼C₄ alkenyl, C₂∼C₄ alkynyl, acylamino, sulfonylamino, ester group, cyano, -OCH₂F, -OCHF₂, -OCF₃, -SCF₃, or -N(CH₃)₂;
   Y is selected from COOR₄, CONR₄R₅, C(O)NHSO₂R₄, SO₂NHC(O)R₄, or tetrazole attached to Ar₂ via carbon atom;
   each of R₄ and R₅ is independently selected from hydrogen, a metal ion, 1 to 5 R₉ substituted or unsubstituted C₁∼C₆ alkyl, 1 to 5 R₉ substituted or unsubstituted C₂∼C₆ alkenyl, 1 to 5 R₉ substituted or unsubstituted C₂∼C₆ alkynyl, or 1 to 5 R₉ substituted or unsubstituted C₃∼C₆ cycloalkyl; and
   each of R₂, R₆, R₇ and R₉ is independently selected from hydrogen, halogen, cyano, acylamino, sulfonylamido, amino, ester group, nitro, C₁∼C₃ alkyl, monohalogenated or polyhalogenated C₁∼C₃ alkyl, C₁∼C₃ alkoxy, monohalogenated or polyhalogenated C₁∼C₃ alkoxy, or 1 to 3 fluorine atoms substituted or unsubstituted C₃∼C₅ cycloalkyl.

Preferably, in the above-mentioned compounds, A and M are C or N, and at least one of A and M is C; P and Q is C, N or O, and P and Q are not both be C.

In some examples, A is C; P is N; Q is O; and M is C.

In some examples, Ar₂ is selected from one of the following groups: 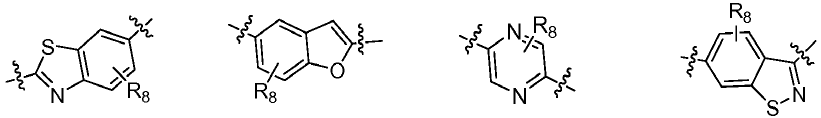 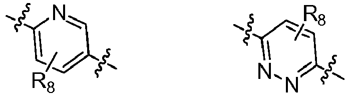 wherein,
R₈ is selected from hydrogen, halogen, C₁∼C₄ alkyl, or C₁∼C₄ alkoxy.

In some examples, X is selected from one of the following groups: 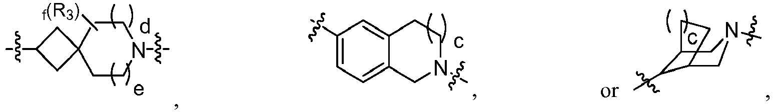 wherein, c, d, e, f, and R₃ are defined as above.

In some examples, the modulator of FXR receptor is selected from a compound having a structural formula II: 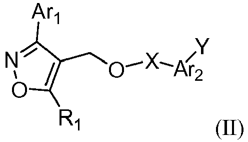 wherein,
R₁ is selected from 1 to 5 R₂ substituted or unsubstituted C₃∼C₆;
Ar₁ is selected from 1 to 5 R₆ substituted or unsubstituted C₅∼C₁₀ aryl, and preferably selected from 1 to 3 R₆ substituted or unsubstituted phenyl or 1 to 3 R₆ substituted or unsubstituted pyridyl;
X is selected from one of the following groups: 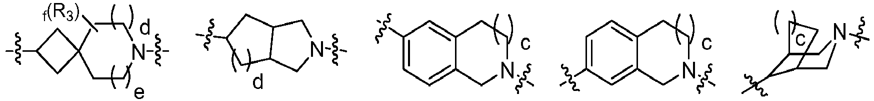 wherein,
   c is selected from 0, 1, 2, or 3;
   d is selected from 0, 1, 2;
   e is selected from 0 or 1;
   f is selected from 0 or 1;
   R₃ is independently selected from hydrogen, halogen, or 1 to 5 R₇ substituted or unsubstituted C₁∼C₃ alkyl;
   Ar₂ is selected from one of the following groups: 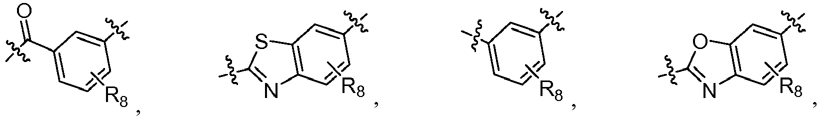 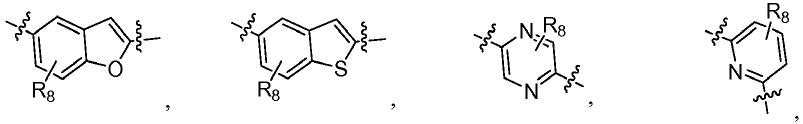 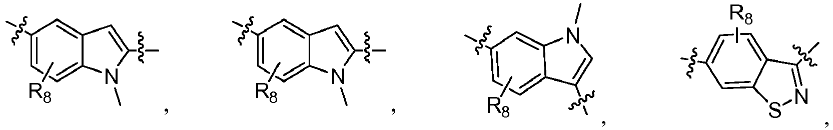 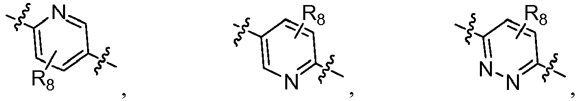 wherein,
      R₈ is selected from hydrogen, halogen, C₁∼C₄ alkyl, or C₁∼C₄ alkoxy;
      Y is selected from COOR₄ or C(O)NHSO₂R₄;
      R₄ is selected from hydrogen, a metal ion, or 1 to 5 R₉ substituted or unsubstituted C₁∼C₆ alkyl; and
      each of R₂, R₆, R₇ and R₉ is independently selected from hydrogen, halogen, cyano, nitro, C₁∼C₃ alkyl, monohalogenated or polyhalogenated C₁∼C₃ alkyl, C₁∼C₃ alkoxy, or monohalogenated or polyhalogenated C₁∼C₃ alkoxy.

In some examples, A is C;
P is N;
Q is O;
M is C;
Ar₁ is 1 to 3 R₆ substituted or unsubstituted phenyl;
R₆ is selected from halogen, 1 to 3 fluorine atoms substituted or unsubstituted C₁∼C₃ alkyl, or 1 to 3 fluorine atoms substituted or unsubstituted C₃∼C₅ cycloalkyl;
R₁ is cyclopropyl;
X is selected from: 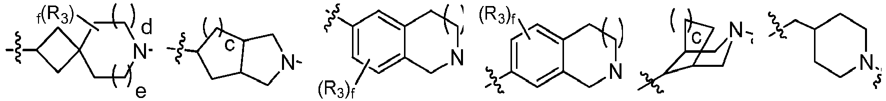 wherein,
c is 0, 1 or 2;
d is 0, 1, or 2;
e is 0, 1 or 2; and
f is 0, 1 or 2.

In some examples, the modulator is selected from one of the following compounds:

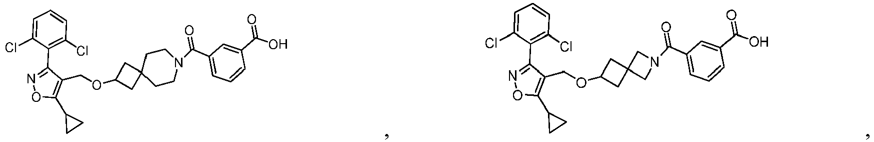

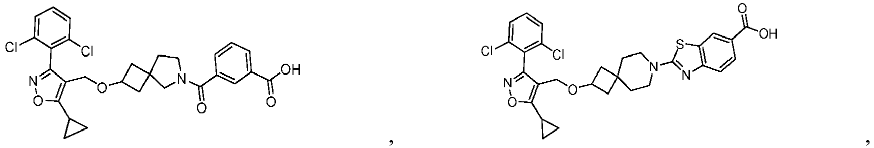

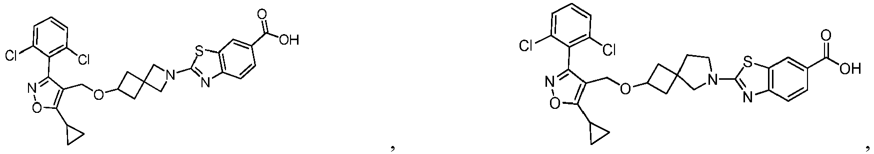

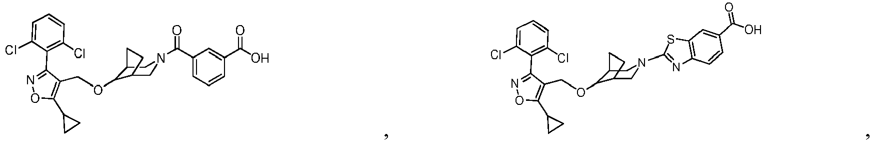

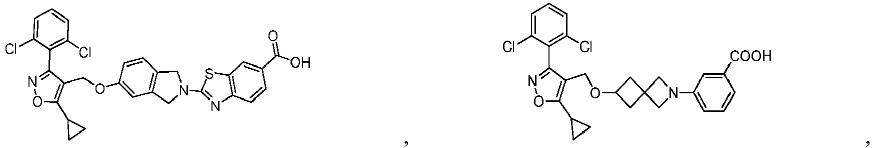

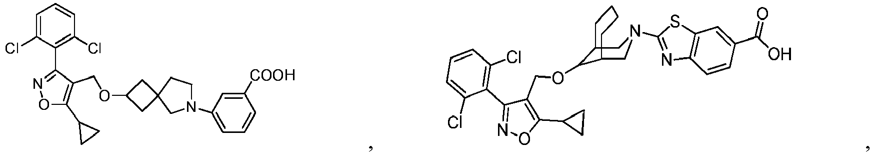

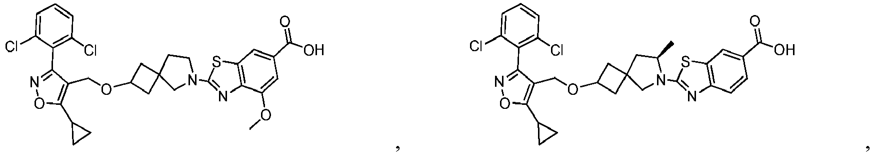

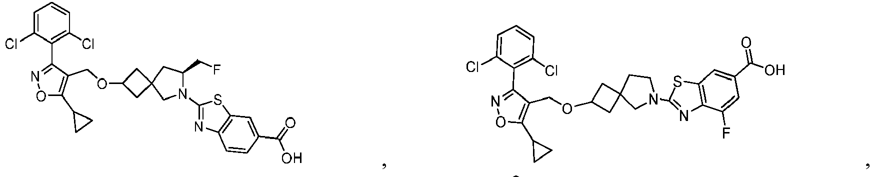

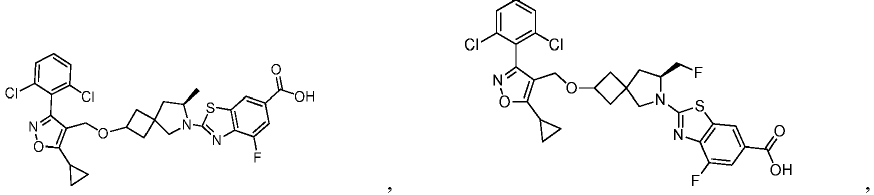

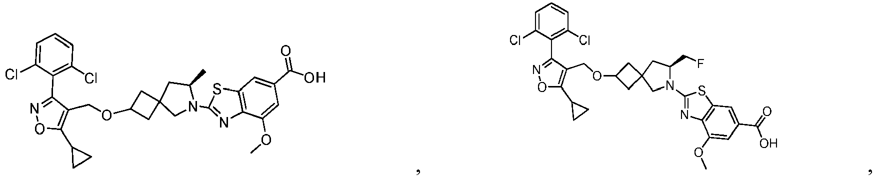

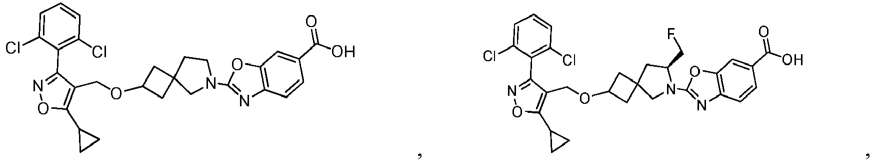

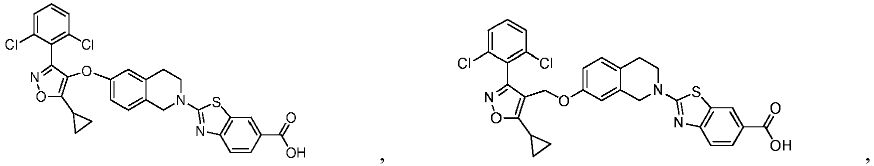

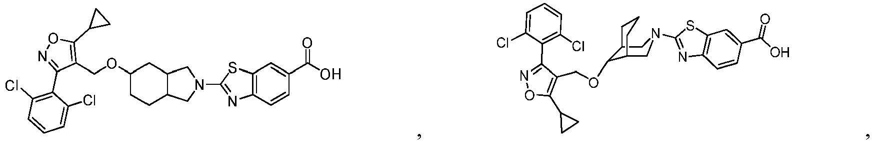

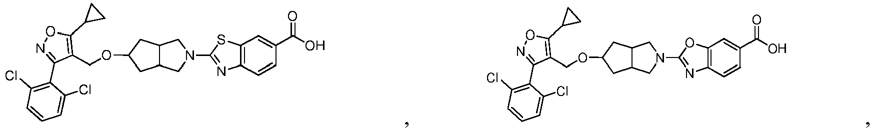

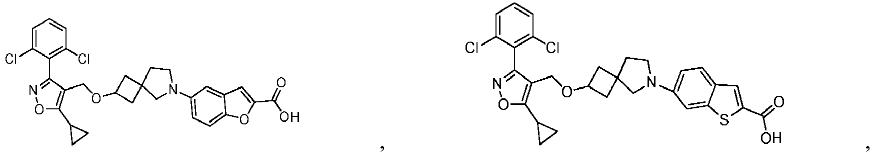

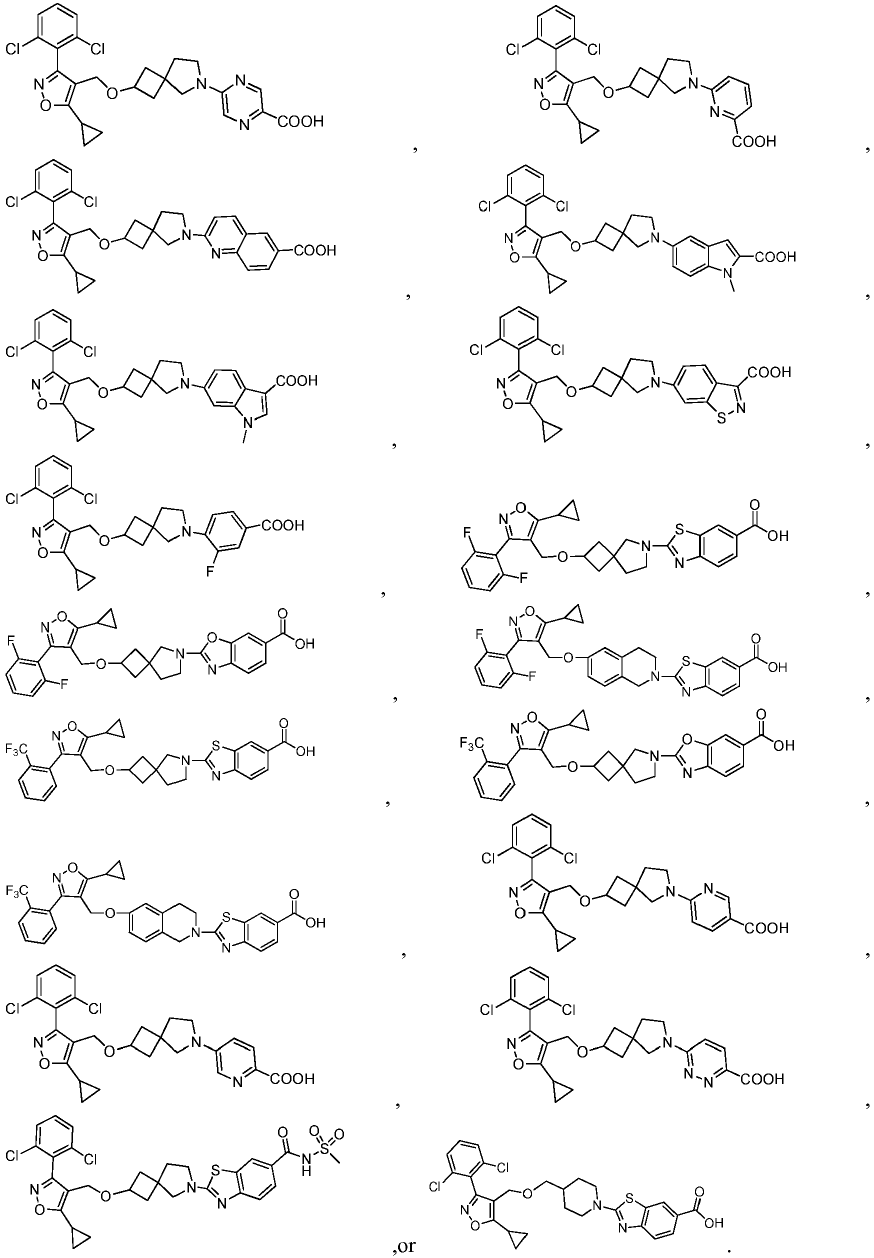

It is another objective of the present disclosure to provide a method for preparing foresaid modulator of FXR receptor, and synthetic schemes are shown as follow: 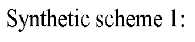 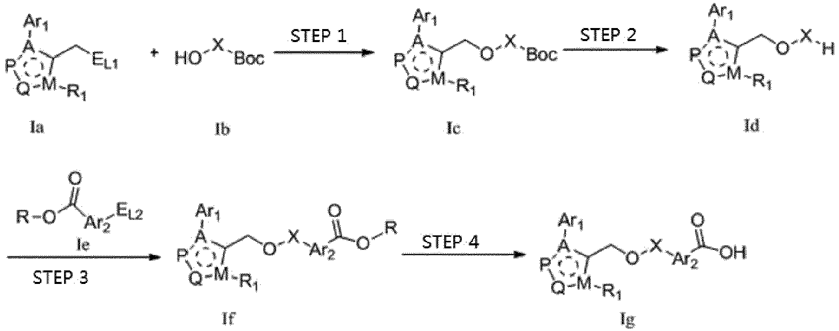 wherein, A, M, P, Q, X, R₁, Ar₁ and Ar₂ are defined as above;
E_{L1} is selected from halogen, alkylsulfonyloxy or arylsulfonyloxy;
E_{L2} is selected from halogen; and
R is selected from C₁∼C₆ alkyl.

In step 1, compound Iₐ reacts with compound I_{b} through a nucleophilic substitution reaction to form intermediate Iₑ. The nucleophilic substitution reaction takes place by using a common method in the art, for example the compound Iₐ is dissolved in an organic solvent, followed by adding phase-transfer catalyst and alkaline reagent, and the mixture reacts for 0.2 to 1 hr, then adding the compound I_{b}, reacting at a temperature ranging from room temperature to 80°C for 1 to 24 hrs. The organic solvent is tetrahydrofuran, DMF, methylbenzene, acetonitrile and the like, preferably tetrahydrofuran; the phase-transfer catalyst is 18-crown-6, terabutylammonium iodide, tetrabutylammonium hydrogen sulfate and so on, preferably 18-crown-6; the alkaline reagent is sodium hydrogen, potassium *tert*-butanol, sodium *tert*-butanol, sodium hydroxide, potassium hydroxide, sodium methanol, sodium ethanol and the like, preferably potassium *tert*-butanol or sodium *tert*-butanol.

In step 2, the protecting group Boc of intermediate I_{c} is removed, to form intermediate I_{d} with a conventional method in the art for removing Boc, for example, the intermediate Ic is dissolved in organic solvents, then adding acidic reagents, and then the mixture reacts at 0 °C∼60 °C for 0.5∼24 hrs. The organic solvent is dichloromethane, dichloroethane, chloroform, ethyl acetate, dioxane, tetrahydrofuran, etc, preferably dichloromethane or dioxane; the acidic reagent is trifluoroacetic acid, *p*-toluenesulfonic acid, methanesulfonic acid, hydrochloric acid, HCl in ethyl acetate, HCl in dioxane, preferably trifluoroacetic acid, HCl in ethyl acetate or HCl in dioxane .

In step 3, the intermediate I_{d} reacts with intermediate Iₑ through a nucleophilic substitution reaction or a metal-catalyzed coupling reaction to form intermediate I_{f}. The nucleophilic substitution reaction takes place by using a common method in the art, for example, the intermediate I_{d} is dissolved in organic solvents, followed by adding alkaline reagents and intermediate Iₑ, and the mixture reacts at a temperature ranging from room temperature to 150 °C for 1 to 24 hr. The organic solvent is tetrahydrofuran, DMF, methylbenzene, acetonitrile, propionitrile, NMP and the like, preferably DMF or acetonitrile; the alkaline reagent is sodium hydrogen, sodium hydroxide, potassium hydroxide, caesium carbonate, potassium carbonate, sodium carbonate, triethylamine, DIPEA, DBU and the like, preferably potassium carbonate, triethylamine or DIPEA; the reaction condition is preferably at 80 °C∼100 °C. The metal-catalyzed coupling reaction takes place by using a common method in the art, for example, intermediate I_{d} is dissolved in organic solvent, followed by adding palladium catalyst, phosphine ligands, alkaline reagents and intermediate Iₑ, with a reaction condition of under the protection of nitrogen or argon at 80 °C∼150 °C for 8∼48 hr. The organic solvent is methylbenzene, dimethylbenzene, dioxane, propionitrile, NMP and so on, preferably methylbenzene, dimethylbenzene or dioxane; the palladium catalyst is palladium acetate, Pd₂(db)₃, ^{3nd} Ruphos Pd, tetrakis(triphenylphosphine)palladium, etc, preferably palladium acetate or Pd₂(db)₃; the phosphine ligand is BINAP, Xantphos, tri-*tert*-butylphosphine, tri-o-tolylphosphine, etc, preferably BINAP or Xantphos; the alkaline reagent is potassium carbonate, sodium carbonate, caesium carbonate, potassium *tert*-butanol, sodium *tert*-butanol, etc, preferably caesium carbonate or potassium *tert*-butanol.

In step 4, intermediate I_{f} is hydrolyzed to obtain compound I_{g}. The hydrolysis reaction take place by using a common method in the art, for example, the intermediate I_{f} is dissolved in water and any water-miscible organic solvents, with an alkaline condition, the mixture reacts at a temperature ranging from room temperature to 100 °C for 1∼24 hrs. The organic solvent is any water-miscible organic solvents, preferably ethanol, methanol, tertrahydrofuran, dioxane, etc. The alkaline reagent is lithium hydroxide, sodium hydroxide, potassium hydroxide, potassium carbonate, caesium carbonate, sodium cabornate, etc., preferably lithium hydroxide, sodium hydroxide or potassium hydroxide.

In addition, a second synthetic scheme is provided as follows: 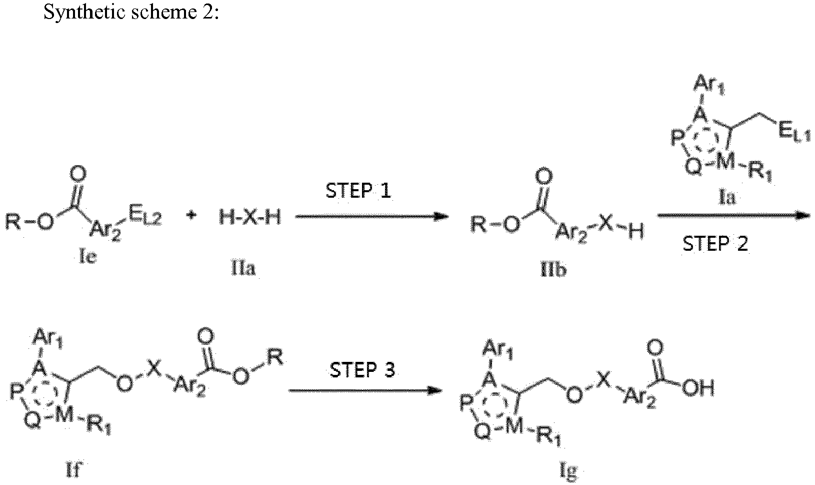 wherein, A, M, P, Q, X, R₁, Aᵣ₁ and Ar₂ are defined as above;
E_{L1} is selected from halogen, alkylsulfonyloxy or arylsulfonyloxy;
E_{L2} is selected from halogen; and
R is selected from C₁∼C₆ alkyl.

In step 1, intermediate Iₑ reacts with intermediate IIₐ through a nucleophilic substitution reaction or a metal-catalyzed coupling reaction to obtain intermediate II_{b}. The nucleophilic substitution reaction takes place by using a common method in the art, for example, the intermediate Iₑ is dissolved in organic solvents, followed by adding alkaline reagents and intermediate IIₐ, and the mixture reacts at a temperature ranging from room temperature to 150 °C for 1 to 24 hrs. The organic solvent is tetrahydrofuran, DMF, methylbenzene, acetonitrile, propionitrile, NMP, etc, preferably DMF or acetonitrile; the alkaline reagent is sodium hydrogen, sodium hydroxide, potassium hydroxide, caesium carbonate, potassium carbonate, sodium carbonate, triethylamine, DIPEA, DBU, etc, preferably potassium carbonate, triethylamine or DIPEA; the reaction condition is preferably at 80 °C∼100 °C. The metal-catalyzed coupling reaction takes place by using a common method in the art, for example, intermediate Iₐ is dissolved in organic solvents, followed by adding palladium catalyst, phosphine ligands, alkaline reagents and intermediate IIₐ, with a reaction condition of under the protection of nitrogen or argon at 80 °C∼150 °C for 8∼48 hrs. The organic solvent is methylbenzene, dimethylbenzene, dioxane, propionitrile, NMP and so on, preferably methylbenzene, dimethlbenzene or dioxane; the palladium catalyst is palladium acetate, Pd₂(db)₃, ^{3nd} Ruphos Pd, tetrakis(triphenylphosphine)palladium, etc, preferably palladium acetate or Pd₂(db)₃; the phosphine ligand is BINAP, Xantphos, tri-(*tert*-butyl)phosphine, tri-o-tolylphosphine, etc, preferably BINAP or Xantphos; the alkaline reagent is potassium carbonate, sodium carbonate, caesium carbonate, potassium *tert*-butanol, sodium *tert*-butanol, etc, preferably caesium carbonate or potassium tert-butanol.

In step 2, nucleophilic substitution reaction between the intermediate II_{b} and intermediate Iₐ takes place to obtain intermediate I_{f}. The nucleophilic substitution reaction takes place by using a common method in the art, for example, the intermediate II_{b} is dissolved in organic solvent, then alkaline reagents and intermediate Iₐ are added, and the mixture reacts at 60 °C∼120 °C for 1∼24 hrs. The organic solvent is tetrahydrofuran, DMF, methylbenzene, acetonitrile, propionitrile, etc, preferably acetonitrile and DMF; the alkaline reagent is sodium hydrogen, potassium *tert*-butanol, sodium *tert*-butanol, sodium hydroxide, potassium hydroxide, potassium carbonate, caesium carbonate, etc, preferably sodium hydrogen, caesium carbonate.

In step 3, the intermediate I_{f} is hydrolyzed to obtain compound I_{g}. The hydrolysis reaction takes place by using a common method in the art, for example, the intermediate I_{f} is dissolved in water and any water-soluble organic solvents, with an alkaline condition, and the reaction takes place at a temperature ranging from room temperature to 100 °C for 1∼24 hrs. The organic solvent is any water-soluble organic solvent, preferably ethanol, methanol, tetrahydrofuran, dioxane, etc. The alkaline reagent is lithium hydroxide, sodium hydroxide, potassium hydroxide, potassium carbonate, caesium carbonate, sodium cabornate, etc., preferably lithium hydroxide, sodium hydroxide or potassium hydroxide.

After the compound I_{g} is prepared, a derivative is prepared through a third synthetic scheme as following: 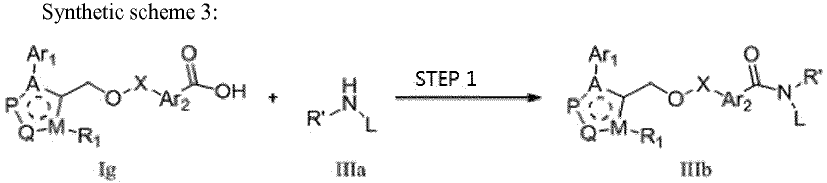 wherein, A, M, P, Q, X, R₁, Ar₁ and Ar₂ are described as above;
R' is selected from hydrogen or C₁∼C₆ alkyl;
L is selected from C₁∼C₆ alkyl, C₂∼C₆ alkenyl, C₂∼C₆ alkynyl, C₃∼C₆ cycloalkyl, C(O)R₆ or SO₂R₆; and
R₆ is selected from C₁∼C₃ alkyl, aryl or heteroaryl.

In step 1, condensation reaction between compound I_{g} and intermediate IIIₐ takes place to obtain compound III_{b}. The condensation reaction takes places by using a common method in the art, for example, the compound I_{g} is dissolved in organic solvents, condensation agents, alkaline reagents and the intermediate IIIₐ are added, and the reaction take places at a temperature ranging from room temperature to 100 °C for 1∼24 hrs. The organic solvent is dichloromethane, dichloroethane, chloroform, acetonitrile, DMF; the condensation agent is HATU, HBTU, EDC, HOBt, BOP, PyBOP, etc, preferably HATU, EDC, HOBt, PyBOP; the alkaline reagent is triethylamine, DIPEA, DBU, morpholine, pyridine and so on, preferably triethylamine and DIPEA.

It is a further objective of the present disclosure to provide a use of the modulator of FXR receptor or a pharmaceutically acceptable salt, stereoisomer, solvate or prodrugs thereof in preparation of drugs for preventing or treating a disease mediated by FXR.

In some examples, the diseases mediated by FXR comprise non-alcoholic fatty liver disease, non-alcoholic steatohepatitis, chronic intrahepatic or extrahepatic cholestasis, hepatic fibrosis caused by chronic cholestasis or acute intrahepatic cholestasis, chronic hepatitis B, gallstone, hepatic carcinoma, colon cancer, or intestinal inflammatory disease.

It is a further objective of the present disclosure to provide a pharmaceutical composition comprising the above defined modulator of FXR receptor or a pharmaceutically acceptable salt, stereoisomer, solvate or prodrug thereof and a pharmaceutically acceptable excipient or carrier.

The pharmaceutically acceptable excipient or carrier is excipient or slow release agent and so on. The pharmaceutical compositions can be in a variety of forms such as tablet, capsule, powder, sirup, solution, suspension and aerosol, etc, an also can exist in solid or liquid carrier or diluted solution. The pharmaceutical composition of the present disclosure can be stored at appropriate sterile injection or drip equipments. Furthermore, the pharmaceutical composition can also comprise odorant, flavoring agent and so on.

Compared with the prior arts, the present disclosure provides the following advantages:
The modulator of FXR receptor having a structural formula I or a pharmaceutically acceptable salt, stereoisomer, solvate or prodrug thereof of the present disclosure, can combine with FXR receptor (that is NR1H4) and be served as an agonist or a partial agonist of the FXR receptor for preventing or treating a disease mediated by FXR, such as non-alcoholic fatty liver disease, non-alcoholic steatohepatitis, chronic intrahepatic or extrahepatic cholestasis , hepatic fibrosis caused by chronic cholestasis or acute intrahepatic cholestasis, chronic hepatitis B, gallstone, hepatic carcinoma, colon cancer or intestinal inflammatory disease.

Particularly, for some compounds, their EC50 for the FXR agonist activity reach below 100nM, which show a prominent FXR activity and an excellent prospect to provide a new pharmaceutical selection in clinical treatment for the disease mediated by FXR.

### DETAILED DESCRIPTION

The term "alkyl" herein refers to saturated chained alkyl group, and "chained alkyl" refers to linear or branched alkyl group, for example, C₁∼C₆ alkyl group refers to saturated linear or branched alkyl group having 1∼6 carbon atoms, and examples of liner alkyl include but are not limited to ethyl, n-propyl, etc, and examples of branched alkyl include but are not limited to isopropyl, *tert*-butyl, etc; the term "cycloalkyl" denotes an alkyl group having a cyclic structure, for example, C₃∼C₄ cycloalkyl is an alkyl group having a cyclic structure with 3∼4 carbon atoms, and examples of cycloalkyl include but are not limited to cyclopropyl, cyclobutyl, methyl-substituted cyclopropyl, etc. The term " alkenyl" refers to unsaturated chained alkyl group, for example, C₂∼C₆ alkenyl group denotes linear or branched alkenyl group having a double bond with 2∼6 carbon atoms, and examples of alkenyl include but are not limited to vinyl, propylene, butenyl, isobutylene, pentenyl, hexenyl, etc.

The term "heterocycle" represents a saturated single-ring compound having a 5∼7-membered ring with 1∼4 heteroatoms selected from the group consisted of N, O, S. Examples of heterocycle include but are not limited to tetrahydrofuran, tetrahydropyrrole, piperidine, piperazine, morpholine, etc.

The term "aromatic heterocycle" refers to a single-ring compound having a 5∼6-membered ring with 1∼4 heteroatoms(selected from N, O, S) and obeying the Hückel's Rule. Examples of aromatic heterocycle include but are not limited to pyridine ring, pyrimidine ring, pyridazine ring, pyrazine ring, furan ring, thiophene ring, thiazole ring, oxazole ring, isoxazole ring, isothiazole ring, imidazole ring, pyrazole ring, triazole ring, tetraazole ring, etc.

The term "alkoxy" represents straight or branched alkyl with an oxygen atom at its end, and examples include but are not limited to methoxy, ethoxy, n-propoxy, isopropoxy, n-butoxy, etc.

The term "substitution" means that a hydrogen atom in a specific structure is replaced by a specified substituent. The substitution on alkyl or cycloalkyl herein can take place at any carbon atoms where the number of substituent groups fails to reach saturation, if no specific carbon atom is specified, When multiple substituents are selected from the same series, they may be the same or not. The substitution on phenyl ring, aromatic heterocycle or heterocycle hereinafter can take place at any position where it is not substituted by other atoms except hydrogen atom. When multiple substituents are selected from the same series, they may be the same or not.

The term "solvate" herein refers to a molecular complex comprising a chemical compound of the present disclosure and one or more pharmaceutically acceptable solvents (such as ethanol) in chemometrics ratio. If the solvent is water, the term "aqueous compound" is used.

In formula I, a dotted circle shows a double bond is in an unsure position of a five-membered ring, and the whole ring forms a conjugated system.

In general formula, ring structures have substituent groups at the position where a single line pointing in a direction from the outer of a ring structure towards the inner of the ring means that the ring has a substituent with an unsure position.

The present disclosure includes the compounds of formula I-II in a free state and also includes pharmaceutically acceptable salt, stereoisomer, solvate and prodrug thereof. The pharmaceutically acceptable salt of the present disclosure, through common chemical synthetic methods, can be synthesized from the compounds of the present disclosure comprising alkaline or acidic parts. In general, , the salt of alkaline compound is synthesized through ion exchange chromatography or through a reaction between free alkaline with inorganic or organic acids, which are in equivalent or excess chemometry for preparing the required salts in suitable solvents or combinations thereof. Similarly, the salt of acidic compound is prepared through a reaction with a suitable inorganic or organic alkaline.

Therefore, the pharmacologically acceptable salt of the present disclosure includes conventional non-toxic salt of the present disclosure synthesized by the reaction between the alkaline compounds of the present disclosure and inorganic or organic acids. For example, the conventional non-toxic salt compounds include the salt prepared from inorganic acids, such as hydrochloric acid, hydrobromic acid, sulfuric acid, sulfamic acid, phosphoric acid, nitric acid and so on, and also include the salt prepared from organic acids, such as acetic acid, propionic acid, succinic acid, glycolic acid, stearic acid, lactic acid, malic acid, tartaric acid, citric acid, ascorbic acid, pamoic acid, maleic acid, hydroxy maleic acid, benzene acetic acid, glutamic acid, benzoic acid, salicylic acid, p-aminobenzene sulfonic acid, p-acetoxy-benzoic acid, fumaric acid, toluenesulfonic acid, methanesulfonic acid, 1,2-ethanedisulfonic acid, oxalic acid, hydroxyethanesulphonic acid and trifluoroacetic acid, etc.

If the compound of the present disclosure is an acidic compound, an appropriate "pharmacologically acceptable salt" means a salt prepared by a pharmacologically acceptable non-toxic alkaline comprising inorganic or organic alkaline. The salts prepared by inorganic alkaline include aluminum salt, ammonium salt, calcium salt, cupric salt, ferric salt, ferrous salt, lithium salt, magnesium salt, manganese salt, manganous salt, potassium salt, sodium salt, zinc salt, etc, and preferably ammonium salt, calcium salt, magnesium salt, potassium salt and sodium salt. The salt prepared by organic non-toxic alkaline, includes primary amine, secondary amine, and tertiary amine salt, substituted amines comprising natural- existing substituted amines, cyclic amines and alkaline ion exchange resin such as arginine, trimethylglycine, caffeine, choline, N,N'-bis(phenylmethyl)-1,2-ethanediamine, diethylamine, 2-diethylaminoethanol, 2-dimethylaminoethanol, aminoethanol, ethanolamino, ethylenediamine , N-ethylmorpholine, N-ethyl piperidine, glucosamine, glucosamine, histidine, hydroxycobalamine, isopropylamine, lysine, methyl-glucosamine, morpholine, piperazine, piperidine, piperidine, polyamine resin, procaine, purine, theobromine, triethylamine and trimethylamine, tripropylamine, tromethamine, etc.

In addition to the conventional methods disclosed in the literatures or exemplified in the experimental procedures, the compounds of the present disclosure can be synthesized by the approaches described in the following examples. The compounds and synthetic approaches described in the present disclosure can be better understood in combination with the synthetic schemes described below. All parameters and other explanations of the examples are based on quality unless otherwise stated. The filler used for column chromatography separation process is silica gel unless otherwise stated. The experimental approaches in the following examples which do not specify the specific conditions are usually carried out according to conventional conditions or conditions recommended by the manufacturers. The synthetic schemes describe methods for preparing compounds of the present disclosure, which are merely used for explaining the examples and are not limited the scope of the disclosure.

In the following examples, abbreviations are defined as follows:
DMF: N,N-Dimethylformamide
NCS: N-Chlorosuccinimide
HATU: 2-(7-Aza-1H-benzotriazole-1-yl)-1,1,3 ,3 -tetramethyluronium hexafluorophosphate
PCC: Pyridinium Chlorochromate
THF: Tetrahydrofuran
NMP: N-Methyl-2-pyrrolidone
Boc: *tert*-Butyloxycarbonyl
DIPEA: N,N-diisopropylethylamine
DBU: 1,8-Diazabicyclo[5.4.0]undec-7-ene
3nd Ruphos Pd:
   2-Dicyclohexylphosphino-2',6'-diisopropoxy-1,1'-biphenyl)[2-(2'-amino-1,1'-biphenyl)]palla dium(II) methanesulfonate
Pd2(dba)3: Tris(dibenzylideneacetone)dipalladium
BINAP: 2,2'-bis(diphenylphosphino)-1,1'-binaphthyl
Xantphos: 4,5-Bis(diphenylphosphino)-9,9-dimethylxanthene

### Example 1

### 3-(2-((5-cyclopropyl-3-(2,6-dichlorophenyl)isoxazol-4-yl)methoxy)-7-azaspiro[3.5]nonan-7-yl)be nzoic acid is prepared.

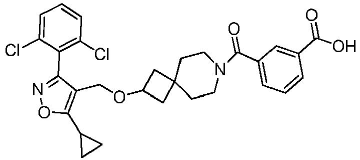

The synthetic scheme is performed as the following steps:

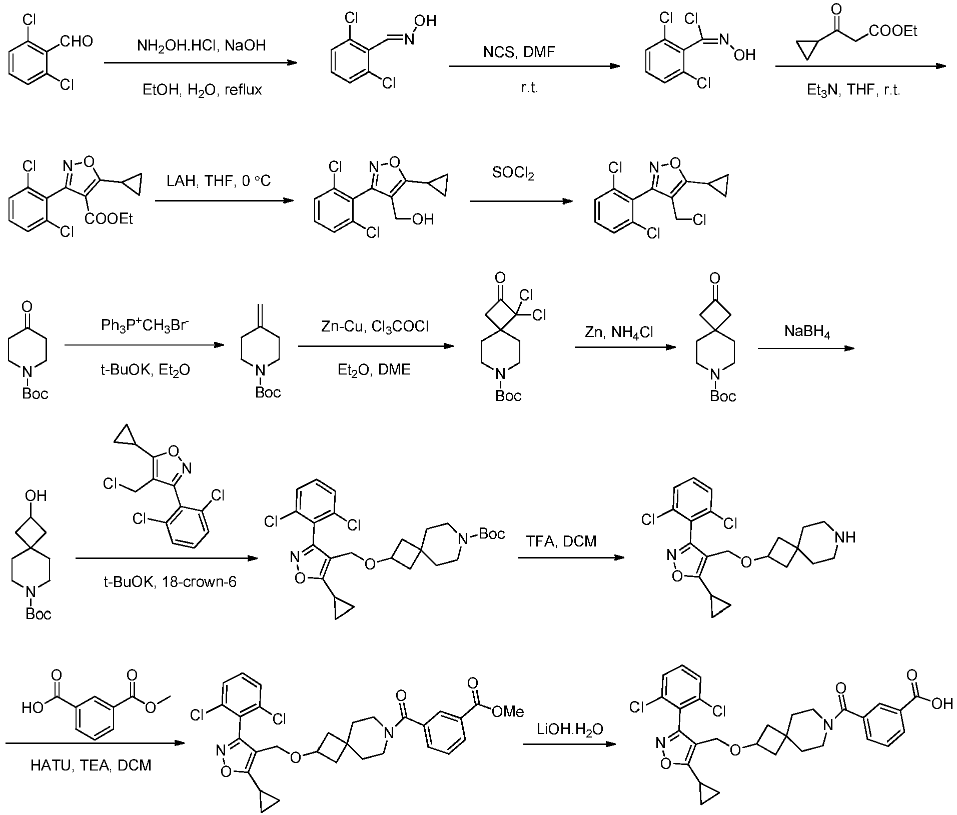

### (1) Preparation of 2,6-dichlorobenzaldehyde oxime.

2,6-Dichlorobenzaldehyde (5.0 g, 28.6 mmol) is dissolved in anhydrous ethanol (45 ml), and the mixture is stirred at room temperature, followed by adding NH₂OH•HCl (2.3 g, 33.1 mmol), NaOH (1.3 g, 32.5 mmol) and water (20 mL) with overnight reflux in an oil bath at 90 °C.When the reaction mixture is cooled to room temperature, the mixture is rotated to remove solvents, followed by adding water (100 mL), and then it is extracted with ethyl acetate (2 × 100 mL), then the organic solution is combined and washed with saturated salt water (2 × 100 mL), and then is dried with anhydrous Na₂SO₄, then filtered and rotated to dryness, after that, adding petroleum ether (100 mL), and stirring well, finally the product is filtered and dried, so that a white solid, that is 2,6-dichlorobenzaldehyde oxiame, is obtained with a yield of 64%.

### (2) Preparation of 2,6-dichloro-N-hydroxybenzimidoyl chloride.

2,6-Dichlorobenzaldehyde oxime (4.7 g, 24.7 mmol) is dissolved in DMF (40 mL), followed by adding NCS (3.3 g, 24.7 mmol), and the mixture is stirred at room temperature for 1 hr. After that, water (100 mL) is poured into the reaction mixture, and then the mixture is extracted with diethyl ether (2 × 150 mL), the organic solution is combined and washed with saturated salt water (3 × 100 mL), followed by adding anhydrous Na₂SO₄ for drying , and subsequently the resulted product is filtered and rotated to dryness. Finally, 5.5 g of yellow oily matter, 2,6-dichloro-N-hydroxybenzimidoyl chloride is obtained and it is directly used in the next step without purification.

### (3) Preparation of ethyl 5-cyclopropyl-3-(2,6-dichlorophenyl)isoxazole-4-carboxylate.

2,6-Dichloro-N-hydroxybenzimidoyl chloride (4.8 g, 21.1 mmol) is dissolved in Et₃N (40 mL), followed by successively adding ethyl 3-cyclopropyl-3-oxo-propionate (6.3 g, 40.6 mmol)and THF (20 mL), and the mixture is stirred overnight at room temperature. After that, the reaction mixture is poured into water (150 mL) and extracted with ethyl acetate (2 × 150 mL), and then the organic solution is washed with saturated salt water (2 × 150 mL), added anhydrous Na₂SO₄ for drying, then the resulted product is filtered, rotated to dryness and purified by column chromatography (eluent: PE / EA = 100 / 1). 4.2 g of white solid, ethyl 5-cyclopropyl-3-(2,6-dichlorophenyl)isoxazole-4-carboxylate is obtained with a yield of 60%.

### (4) Preparation of (5-cyclopropyl-3-(2,6-dichlorophenyl)isoxazol-4-yl)methanol.

Lithium aluminum hydride (750 mg, 19.8 mmol) is dissolved in anhydrous THF (40 mL) in a 250 ml two-neck flask at 0 °C under the protection of nitrogen, and the reaction mixture is stirred well, followed by adding ethyl 5-cyclopropyl-3-(2,6-dichlorophenyl)isoxazole-4-carboxylate(4.15 g, 12.7 mmol) dissolved in the anhydrous THF (40 mL), then the mixture continues to react at 0 °C for 2 hr. Successively, water (0.8 mL), 15% NaOH solvent (0.8 mL) and water (2.4 mL) are added dropwise, then the temperature is increased to room temperature, and then the mixture is stirred for 15 min, and filtered with siliceousearth, washed with ethyl acetate, and then it is rotated to dryness and purified by column chromatography (eluent: PE / EA = 10 / 1), so as to obtain 2.8 g of white solid, (5-cyclopropyl-3-(2,6-dichlorophenyl)isoxazol-4-yl)methanol, with a yield of 78%.

### (5) Preparation of 4-(chloromethyl)-5-cyclopropyl-3-(2,6-dichlorophenyl)isoxazole.

(5-cyclopropyl-3-(2,6-dichlorophenyl)isoxazol-4-yl)methanol (180 mg, 0.63 mmol) is dissolved in DCM (5 mL), cooled in a ice bath, followed by adding thionyl chloride (138 µL,1.90 mmol) and DMF (3 drops), reacting at room temperature for 1hr, after that, the mixture is rotated to dryness and the product is purified by column chromatography, so as to obtain 173 mg of light yellow oily matter, that is 4-(chloromethyl)-5-cyclopropyl-3-(2,6-dichlorophenyl)isoxazole, with a yield of 91%.

### (6) Preparation of tert-butyl 4-methylenepiperidine-1-carboxylate.

Methyltriphenylphosp bromide (14.7 g, 40.5 mmol) and potassium *tert*-butoxide (4.38 g, 39 mmol) are added to diethyl ether (90 mL) at 0 °C, stirring at room temperature for 2 hrs. Subsequently, the reaction mixture is cooled in an ice bath, and *tert*-butyl 4-oxopiperidine-1-carboxylate (5.98 g, 30 mmol) is dissolved in diethyl ether (30 mL) and transferred to the above reaction mixture dropwise, and then the mixture liquid reacts overnight at room temperature, filtered, rotated to dryness and purified by column chromatography (eluent: petroleum ether : ethyl acetate = 50 : 1), so as to obtain 5.2 g of colorless oily liquid, that is, *tert*-butyl 4-methylenepiperidine-1-carboxylate, with a yield of 88%.
Spectrum is: ¹H NMR(400 MHz, CDCl₃)*δ*: 4.75(s, 2H), 3.43(t, *J=* 5.6 Hz, 4H), 2.19(t, *J=* 5.6 Hz, 4H), 1.48(s, 9H).

### (7) Preparation of tert-butyl 1,2-dichloro-2-oxo-7-azaspiro[3.5]nonan-7-carboxylate.

*Tert*-butyl 4-methylenepiperidine-1-carboxylate (1 g, 5.07 mmol) is dissolved in diethyl ether (20 mL), followed by adding zinc-copper couple (4.4 g, 34.5 mmol) under the protection of nitrogen at 10 °C, and dripping trichloro-acetic chloride (1.84 mL, 16.5 mmol) dissolved in the DME solution (5 mL), and the reaction mixture continues to react overnight at room temperature. Subsequently, the reaction mixture is slowly poured into -10 °C saturated salt solution (30 mL), filtered with siliceousearth, extracted with ethyl acetate (20 mL × 3), washed with saturated salt solution (30 mL × 2), dried, rotated to dryness and purified by column chromatography (eluent: petroleum ether : ethyl acetate = 15 : 1), so as to obtain 880 mg of a brown oily liquid, that is *tert*-butyl 1,1-dichloro-2-oxo-7-azaspiro[3.5]nonan-7-carboxylate with a yield of 56%. The obtained product is directly used in the next step without purification.

### (8) Preparation of tert-butyl 2-oxo-7-azaspiro[3.5]nonan-7-carboxylate.

*Tert*-butyl 1,2-dichloro-2-oxo-7-azaspiro[3.5]nonan-7-carboxylate (880 mg, 2.88 mmol) is dissolved in methanol(10 mL), followed by adding saturated ammonium chloride solution (10 mL) and zinc powder (1.12 g), the reaction mixture reacts overnight at room temperature, filtered, rotated to dryness, followed by adding water (20 mL), and then the mixture is extracted with ethyl acetate (20 mL × 2), washed with saturated salt solution(20 mL × 2), the organic solution is dried with anhydrous Na₂SO₄, rotated and purified by column chromatography (eluent: PE : EA = 8 : 1), so as to obtain 490 mg of colorless oily substance, that is *tert*-butyl 2-oxo-7-azaspiro[3.5]nonan-7-carboxylate, with a yield of 72%.
Spectrum is: ¹H NMR(400 MHz, CDCl₃)*δ*: 3.43(t, J = 5.6 Hz, 4H), 2.84(s, 4H), 1.72(t, J = 5.6 Hz, 4H), 1.49(s, 9H).

### (9) Preparation of tert-butyl 2-hydroxy-7-azaspiro[3.5]nonan-7-carboxylate.

*Tert*-butyl 2-oxo-7-azaspiro[3.5]nonan-7-carboxylate(490 mg, 2.05 mmol) is dissolved in methanol (10 mL), cooled in an ice bath, followed by adding sodium borohydride (156 mg, 4.10 mmol) in batches, and the reaction mixture reacts at room temperature for 1 hr, then rotated for drying, followed by adding water (20 mL), and the liquid is extracted with ethyl acetate (20 mL × 2), washed with saturated salt solution(20 mL × 2), and then the organic solution is dried with anhydrous Na₂SO₄, rotated to dryness and purified by column chromatography (eluent: PE : EA = 3 : 1), so as to obtain 450 mg of a white solid, that is *tert*-butyl 2-hydroxy-7-azaspiro[3.5]nonan-7-carboxylate, with a yield of 91%.
Spectrum is: ¹H NMR (400 MHz, CDCl₃) *δ*: 4.35(m, 1H), 3.33(m, 4H), 2.30(m, 2H), 1.70(m, 3H), 1.53(m, 4H), 1.47(m, 9H).

### (10) Preparation of tert-butyl 2-((5-cyclopropyl-3-(2,6-dichlorophenyl)isoxazol-4-yl)methoxy)-7-azaspiro[3.5]nonan-7-carboxyl tae.

*Tert*-butyl 2-hydroxy-7-azaspiro[3.5]nonan-7-carboxylate (241 mg, 1 mmol)
is dissolved in THF (5 mL), followed by adding 18-crown-6 (317 mg, 1.2 mmol) and potassium *tert*-butoxide (135 mg, 1.2 mmol), and the reaction mixture is stirred at room temperature for 10 min, then added 4-(chloromethyl)-5-cyclopropyl-3-(2,6-dichlorophenyl)isoxazole (302 mg, 1 mmol), continuing to react overnight at room temperature. Subsequently, the solution is rotated for drying, and then water (20 mL) is added, the solution is extracted with ethyl acetate (15 m L × 2), wherein then the organic solution is dried with anhydrous Na₂SO₄, and is rotated to dryness and purified by column chromatography (PE : EA = 10 : 1), so as to obtain a light yellow slurry sample of 370 mg, that is *tert-butyl*
2-((5-cyclopropyl-3-(2,6-dichlorophenyl)isoxazol-4-yl)methoxy)-7-azaspiro[3.5]nonan-7-carboxyl tae , with a yield of 73%.
Spectrum is: ¹H NMR (400 MHz, CDCl₃) *δ*: 7.45-7.43(m, 2H), 7.34(m, 1H), 4.17(s, 2H), 3.91(m, 1H), 3.29(t, *J* = 5.6 Hz, 2H), 3.25(t, *J* = 5.6 Hz, 2H), 2.15(m, 1H), 2.00(m, 2H), 1.49-1.38(m, 15H), 1.28(m, 2H), 1.14(m, 2H).

### (11) Preparation of 4-(((7-azaspiro[3.5]nonan-2-yl)oxy)methyl)-5-cyclopropyl-3-(2,6-dichlorophenyl)isoxazole. Tert-butyl

2-((5-cyclopropyl-3-(2,6-dichlorophenyl)isoxazol-4-yl)methoxy)-7-azaspiro[3.5]nonan-7-carboxyl tae (370 mg, 0.37 mmol) is dissolved in dichloromethane (5 mL), followed by adding trifluoroacetic acid (1 mL), and the reaction mixture is stirred at room temperature for 1hr, then rotated for drying, then saturated sodium bicarbonate (15 mL) is added, and then the solution is extracted with dichloromethane (15 mL × 2), anhydrous Na₂SO₄ is added to dry the organic solution, subsequently, the resulted product is rotated to dryness, to obtain a yellow slury sample of 235 mg, that is
4-(((7-azaspiro[3.5]nonan-2-yl)oxy)methyl)-5-cyclopropyl-3-(2,6-dichlorophenyl)isoxazole, with a yield of 80%. The product is used for the next reaction without purification.

### (12) Preparing methyl 3-(2-((5-cyclopropyl-3-(2,6-dichlorophenyl)isoxazol-4-yl)methoxy)-7-azaspiro[3.5]nonan-7-carbo nyl)benzoate.

4-(((7-azaspiro[3.5]nonan-2-yl)oxy)methyl)-5-cyclopropyl-3-(2,6-dichlorophenyl)isoxazole (100 mg, 0.25 mmol) is dissolved in dichloromethane(5 mL), followed by successively adding mono-methyl isophthalate (57 mg, 0.32 mmol), triethylamine (104 µL, 0.75 mmol) and HATU (131 mg, 0.34 mmol), and the reaction mixture is stirred overnight at room temperature. Then, the reaction mixture is poured into water (15 mL) and the mixture is extracted with dichloromethane (15 mL × 2), then the organic solution is dried with anhydrous Na₂SO₄, filtered, rotated to dryness and purified by column chromatography (PE : EA = 3 : 1), so as to obtain a white solid of 95 mg, that is
3-(2-((5-cyclopropyl-3-(2,6-dichlorophenyl)isoxazol-4-yl)methoxy)-7-azaspiro[3.5]nonan-7-carbo nyl)benzoate, with a yield of 68%.
Spectrum is: ¹H NMR (400 MHz, CDCl₃) *δ*: 8.10(m, 1H), 8.05(m, 1H), 7.59(m, 1H), 7.51(t, J = 7.6 Hz, 1H), 7.45-7.43(m, 2H), 7.39-7.35(m, 1H), 4.18(s, 2H), 3.95(m, 4H), 3.63(m, 2H), 3.22(m, 2H), 2.15(m, 1H), 2.05(m, 2H), 1.61-1.44(m, 6H), 1.29(m, 2H), 1.14(m, 2H).

### (13) Preparation of 3-(2-(2-((5-cyclopropyl-3-(2,6-dichlorophenyl)isoxazol-4-yl)methoxy)-7-azaspiro[3.5]nonan-7-yl) benzoic acid.

3-(2-((5-cyclopropyl-3-(2,6-dichlorophenyl)isoxazol-4-yl)methoxy)-7-azaspiro[3.5]nonan-7-carbo nyl)benzoate (90 mg, 0.16 mmol) is dissolved in THF(2 mL), methanol (2 mL) and water (1 mL), then lithium hydroxide-monohydrates (33 mg, 0.79 mmol) is added, and the mixture reacts at room temperature for 2 hrs. The mixture is then rotated to dryness, followed by adding water(15 mL) and its pH is adjusted to approximately 2 with 1N HCl, and then the mixture is filtered, dried, so as to obtain a white solid product of 75 mg, that is
3-(2-(2-((5-cyclopropyl-3-(2,6-dichlorophenyl)isoxazol-4-yl)methoxy)-7-azaspiro[3.5]nonan-7-yl) benzoic acid, with a yield of 86%.
Spectrum is: ¹H NMR (400 MHz, CDCl₃) *δ*: 8.15(m, 1H), 8.10(m, 1H), 7.65(m, 1H), 7.54(t, *J* = 7.6 Hz, 1H), 7.45-7.43(m, 2H), 7.38-7.34(m, 1H), 4.19(s, 2H), 3.93(m, 1H), 3.64(m, 2H), 3.25(m, 2H), 2.15(m, 1H), 2.07(m, 2H), 1.55-1.45(m, 6H), 1.28(m, 2H), 1.14(m, 2H).

### Example 2 2-(2((5-cyclopropyl-3-(2,6-dichlorophenyl)isoxazol-4-yl)methoxy)-7-azaspiro[3.5]nonan-7-yl)ben zo[d]thiazole-6-carboxylic acid is prepared.

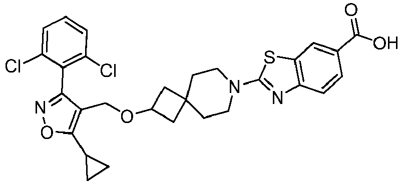

### (1) Preparation of ethyl 2-aminobenzo[d]thiazole-6-carboxylate.

Ethyl 4-aminobenzoate (4.9 g, 30.0 mmol) and potassium thiocyanate (11.6 g,120.0 mmol) are dissolved in acetic acid(180 mL), followed by dripping acetic acid solution(20 mL) with bromine (1.5 mL,30.0 mmol) at 19 °C in 20 min, then the temperature is increased to room temperature and the mixture continues to react overnight. Then, the reaction solution is poured into water (100 mL), and stirred for 10 min, then ammonium hydroxide is dripped into the mixture to regulate pH to approximately 8, then the mixture is filtered, wherein filter cake is successively washed with water and petroleum ether, and finally the product is vacuumed to dryness, so as to obtain a yellow solid of 6.7 g, that is ethyl 2-aminobenzo[*d*]thiazole-6-carboxylate, with a yield of 100%.
Spectrum is: ¹H NMR (400 MHz, DMSO) *δ*: 8.28(d, *J* = 1.6 Hz, 1H), 7.88(s ,2H), 7.82(dd, *J* = 1.6 Hz, 8.4 Hz, 1H), 7.37(d, *J* = 8.4Hz, 1H), 4.29(q, *J* = 7.2 Hz ,2H), 1.32(t, *J* = 7.2 Hz, 3H).

### (2) Preparation of ethyl 2-chlorobenzo[d]thiazole-6-carboxylate.

Cupric chloride (6.0 g, 44.6 mmol), *tert*-butyl nitrite (7.1 mL, 59.4 mmol) and acetonitrile (200 mL) are added to a 500 ml single neck bottle and the mixture is cooled to 0 °C, followed by adding ethyl 2-aminobenzo[*d*]thiazole-6-carboxylate (6.6 g, 29.7 mmol) in batches in 30 min, then the temperature is increased to room temperate, the mixture reacts overnight, then it is filtered with siliceousearth, and 1HCL (60 mL) is dripped into the filtrate, and the mixture is stirred for 10min, and forms layering, wherein the aqueous layer is extracted with ethyl acetate (100 mL), and the organic layer is combined, washed with saturated salt solution, dried with anhydrous Na₂SO₄, then filtered again, rotated to dryness and purified by column chromatography (PE:EA= 25:1), so as to obtain a light yellow of 6.2 g, that is ethyl 2-chlorobenzo[*d*]thiazole-6-carboxylate, with a yield of 86%.
Spectrum is: ¹H NMR (400 MHz, CDCl₃) *δ*: 8.54(d, *J* = 0.8 Hz, 1H), 8.20(dd, *J* = 1.6 Hz, 8.4 Hz, 1H), 8.01(d, *J* = 8.0Hz, 1H), 4.45(q, *J* = 7.2 Hz, 2H), 1.45(t, *J* = 7.2 Hz, 3H).

### (3) Preparation of ethyl 2-(2((5-cyclopropyl-3-(2,6-dichlorophenyl)isoxazol-4-yl)methoxy)-7-azaspiro[3.5]nonan-7-yl)ben zo[d]thiazole-6-carboxylate.

4-(((7-azaspiro[3.5]nonan-2-yl)oxy)methyl)-5-cyclopropyl-3-(2,6-dichlorophenyl)isoxazole(130 mg, 0.32 mmol) is dissolved in DMF(3 mL), followed by adding ethyl 2-chlorobenzo[*d*]thiazole-6-carboxylate (100 mg, 0.42 mmol) and triethylamine (89 µL, 0.65 mmol), and the reaction mixture reacts at 100 °C for 1 hr. When the reaction mixture is cooled to room temperature, the mixture is added into water (20 mL), and then is extracted with ethyl acetate (15 mL × 2), wherein the organic layer is washed with saturated salt solution (15 mL × 2), then dried with anhydrous Na₂SO₄, filtered, rotated to dryness and purified by column chromatography (PE:EA = 5:1), so as to obtain a light yellow solid of 180 mg, that is 2-(2((5-cyclopropyl-3-(2,6-dichlorophenyl)isoxazol-4-yl)methoxy)-7-azaspiro[3.5]nonan-7-yl)ben zo[*d*]thiazole-6-carboxylate, with a yield of 93%.
Spectrum is: ¹H NMR (400 MHz, CDCl₃) *δ*: 8.30(d, *J* = 1.6 Hz, 1H), 8.01(dd, *J* = 8.4Hz, 1.6Hz, 1H), 7.51(d, *J* = 8.4Hz, 1H), 7.46-7.44(m, 2H), 7.39-7.35(m, 1H), 4.39(q, *J* = 7.2 Hz, 2H), 4.20(s, 2H), 3.97(m, 1H), 3.58(t, *J* = 5.6 Hz, 2H), 3.52(t, *J* = 5.6 Hz, 2H), 2.16(m, 1H), 2.08(m, 2H), 1.65-1.54(m, 6H), 1.29(m, 2H), 1.15(m, 2H).

### (4) Preparation of 2-(2((5-cyclopropyl-3-(2,6-dichlorophenyl)isoxazol-4-yl)methoxy)-7-azaspiro[3.5]nonan-7-yl)ben zo[d]thiazole-6-carboxylic acid.

Ethyl 2-(2-((5-cyclopropyl-3-(2,6-dichlorophenyl)isoxazol-4-yl)methoxy)-7-azaspiro[3.5]nonan-7-yl)be nzo[*d*]thiazole-6-carboxylate (180 mg, 0.29 mmol) is dissolved in THF (2 mL), methanol (2 mL) and water (1 mL), followed by adding lithium hydroxide monohydrate (62 mg, 1.47 mmol), and the reaction mixture reacts overnight at room temperature, and then is rotated to dryness, and subsequently adding water (15 mL) and adjusting its pH to approximately 2 with IN HCl, the resulted product is filtered and dried, so as to obtain a white solid of 146 mg, that is 2-(2-((5-cyclopropyl-3-(2,6-dichlorophenyl)isoxazol-4-yl)methoxy)-7-azaspiro[3.5]nonan-7-yl)be nzo[*d*]thiazole-6-carboxylic acid, with a yield of 85%.
Spectrum is: ¹H NMR (400 MHz, CDCl₃) *δ*: 8.36(s, 1H), 8.05(m, 1H), 7.53(d, *J* = 8.4Hz, 1H), 7.47-7.44(m, 2H), 7.39-7.35(m, 1H), 4.20(s, 2H), 3.97(m, 1H), 3.59(m, 2H), 3.52(m, 2H), 2.16(m, 1H), 2.08(m, 2H), 1.65-1.54(m, 6H), 1.29(m, 2H), 1.15(m, 2H).

### Example 3 3-(6((5-cyclopropyl-3(2,6-dichlorophenyl)isoxazol-4-yl)methoxy)-2-azaspiro[3.3]heptan-2-carbon yl)benzoic acid is prepared.

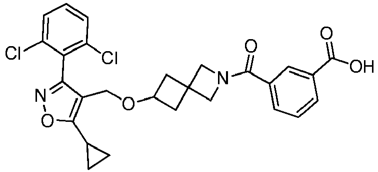

### (1) Preparation of tert-butyl 6-hydroxy-2-azaspiro[3.3]heptan-2-carboxylate.

With reference to the method for preparing *tert*-butyl
2-hydroxy-7-azaspiro[3.5]nonan-7-carboxylate of Example 1, *tert*-butyl
6-hydroxy-2-azaspiro[3.3]heptan-2-carboxylate is synthesized, and *tert*-butyl 3-oxoazetidine-1-carboxylate is used to replace *tert*-butyl 4-oxopiperidine-1-carboxylate.
Spectrum is: ¹H NMR (400 MHz, CDCl₃) *δ*: 4.20(m, 1H), 3.90(s, 2H), 3.89(s, 2H), 2.55(m, 2H), 2.09(m, 2H), 1.95(m, 1H), 1.44(s, 9H).

### (2) Preparation of 3-(6((5-cyclopropyl-3(2,6-dichlorophenyl)isoxazol-4-yl)methoxy)-2-azaspiro[3.3]heptan-2-carbon yl)benzoic acid.

With reference to the method for preparing
3-(2-((5-cyclopropyl-3(2,6-dichlorophenyl)isoxazol-4-yl)methoxy)-7-azaspiro[3.5]nonan-7-carbo nyl)benzoic acid of Example 1,
3-(6((5-cyclopropyl-3(2,6-dichlorophenyl)isoxazol-4-yl)methoxy)-2-azaspiro[3.3]heptan-2-carbon yl)benzoic acid is synthesized, and *tert*-butyl 6-hydroxy-2-azaspiro[3.3]heptan-2-carboxylate is used to replace *tert*-butyl 2-hydroxy-7-azaspiro[3.5]nonan-7-carboxylate.
Spectrum is: ¹H NMR (400 MHz, CDCl₃) *δ*: 8.31(s, 1H), 8.21(d, *J* = 8.0 Hz, 1H), 7.92(d, *J* = 7.6 Hz, 1H), 7.56(t, *J* = 7.6 Hz, 1H), 7.45-7.33(m, 3H), 4.25-4.10(m, 6H), 3.83(m, 1H), 2.38(m, 2H), 2.13(m, 1H), 1.94(m, 2H), 1.28(m, 2H), 1.14(m, 2H).

### Example 4 2-(6((5-cyclopropyl-3-(2,6-dichlorophenyl) isoxazol-4-yl)methoxy)2-azaspiro[3.3]heptan-2-yl)benzo[d]thiazole-6-carboxylic acid is prepared.

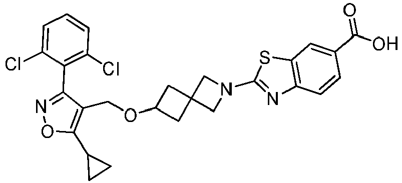

The synthetic method refers to the method used in Example 2, and *tert*-butyl
6-hydroxy-2-azaspiro[3.3]heptan-2-carboxylate is just used to replace *tert*-butyl 2-hydroxy-7-azaspiro[3.5]nonan-7-carboxylate.
Spectrum is: ¹H NMR (400 MHz, CDCl₃) *δ*: 8.38(d, *J* = 1.6 Hz, 1H), 8.08(dd, *J* = 1.6 Hz, 8.4 Hz, 1H), 7.61(d, *J* = 8.8 Hz, 1H), 7.47-7.45(m, 2H), 7.40-7.36(m, 1H), 4.20(s, 2H), 4.17(s, 2H), 4.10(s, 2H), 3.87(m, 1H), 2.42(m, 2H), 2.15(m, 1H), 2.02(m, 2H), 1.28(m, 2H), 1.15(m, 2H).

### Example 5 3-(8((5-cyclopropyl-3-(2,6-dichlorophenyl) isoxazol-4-yl)methoxy)3-azabicyclo[3.2.1]octan-3-carbonyl)benzoic acid is prepared.

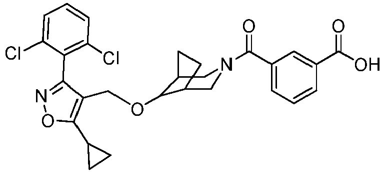

### (1) Preparation of 3-benzyl-3-azabicyclo[3.2.1]octan-8-one.

3-benzyl-3-zazbicyclo[3.2.1]octan-8-one is synthesized with respect to the method describled in WO2011025690. Benzylamine (2.68 g, 25 mmol) is dissolved in *tert*-butyl alcohol (6 mL), followed by adding paraformaldehyde (1.5 g, 50 mmol), concentrated HCl (2.37 mL, 27.5 mmol) and cyclopentanone (5.46 g, 65 mmol), the reaction mixture reacts at 80 °C for 3 hrs. Then the mixture is cooled, rotated to dryness, followed by adding acetone (30 mL), filtered, then 3.58g of while solid product is formed. Acetic acid (20 mL), concentrated HCl (1.25 mL, 13.5 mmol) and polyformaldehyde are added into a 100 mL single neck bottle and heated to 95 °C. The above prepared solid product is dissolved in acetic acid (20 mL), and is successively dripped into the above reaction solution in half hour, then the mixture continues to react for 1 hr. After that, the reaction mixture is cooled and rotated to remove the solvent, followed by adding water (30 mL) and dichloromethane (30 mL), and adjusting pH to 10 with NaOH, then the mixture is layered, wherein the aqueous layer is extracted with dichloromethane (30 mL × 2) and the organic layer is combined and dried with Na₂SO₄, then rotated to dryness and purified by column chromatography (PE:EA = 25:1), so as to obtain a colorless oily liquid of 2.21 g, that is 3-benzyl-3-azabicyclo[3.2.1]octan-8-one, with a yield of 41%.
Spectrum is: ¹H NMR (400 MHz, CDCl₃) *δ*: 7.38-7.26(m, 5H), 3.62(s, 2H), 3.00(m, 2H), 2.57(m, 2H), 2.18(m, 2H), 2.09(m, 2H), 1.88(m, 2H).

### (2) Preparation of 3-benzyl-3-azabicyclo[3.2.1]octan-8-ol.

3-benzyl-3-azabicyclo[3.2.1]octan-8-one (2.15 g, 10 mmol) is dissolved in methanol(40 mL), cooled in an ice bath, and sodium borohydride (570 mg, 15 mmol) is added in batches, and then the temperature is increased to room temperature, the reaction mixture continues to react for 2 hrs, then rotated to dryness, followed by adding water (50 mL), and the mixture is extracted with ethyl acetate (50 mL × 2), washed with saturated salt solution (50 mL × 2), dried with anhydrous Na₂SO₄, filtered and rotated to dryness, so as to obtain a light yellow solid of 2.0 g, that is 3-benzyl-3-azabicyclo[3.2.1] octan-8-ol. The product is used for next step without purification.

### (3) Preparation of tert-butyl 8-hydroxy-3-azabicyclo[3.2.1] octan-3-carboxylate.

3-benzyl-3-azabicyclo[3.2.1]octan-8-ol(2.0 g, 9.20 mmol) is dissolved in methanol (30 mL), with the hydrogen gas bubbled into the solution, and the reaction mixture reacts overnight at room temperature, and then filtered with siliceousearth, rotated to dryness, after that the resulted mixture is dissolved in dichloromethane (30 mL), followed by adding triethylamine (2.55 mL, 18.4 mmol) and Boc-anhydride (2.29 mL, 11.0 mmol), and the mixture continues to react at room temperature for 4hrs, rotated to dryness and purified by column chromatography, so as to obtain a white solid of 1.5g, that is *tert*-butyl 8-hydroxy-3-azabicyclo[3.2.1] octan-3-carboxylate, with a yield of 72%.
Spectrum is: ¹H NMR (400 MHz, CDCl₃) *δ*: 4.06(m, 1H), 3.71(m, 1H), 3.57(m, 1H), 3.39(m, 1H), 3.31(m, 1H), 2.01(m, 2H), 1.74(m, 3H), 1.63-1.57(m, 2H), 1.48(s, 9H).

### (4) Preparation of 3-(8((5-cyclopropyl-3-(2,6-dichlorophenyl) isoxazol-4-yl)methoxy)3-azabicyclo[3.2.1]octan-3-carbonyl)benzoic acid.

With reference to the method for preparing
3-(2-((5-cyclopropyl-3(2,6-dichlorophenyl)isoxazol-4-yl)methoxy)-7-azaspiro[3.5]nonan-7-carbo nyl)benzoic acid of Example 1, 3-(8((5-cyclopropyl-3-(2,6-dichlorophenyl) isoxazol-4-yl)methoxy)3-azabicyclo[3.2.1]octan-3-carbonyl)benzoic acid is synthesized, and *tert*-butyl 8-hydroxy-3-azabicyclo[3.2.1]octan-3-carboxylate is used to replace *tert*-butyl 2-hydroxy-7-azabicyclo[3.5]nonan-7-carboxylate.
Spectrum is: ¹H NMR (400 MHz, CDCl₃) *δ*: 8.14(m, 1H), 8.06(s, 1H), 7.62(m, 1H), 7.53(m, 1H), 7.49-7.45(m, 2H), 7.43-7.39(m, 1H), 4.37(m, 2H), 4.20(m, 1H), 3.61(m, 1H), 3.26(m, 1H), 3.02(m, 1H), 2.88(m, 1H), 2.15(m, 2H), 1.91(m, 1H), 1.66(m, 3H), 1.40(m, 1H), 1.29(m, 2H), 1.16(m, 2H).

### Example 6 2-(8-((5-cyclopropyl-3-(2,6-dichlorophenyl)isoxazol-4-yl)methoxy)-3-azabicyclo[3.2.1]octan-3-yl )benzo[d]thiazole-6-carboxylic acid is prepared.

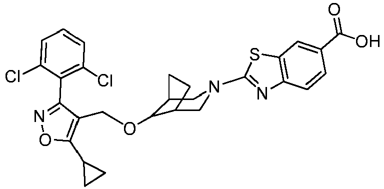

The synthetic method refers to the method of Example 2, and *tert*-butyl 8-hydroxy-3-azaspiro[3.2.1]octan-3-carboxylate is used to replace *tert*-butyl 2-hydroxy-7-azaspiro[3.5]nonan-7-carboxylate.
Spectrum is: ¹H NMR (400 MHz, CDCl₃) *δ*: 8.42 (d, *J* = 1.6 Hz, 1H), 8.11(dd, *J* = 1.6 Hz, 8.4 Hz, 1H), 7.60(d, *J* = 8.4 Hz, 1H), 7.29-7.27(m, 2H), 7.07(m, 1H), 4.42(s, 2H), 3.73(t, *J* = 5.2 Hz, 1H), 3.49(m, 2H), 3.21(m, 2H), 2.24(m, 2H), 2.13(m, 2H), 1.75-1.62(m, 4H), 1.28(m, 2H), 1.15(m, 2H).

### Example 7 3-(2-((5-cyclopropyl-3-(2,6-dichlorophenyl)isoxazol-4-yl)methoxy)-6-azaspiro[3.4]octan-6-carbo nyl)benzoic acid is prepared.

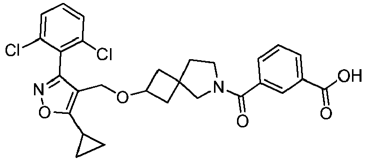

### (1) Preparation of tert-butyl 2-hydroxy-6-azaspiro[3.4]octan-6-carboxylate.

The synthetic method refers to the method for preparing *tert*-butyl
2-hydroxy-7-azaspiro[3.5]nonan-7-carboxylate of Example 1, and *tert*-butyl
3-oxopyrrolidine-1-carboxylate is used to replace *tert*-butyl 4-oxopiperidine-1-carboxylate.
Spectrum is: ¹H NMR (400 MHz, CDCl₃) *δ*: 4.35(m, 1H), 3.31(m, 4H), 2.36(m, 2H), 1.98-1.75(m, 5H), 1.48(s, 9H).

### (2) Preparation of 3-(2-((5-cyclopropyl-3-(2,6-dichlorophenyl)isoxazol-4-yl)methoxy)-6-azaspiro[3.4]octan-6-carbo nyl)benzoic acid.

The synthetic method refers to the method of Example 1, and *tert*-butyl
2-hydroxy-6-azaspiro[3.4]octan-6-carboxylate is used to replace *tert*-butyl
2-hydroxy-7-azaspiro[3.5]nonan-7-carboxylate.
Spectrum is: ¹H NMR (400 MHz, CDCl₃) *δ*: 8.24(s, 1H), 8.18(m, 1H), 7.78(d, *J* = 7.6 Hz, 1H), 7.55(m, 1H), 7.48-7.42(m, 1H), 7.38-7.33(m, 2H), 4.22(s, 1H), 4.14(s, 1H), 3.93(m, 1H), 3.68-3.60(m, 1H), 3.52(m, 1H), 3.43(m, 1H), 3.30(d, *J* = 11.6 Hz, 1H), 2.28-1.98(m, 3H), 1.89-1.82(m, 3H), 1.74-1.61(m, 1H), 1.27(m, 2H), 1.13(m, 2H).

### Example 8 2-(2-((5-cyclopropyl-3-(2,6-dichlorophenyl)isoxazol-4-yl)methoxy)-6-azaspiro[3.4]octan-6-yl)ben zo[d]thiazole-6-carboxylic acid is prepared.

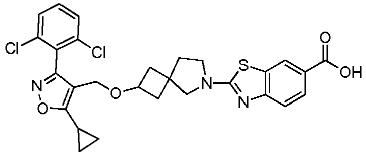

The synthetic method refers to the method of Example 2, and *tert*-butyl
2-hydroxy-6-azaspiro[3.4]octan-6-carboxylate is used to replace *tert*-butyl
2-hydroxy-7-azaspiro[3.5]nonan-7-carboxylate.
Spectrum is: ¹H NMR (400 MHz, CDCl₃) *δ*: 8.39(s, 1H), 8.08(d, *J* = 8.4 Hz, 1H), 7.59(d, *J* = 8.4 Hz, 1H), 7.47-7.44(m, 2H), 7.40-7.34(m, 1H), 4.22(s, 1H), 3.98(m, 1H), 3.54(m, 4H), 2.26-2.14(m, 3H), 2.01(t, *J* = 6.8 Hz, 2H), 1.85(m, 2H), 1.29(m, 2H), 1.16(m, 2H).

### Example 9 3-(6-((5-cyclopropyl-3-(2,6-dichlorophenyl)isoxazol-4-yl)methoxy)-2-azaspiro[3.3]heptan-2-yl)be nzoic acid is prepared.

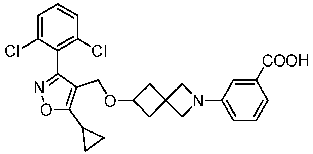

### (1) Preparation of methyl 3-(6-((5-cyclopropyl-3-(2,6-dichlorophenyl)isoxazol-4-yl)methoxy)-2-azaspiro[3.3]heptan-2-yl)be nzoate.

4-((2-azaspiro[3.3]heptan-6-oxy)methyl)-5-cyclopropyl-3-(2,6-dichlorophenyl)isoxazole(230 mg, 0.61 mmol) is dissolved in methylbenzene (8 mL), followed by adding 3-bromomethyl benzoate(156 mg, 0.73 mmol), palladium acetate (6.8 mg, 0.03 mmol), BINAP (19 mg, 0.03 mmol) and cesium carbonate (298 mg, 0.91 mmol) and the reaction mixture reacts overnight under the protection of argon gas at 100 °C. Then the reaction mixture is cooled to room temperature, and filtered with siliceousearth and rotated to dryness and purified by column chromatography, so as to obtain a white solid of 130 mg, that is
3-(6-((5-cyclopropyl-3-(2,6-dichlorophenyl)isoxazol-4-yl)methoxy)-2-azaspiro[3.3]heptan-2-yl)be nzoate, with a yield of 41%.
Spectrum is: ¹H NMR (400 MHz, CDCl₃) *δ*: 7.48-7.45(m, 2H), 7.42-7.37(m, 2H), 7.26(m, 1H), 7.07(m, 1H), 6.59(m, 1H), 4.20(s, 2H), 3.91(s, 3H), 3.87(m, 1H), 3.82(s, 2H), 3.76(s, 2H), 2.36(m, 2H), 2.16(m, 1H), 1.95(m, 2H), 1.29(m, 2H), 1.15(m, 2H).

### (2) Preparation of 3-(6-((5-cyclopropyl-3-(2,6-dichlorophenyl)isoxazol-4-yl)methoxy)-2-azaspiro[3.3]heptan-2-yl)be nzoic acid.

3-(6-((5-cyclopropyl-3-(2,6-dichlorophenyl)isoxazol-4-yl)methoxy)-2-azaspiro[3.3]heptan-2-yl)be nzoate (130 mg, 0.25 mmol) is dissolved in THF (2 mL), methanol (2 mL) and water (1 mL), then lithium hydroxide monohydrate (53 mg, 1.27 mmol) is added, and the mixture continues to react at room temperature for 20 hrs. Then the mixture is rotated to dryness, followed by adding water (100 mL), adjusting pH to 3 with IN HCl, after that the resulted product is filtered, and dried, so as to obtain a grey white solid of 110 mg, that is
3-(6-((5-cyclopropyl-3-(2,6-dichlorophenyl)isoxazol-4-yl)methoxy)-2-azaspiro[3.3]heptan-2-yl)be nzoic acid, with a yield of 90%.
Spectrum is: ¹H NMR (400 MHz, DMSO) *δ*: 7.69-7.66(m, 2H), 7.62-7.58(m, 2H), 7.24(m, 1H), 6.89(m, 1H), 6.59(m, 1H), 4.12(s, 2H), 3.80(m, 1H), 3.74(s, 2H), 3.64(s, 2H), 2.30(m, 3H), 1.75(m, 2H), 1.15(m, 2H), 1.10(m, 2H).

### Example 10 3-(2-((5-cyclopropyl-3-(2,6-dichlorophenyl)isoxazol-4-yl)methoxy)-6-azaspiro[3.4]octan-6-yl)ben zoic acid is prepared.

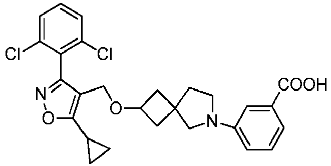

The synthetic method refers to the method of Example 9, and tert-butyl
2-hydroxy-6-azaspiro[3.4]octan-6-carboxylate is used to replace tert-butyl
6-hydroxy-2-azaspiro[3.3]heptan-2-carboxylate.
Spectrum is: ¹H NMR (400 MHz, DMSO) *δ*: 7.68-7.64(m, 2H), 7.60-7.55(m, 1H), 7.27-7.22(m, 1H), 7.16(m, 1H), 7.01(m, 1H), 6.68(m, 1H), 4.13(d, *J* = 1.6 Hz, 2H), 3.91(m, 1H), 3.22-3.15(m, 4H), 2.32(m, 1H), 2.07(m, 2H), 1.83(m, 2H), 1.59(m, 2H), 1.14(m, 2H), 1.10(m, 2H).

### Example 11 2-(10-((5-cyclopropyl-3-(2,6-dichlorophenyl)isoxazol-4-yl)methoxy)-8-azabicyclo[4.3.1]decan-8-yl)benzo[d]thiazole-6-carboxylic acid is prepared.

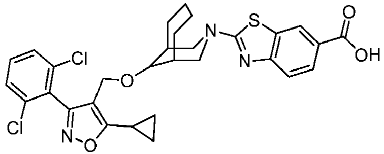

### (1) Preparation of tert-butyl 10-hydroxy-8-azabicyclo[4.3.1]decan-8-carboxylate.

The synthetic method refers to the method for preparing *tert*-butyl 8-hydroxy-3-azabicyclo[3.2.1]octan-3-carboxylate of Example 5, and cycloheptanone is used to replace cyclopentanone.
Spectrum is: ¹H NMR (400 MHz, CDCl₃) *δ*: 4.16(m, 1H), 4.06(m, 1H), 4.00(m, 1H), 2.85(m, 2H), 2.17(m, 2H), 2.00(m, 2H), 1.72(m, 2H), 1.62-1.51(m, 4H), 1.48(s, 9H).

### (2) Preparation of 2-(10-((5-cyclopropyl-3-(2,6-dichlorophenyl)isoxazol-4-yl)methoxy)-8-azabicyclo[4.3.1]decan-8-yl)benzo[d]thiazole-6-carboxylic acid.

The synthetic method refers to the method of Example 2, and *tert*-butyl 10-hydroxy-8-azabicyclo[4.3.1]decan-8-carboxylate is t used to replace *tert*-butyl 2-hydroxy-7-azapiro[3.5]nonan-7-carboxylate.
Spectrum is: ¹H-NMR (400 MHz, DMSO)*δ*: 8.14(m, 1H), 8.06(s, 1H), 7.62(m, 1H), 7.53(m, 1H), 7.49-7.45(m, 2H), 7.43-7.39(m, 1H), 4.37(m, 2H), 4.20(m, 1H), 3.61(m, 1H), 3.26(m, 1H), 3.02(m, 1H), 2.88(m, 1H), 2.15(m, 2H), 1.91(m, 1H), 1.66(m, 3H), 1.40(m, 1H), 1.29(m, 2H), 1.16(m, 2H).

### Example 12 2-(2-((5-cyclopropyl-3-(2,6-dichlorophenyl)isoxazol-4-yl)methoxy)-6-azaspiro[3.4]octan-6-yl)-4-methoxybenzo[d]thiazole-6-carboxylic acid is prepared.

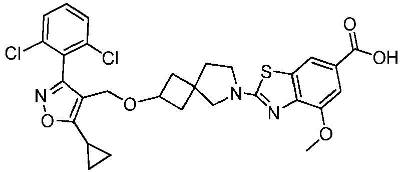

### (1) Preparation of methyl 2-amino-4-methoxybenzo[d]thiazole-6-carboxylate.

Potassium thiocyanate (2.1 g, 22.0 mmol) is dissolved in acetic acid (5 mL), followed by dripping methyl 4-amino-3-methoxybenzoate (1.0 g, 5.5 mmol) dissolved in acetic acid (5 mL) at 10 °C in 10 min, and then dripping bromine (283 µL, 5.5 mmol) dissolved in acetic acid (5 mL). Subsequently, the temperature is increased, and the reaction mixture continues to react for 4 hrs.Then the mixture is diluted with water (25 mL), followed by adjusting its pH to 8∼9 with ammonium hydroxide, and the resulted product is filtered, washed, and vacuumed to dryness, so as to obtain an orange solid of 1.38 g, that is methyl
2-amino-4-methoxybenzo[*d*]thiazole-6-carboxylate, with a yield of 100%.
Spectrum is: ¹H NMR (400 MHz, DMSO) *δ*: 7.94(d , *J*=1.2 Hz, 1H), 7.82(s , 2H), 7.34(d , *J*= 1.2 Hz, 1H), 3.88(s , 3H), 3.83(s , 3H).

### (2) Preparation of methyl 2-chlorie-4-methoxybenzo[d]thiazole-6-carboxylate.

Anhydrous cupric chloride (1.11 g, 8.25 mmol) and *tert*-butyl nitrite (1.3 mL, 11.0 mmol) are added to acetonitrile (20 mL), and the reaction mixture is stirred at room temperature for 10min, then 2-amino-4-methoxybenzo[*d*]thiazole-6-carboxylate (1.38 g, 5.5 mmol) is added in batches in 15 min, and then the reaction continues to react for 16 hrs.Then the resulted mixture is filtered with siliceousearth, and IN HCL (15 mL) is added to the filtrate, and the mixture is stirred for 15 min, to form layering, wherein the aqueous layer is extracted with ethyl acetate (15 mL × 2) and the organic layer is combined and washed with saturated salt solution(15 mL ×2), dried with anhydrous Na₂SO₄, filtered, rotated to dryness and purified by column chromatography (PE:EA = 20:1), so as to obtain a white solid of 600 mg, that is methyl
2-chlorie-4-methoxybenzo[*d*]thiazole-6-carboxylate, with a yield of 42%.
Spectrum is: ¹H NMR (400 MHz, CDCl₃) *δ*: 8.12(d, *J* = 1.6 Hz, 1H), 7.61(d, *J* = 1.6 Hz, 1H), 4.10(s, 3H), 3.98(s, 3H).

### (3) Preparation of 2-(2-((5-cyclopropyl-3-(2,6-dichlorophenyl)isoxazol-4-yl)methoxy)-6-azaspiro[3.4]octan-6-yl)-4-methoxybenzo[d]thiazole-6-carboxylic acid.

The synthetic method refers to the method of Example 8, and *tert*-butyl 2-chlorie-4-methoxybenzo[*d*]thiazole-6-carboxylate is used to replace *tert*-butyl
2-chlorobenzo[*d*]thiazole-6-carboxylate. ¹H-NMR (400 MHz, DMSO) *δ*: 8.00(t, *J* = 1.6 Hz, 1H), 7.69-7.65(m, 2H), 7.61-7.55(m, 1H), 7.38(m, 1H),4.14(d, *J* = 4.0 Hz, 1H), 3.96-3.89(m, 4H), 3.42(m, 4H), 2.32(m, 1H), 2.11(m, 2H), 1.94(m, 1H), 1.89(m, 1H), 1.68(m, 1H), 1.59(m, 1H), 1.16(m,2H), 1.10(m, 2H).

### Example 13 2-(2-((5-cyclopropyl-3-(2,6-dichlorophenyl)isoxazol-4-yl)methoxy)-6-azaspiro[3.4]octan-6-yl)-4-f lurobenzo[d]thiazole-6-carboxylic acid is prepared.

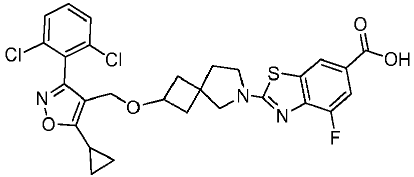

### (1) Preparation of methyl 3 -fluoro-4-aminobenzoate.

Methyl 3-fluoro-4-nitrobenzoate (1.0 g, 5.00 mmol) is dissolved in ethyl acetate (5 mL) and methanol (5 mL), followed by adding 10% Pd/C (150 mg), and the reaction mixture reacts at room temperature for 6 hrs in a hydrogen gas atmosphere, then filtered with siliceousearth, rotated to remove the solvent, so as to obtain a white solid, that is methyl 3-fluoro-4-aminobenzoate, with the yield of 98%.
Spectrum is : ¹H NMR (400 MHz, CDCl₃) *δ*: 7.69(m, 1H), 7.66(m, 1H), 6.76(m, 1H), 4.17(s , 2H), 3.88(s , 3H).

### (2) Preparation of methyl 2-amnio-4-flurobenzo[d]thiazole-6-carboxylate.

Methyl 3-fluoro-4-aminobenzoate (830 mg, 4.9 mmol) and potassium sulfocyanide (1.9 mg,19.6 mmol) are dissolved in ethyl acetate (10 mL), followed by dripping broime (250 µL, 4.9 mmol) dissolved in ethyl acetate (5 mL) at 10 °C in 30 min, after that, the temperature is increased to 30 °C ,and the reaction mixture continues to react for 48 hrs, then the mixture is diluted with water (30 mL), followed by adjusting pH to 8∼9 with ammonium hydroxide, and the resulted mixture is filtered, washed, and vacuumed to dryness, so as to obtain a yellow solid of 530 mg, that is methyl 2-amnio-4-flurobenzo[*d*]thiazole-6-carboxylate, with a yield of 48%.
Spectrum is: ¹H NMR (400 MHz, DMSO) *δ*: 8.16(m, 1H), 8.14(s , 2H), 7.56(m, 1H), 3.83(s , 3H).

### (3) Preparation of methyl 2-chloro-4-flurobenzo[d]thiazole-6-carboxylate.

Anhydrous cupric chloride(474 mg, 3.53 mmol) and *tert*-butyl nitrite (0.56 ml, 4.7 mmol) are added to acetonitrile (10 mL), and the reaction mixture is stirred at room temperature for 10 min, then methyl 2-amnio-4-flurobenzo[*d*]thiazole-6-carboxylate (530 mg, 2.35 mmol) is added in several parts in 15 min, then the reaction mixture continues to react for 16 hrs. And then the mixture is filtered with siliceousearth and 1N HCl (15 mL) is added into the filtrate and stirred well for 15 min to form a layering, wherein the aqueous layer is extracted with ethyl acetate (15 mL × 2) and the organic layer is combined, washed with saturated salt solution (15 mL × 2), dried with anhydrous Na₂SO₄, filtered, rotated to dryness and purified by column chromatography (PE:EA = 30:1), so as to obtain a white solid of 350 mg, that is methyl
2-chloro-4-flurobenzo[*d*]thiazole-6-carboxylate, with a yield of 60%.
Spectrum is: ¹H NMR (400 MHz, CDCl₃) *δ*: 8.33(d, *J* = 1.6 Hz, 1H), 7.88(dd, *J* = 1.6 Hz, 9.2Hz, 1H), 3.99(s , 3H).

### (4) Preparation of 2-(2-((5-cyclopropyl-3-(2,6-dichlorophenyl)isoxazol-4-yl)methoxy)-6-azaspiro[3.4]octan-6-yl)-4-f lurobenzo[d]thiazole-6-carboxylic acid.

The synthetic method refers to the method of Example 8, and methyl
2-chloro-4-flurobenzo[*d*]thiazole-6-carboxylate is used to replace ethyl
2-chlorobenzo[*d*]thiazole-6-carboxylate. ¹H-NMR(400 MHz, CDCl₃)*δ*: 8.18(s, 1H), 7.89(m, 1H), 7.48-7.44(m, 2H), 7.41-7.34(m, 1H), 4.22(s, 2H), 3.98(m, 1H), 3.56(m, 4H), 2.26-2.13(m, 3H), 2.02(t, J = 6.8 Hz, 2H), 1.84(m, 2H), 1.29(m, 2H), 1.16(m, 2H).

### Example 14 2-(2-((5-cyclopropyl-3-(2,6-dichlorophenyl)isoxazol-4-yl)methoxy)-6-azaspiro[3.4]octan-6-yl) benzo[d]oxazole-6-carboxylic acid is prepared.

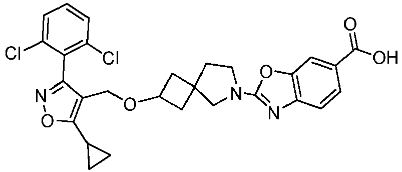

### (1) Preparation of ethyl 2- mereaptobenzo[d]oxazole-6-carboxylate.

Methyl 4-amino-3-hydroxybenzoate(0.5g, 3mm01) is dissolved in ethanol(15 mL), followed by adding potassium ethyl xanthate(577 mg, 3.6 mmol), and the mixture is heated with a reflux reaction overnight. When the reaction mixture is cooled to room temperature, rotated to dryness, the resulted mixture is dissolved with water (25 mL), its pH is adjusted to 5 with IN HCl, and then the mixture is filtered and dried, so as to obtain a brow solid of 370 mg, that is ethyl 2-mercaptobenzo[*d*]oxazole-6-carboxylate, with a yield of 59%.

### (2) Preparation of ethyl 2-chlorobenzo[d]oxazole-6-carboxylate.

Ethyl 2-mercaptobenzo[*d*]oxazole-6-carboxylate (370 mg, 1.77 mmol) is dissolved in thionyl chloride (8 mL), followed by adding 3 drops of DMF, and the reaction mixture reacts at 80 °C for 10 min, cooled to room temperature, rotated to dryness, followed by adding ethyl acetate (30 mL) and water (30 mL), wherein the organic layer is dried with anhydrous Na₂SO₄, rotated to dryness and purified by column chromatography (PE : EA = 15 : 1), so as to obtain a light yellow solid of 270 mg, that is ethyl 2-chlorobenzo[*d*]oxazole-6-carboxylate, with a yield of 72%.

### (3) Preparation of ethyl 2-(2-((5-cyclopropyl-3-(2,6-dichlorophenyl)isoxazol-4-yl)methoxy)-6-azaspiro[3.4]octan-6-yl) benzo[d]oxazole-6-carboxylate.

4-((6- azaspiro[3.4]octan-2-oxy)methyl)-5-cyclopropyl-3-(2,6-dichlorophenyl) isoxazole (80 mg, 0.174 mmol) and ethyl 2-flurobenzo[*d*]oxazole-6-carboxylate (43 mg, 0.191 mmol) are dissolved in 5 ml of acetonitrile, followed by adding triethylamine (60 µl, 0.450 mmol), and the reaction mixture reacts with a reflux at 80 °C for 1.5 hr. When the reaction mixture is cooled to room temperature, it is rotated to dryness and purified by column chromatography (eluant: PE / EA = 2 / 1), so as to obtain a colorless oily substance of 95 mg, that is ethyl
2-(2-((5-cyclopropyl-3-(2,6-dichlorophenyl)isoxazol-4-yl)methoxy)-6-azaspiro[3.4]octan-6-yl) benzo[*d*]oxazole-6-carboxylate with a yield of 89%.

### (4) Preparation of 2-(2-((5-cyclopropyl-3-(2,6-dichlorophenyl)isoxazol-4-yl)methoxy)-6-azaspiro[3.4]octan-6-yl) benzo[d]oxazole-6-carboxylic acid.

Ethyl
2-(2-((5-cyclopropyl-3-(2,6-dichlorophenyl)isoxazol-4-yl)methoxy)-6-azaspiro[3.4]octan-6-yl) benzo[d]oxazole-6-carboxylate (90 mg, 0.156 mmol) is dissolved in 2 ml of THF and 2 ml of methanol, followed by adding lithium hydroxide (33 mg, 0.788 mmol) and water (1 mL), and the reaction mixture reacts at room temperature for 16 hrs, then rotated to dryness, and followed by adding 10 ml of water for dissolving, adjusting its pH to approximately 3∼4 with 1M HCl, and the resulted mixture is filtered, washed and vacuum dried, so as to obtain a white solid of 57 mg, that is 2-(2-((5-cyclopropyl-3-(2,6-dichlorophenyl)isoxazol-4-yl)methoxy)-6-azaspiro[3.4]octan-6-yl) benzo[*d*]oxazole-6-carboxylic acid, with a yield of 65%.
Spectrum is : ¹H NMR (400 MHz, CDCl₃) *δ*: 8.04-8.00(m, 2H), 7.47-7.44(m, 2H), 7.40-7.34(m, 2H), 4.22(s, 2H), 3.98(m, 1H), 3.67(m, 2H), 3.58(s, 1H), 3.54(s, 1H), 2.25-2.13(m, 3H), 1.97(t, *J* = 6.8 Hz, 2H), 1.83(m, 2H), 1.30(m, 2H), 1.15(m, 2H).

### Example 15 (R)-2-(2-((5-cyclopropyl-3-(2,6-dichlorophenyl)isoxazol-4-yl)methoxy)-7-methyl-6-azaspiro[3.4] octan-6-yl)benzo[d]thiazole-6-carboxylic acid is prepared.

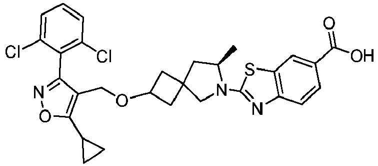

### (1) Preparation of (2S,4R)-methyl 1-tert-butyloxycarboryl-4-((tert-butyldimethyl silicon)-oxy)pyrrolidine-2-carboxylate.

Boc-L-hydroxyproline ethyl ester (5 g, 20.4 mmol) and imidazole (2.92 g, 42.8 mmol) are dissolved in DMF (50 mL), followed by adding TBSCl (3.38 g, 22..4 mmol), and the reaction mixture reacts overnight at room temperature. Then, the reaction mixture is poured into water(100 mL), and extracted with ethyl acetate (100 mL × 2), wherein the organic layer is combined and washed with saturated salt solution (100 mL × 3), dried with anhydrous Na₂SO₄, rotated to dryness and purified by column chromatography, so as to obtain a colorless oily substance of 7.26 g, that is (2S,4R)-methyl 1-*tert*-butyloxycarboryl-4-((*tert*-butyldimethyl silicon)-oxy)pyrrolidine-2-carboxylate, with a yield of 99%.

### (2) Preparation of (2S,4R)- tert-butyl 4-((tert-butyldimethyl silicon)-oxy)2-(hydroxymethyl)pyrrolidine-1-carboxylate.

(2S,4R)-methyl 1-*tert*-butyloxycarboryl-4-((*tert*-butyldimethyl
silicon)-oxy)pyrrolidine-2-carboxylate (7.8 g, 21.7 mmol) is dissolved in THF (60 mL), cooled to 0 °C, followed by adding 2M lithium borohydride in tetrahydrofuran solvent (16.3 mL), and the reaction mixture continues to react at room temperature for 5 hrs, then adding ethyl acetate (20 mL), and the mixture is cooled to 0 °C, quenched a reaction by dripping 1N HCl, and after adding water (100 mL) the mixture is extracted with ethyl acetate (100 mL × 2), then the organic layer is combined and washed with saturated salt solution (100 mL × 2), dried with anhydrous Na₂SO₄, rotated to dryness, so as to obtain a light yellow oily substance of 6.81 g with a yield of 95%.

### (3) Preparation of (2S,4R)-tert-butyl 4-((tert-butyldimethyl silicon)-oxy)2-((methanesulfonyl)oxy)pyrrolidine-1-carboxylate.

(2S,4R)-*tert*-butyl 4-((*tert*-butyldimethyl
silicon)-oxy)2-(hydroxymethyl)pyrrolidine-1-carboxylate (6.81 g, 20.6 mmol) is dissolved in dichloromethane (60mL), followed by adding triethylamine (5.7 mL, 41.2 mmol), and then the mixture is cooled to 0 °C, dripping methyl sulfonyl chloride (3.54 g, 30.9 mmol), the reaction mixture reacts overnight at 0 °C. Then the reaction mixture is poured into water(100 mL), and extracted with dichloromethane (60 mL × 2), then the organic layer is combined and washed with saturated salt solution (100 mL × 2), dried with anhydrous Na₂SO₄, rotated to dryness, so as to obtain a brown oily matter of 8.4 g, that is (2S,4R)-*tert*-butyl 4((*tert*-butyldimethyl silicon)-oxy)2-((methane sulfonyl)oxy)pyrrolidine-1-carboxylate with a yield of 99%.

### (4)Preparation of (2S,4R)-tert-butyl 4-((tert-butyldimethyl silicon)-oxy)2-methylpyrrolidine-1-carboxylate.

(2S,4R)-*tert*-butyl 4-((*tert*-butyldimethyl silicon)-oxy)2-((methane
sulfonyl)oxy)pyrrolidine-1-carboxylate (3 g, 7.32 mmol) is dissolved in THF (30 mL), then cooled to 5 °C, followed by adding 1M sodium triethylborohydride in tetrahydrofuran borohydride solvent (22 mL). Then the temperature is increased to room temperature, and the mixture continues to react at room temperature for 2 hrs, then cooled to 0 °C, quenched a reaction by dripping water, then adding water (50 mL) and the mixture is extracted with ethyl acetate (50 mL × 2), washed with saturated salt solution (100 mL × 2), dried with anhydrous Na₂SO₄, rotated to dryness, so as to obtain a colorless oily matter of 2.2 g, that is (2S,4R)-*tert*-butyl 4-((*tert*-butyldimethyl silicon)-oxy)2-methylpyrrolidine-1-carboxylate with a yield of 98%.

### (5) Preparation of (2S,4R)-tert-butyl 4-hydroxyl-2-methylpyrrolidine-1-carboxylate.

(2S,4R)-*tert*-butyl 4-((*tert*-butyldimethyl silicon)-oxy)2-methylpyrrolidine-1-carboxylate (2.2 g, 6.98 mmol) is dissolved in THF (30 mL), followed by adding tetrabutylammonium fluoride (3.65 g, 14.0 mmol), and the reaction mixture reacts overnight at room temperature. Then the reaction mixture is poured into water (60 mL), and extracted with ethyl acetate (60 mL × 2), washed with saturated salt solution (60 mL × 2), dried with anhydrous Na₂SO₄, rotated to dryness and purified by column chromatography (PE : EA = 2 : 1), so as to obtain a yellow oily matter of 1.47 g, that is (2S,4R)-*tert*-butyl 4-hydroxyl-2-methylpyrrolidine-1-carboxylate with a yield of 99%.

### (6) Preparation of (2R)-tert-butyl 2-methyl-4-oxopyrrolidine-1-carboxylate.

(2S,4R)-*tert*-butyl 4-hydroxyl-2-methylpyrrolidine-1-carboxylate (1.47 g, 7.3 mmol) is dissolved in dichloromethane (20 mL), followed by adding silica gel (2.36 g) and PCC (2.36 g, 11.0 mmol), and the reaction mixture reacts overnight at room temperature, then filtered, rotated to dryness and purified by column chromatography (PE : EA = 5 : 1), so as to obtain a light yellow oily matter of 1.2 g, that is (2R)-*tert*-butyl 2-methyl-4-oxopyrrolidine-1-carboxylate with the yield of 82%.

### (7) Preparation of (2R)-tert-butyl 2-methyl-4-methylenepyrrolidine-1-carboxylate.

Ph₃P⁺CH₃Br⁻ (2.48 g, 6.95 mmol) and potassium *tert*-butoxide (0.85 g, 7.53 mmol) are added to 20mL of diethyl ether at 0 °C, then the temperature is increased to room temperature, and the reaction mixture is stirred for 2 hrs. Then, (2R)-*tert*-butyl
2-methyl-4-oxopyrrolidine-1-carboxylate (1.16 g, 5.79 mmol) dissolved in diethyl ether (10 mL) is dripped into the reaction mixture in 15 min at 0 °C. Then, the temperature is increased to room temperature and the mixture reacts at room temperature for 16 hrs, then filtered with siliceousearth, washed, rotated to dryness and purified by column chromatography (eluant: PE / EA = 80 / 1), so as to obtain a colorless liquid of 680 mg, that is (2R)-*tert*-butyl
2-methyl-4-methylenepyrrolidine-1-carboxylate with a yield of 60%.
Spectrum is: ¹H NMR (400 MHz, CDCl₃) *δ*: 5.0(s, 2H), 4.17-3.98(m , 2H), 3.88(m , 1H), 2.36-2.10(m, 2H), 1.49(s, 9H), 1.14(dd*, J* = 6.0 Hz, 3H).

### (8) Preparation of (7R)-tert-butyl 7-methyl-2-oxy-1,1-dichloro-6-azaspiro[3.4]octan-6-carboxylate.

(2R)-*tert*-butyl 2-methyl-4-methylenepyrrolidine-1-carboxylate (680 mg, 3.45 mmol) is dissolved in diethyl ether (20 mL), followed by adding zinc-copper couple (2.67 g, 20.7 mmol) under the protection of nitrogen. Trichloro-acetic chloride (1.15 mL, 10.35 mmol) is dissolved in glycol dimethyl ether (5 mL) and dripped successively into the above reaction mixture, and the mixture continues to react overnight at room temperature. Then the reaction mixture is poured into 0 °C saturated salt solution (30 mL), and then the mixture is filtered with sillceousearth and extracted with ethyl acetate (30 mL × 2), washed with saturated salt solution (50 mL × 2) again, dried with anhydrous Na₂SO₄, rotated to dryness and purified by column chromatography (PE : EA = 6 : 1), so as to obtain a brown oily substance of 1.06 g, that is (7R)-*tert*-butyl
7-methyl-2-oxy-1,1-dichloro-6-azaspiro[3.4]octan-6-carboxylate, which is directly used in the next reaction without purification

### (9) Preparation of (7R)-tert-butyl 7-methyl-2-oxy-6-azaspiro[3.4]octan-6-carboxylate.

(7R)-*tert*-butyl 7-methyl-2-oxy-1,1-dichloro-6-azaspiro[3.4]octan-6-carboxylate (1.06 g, 3.44 mmol) is dissolved in methanol (10 mL), followed by adding zinc powder (1.34 g, 20.6 mmol) and saturated ammonium chloride (10 mL), and the reaction mixture reacts overnight at room temperature. The solid is removed by filtering, and the filtrate is rotated to dryness, followed by adding water (20mL), and the mixture is extracted with ethyl acetate (20 mL × 2) and washed with saturated salt solution (20 mL × 2), dried with anhydrous Na₂SO₄, rotated to dryness and purified by column chromatography (PE : EA = 5 : 1), so as to obtain a light yellow oily matter, that is (7R)-*tert*-butyl 7-methyl-2-oxy-6-azaspiro[3.4]octan-6-carboxylate with a yield of 40%.
Spectrum is: ¹H-NMR (400 MHz, CDCl₃) *δ*: 3.97(m, 1H), 3.70-3.52(m, 2H), 3.18-2.97(m, 4H), 2.31(m, 1H), 1.85(m, 1H), 1.49(s, 9H), 1.31(m, 3H).

### (10) Preparation of (7R)-tert-butyl 7-methyl-2-hydroxy-6-azaspiro[3.4]octan-6-carboxylate.

(7R)-*tert*-butyl 7-methyl-2-oxy-6-azaspiro[3.4]octan-6-carboxylate (325 mg, 1.36 mmol) is dissolved in methanol (8 mL), cooled down in an ice bath, then sodium borohydride (103 mg, 2.72 mmol) is added in batches, and the reaction mixture reacts at room temperature for 2 hrs, rotated to dryness then water (20 mL) is added, and the resulted mixture is extracted with ethyl acetate (20 mL × 2), washed with saturated salt solution (20 mL × 2), dried with anhydrous Na₂SO₄, rotated to dryness and purified by column chromatography (PE : EA = 3 : 1), so as to obtain a brown oily matter of 300 mg, that is (7R)-*tert*-butyl 7-methyl-2-hydroxy-6-azaspiro[3.4]octan-6-carboxylate, with a yield of 91%.
Spectrum is: ¹H-NMR(400 MHz, CDCl₃)*δ*: 4.31(m, 1H), 3.83(m, 1H), 3.40-3.33(m, 2H), 2.43(m, 1H), 2.33(m, 2H), 2.09-1.85(m, 4H), 1.48(s, 9H), 1.22(m, 3H).

### (11) Preparation of (R)-2-(2-((5-cyclopropyl-3-(2,6-dichlorophenyl)isoxazol-4-yl)methoxy)-7-methyl-6-azaspiro[3.4] octan-6-yl) benzo[d]thiazole-6-carboxylic acid.

The synthetic method refers to the method of Example 2, and (R)-*tert*-butyl
2-hydroxy-7-methyl-6-azaspiro[3.4]octan-6-carboxylate is used to replace *tert*-butyl
2-hydroxy-7-azaspiro[3.4]nonan-7-carboxylate.
Spectrum is: ¹H NMR (400 MHz, CDCl₃) *δ*: 8.40(d, *J* = 1.2 Hz, 1H), 8.08(dd, *J* = 1.2 Hz, 8.4 Hz, 1H), 7.60(d, *J* = 8.4 Hz, 1H), 7.48-7.44(m, 2H), 7.40-7.34(m, 1H), 4.20(s, 2H), 4.08(m, 1H), 3.60(m, 2H), 2.30-2.13(m, 4H), 1.97-1.73(m, 3H), 1.41(dd, *J* = 6.4 Hz, 23.2 Hz, 3H), 1.29(m, 2H), 1.15(m, 2H).

### Example 16 (S)-2-(2-((5-5-cyclopropyl-3-(2,6-dichlorophenyl)isoxazol-4-yl)methoxy)-7-(fluromethyl)-6-azasp iro[3.4]octan-6-yl) benzo[d]thiazole-6-carboxylic acid is prepared.

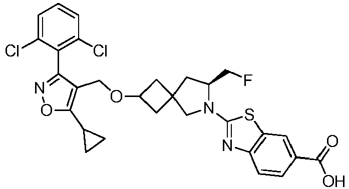

### (1) Preparation of (2S, 4R)-tert-butyl 2-(fluoromethyl)-4-hydroxypyrrolidine-1-carboxylate.

(2S, 4R)-*tert*-butyl 4-((*tert*-butyldimethylsilyl)oxy)-2(((methylsulfonyl)oxy)methyl) pyrrolidine-1-carboxylate (4.8 g, 11.7 mmol) is dissolved in THF (60 mL), followed by adding tetrabutylammonium fluoride (12.2 g, 46.8 mmol), and the reaction mixture reacts at room temperature for 6 hrs, then heated to react overnight. Then the reaction mixture is cooled to room temperature, rotated to dryness, then water (100 mL) is added, and the mixture is extracted with ethyl acetate (80 mL × 2), washed with saturated salt solution (80 mL × 2), dried with anhydrous Na₂SO₄, rotated to dryness and purified by column chromatography (PE : EA = 2.5 : 1), so as to obtain a yellow oily matter of 1.65 g, that is (2S, 4R)-*tert*-butyl
2-(fluoromethyl)-4-hydroxypyrrolidine-1-carboxylate with a yield of 64%.
Spectrum is: ¹H NMR (400 MHz, CDCl₃) *δ*: 4.83-4.63(m, 1H), 4.50(m, 2H), 4.11(m, 1H), 3.48(m, 2H), 2.21-2.08(m, 2H), 1.87(m, 1H), 1.49(m, 9H).

### (2) Preparation of (2S, 4R)-tert-butyl 2-(fluoromethyl)-4-oxypyrrolidine-1-carboxylate.

(2S, 4R)-*tert*-butyl 2-(fluoromethyl)-4-hydroxypyrrolidine-1-carboxylate (1.65 g, 7.56 mmol) is dissolved in DCM (50 mL), followed by adding silica gel of 2.5 g, then PCC (2.44 g, 11.34 mmol) is added in batches at 0 °C, and after that, the temperature is increased to room temperature and the reaction mixture reacts for 16 hrs, then filtered, rotated to dryness and purified by column chromatography (PE:EA= 6:1), so as to obtain light a yellow oily matter of 1.35 g, that is (2S, 4R)-*tert*-butyl 2-(fluoromethyl)-4-oxypyrrolidine-1-carboxylate, with a yield of 82%.
Spectrum is: ¹H NMR (400 MHz, CDCl₃) *δ*: 4.76(m, 1H), 4.52-4.38(m, 2H), 3.90(m, 1H), 3.72(d, *J* = 19.2 Hz, 1H), 2.80(m, 1H), 2.56(d, *J* = 19.2 Hz, 1H), 1.50(s, 9H).

### (3) Preparation of (2S, 4R)-tert-butyl 2-(fluoromethyl)-4-methylenepyrrolidine-1-carboxylate.

Ph₃P⁺CH₃Br⁻ (2.66 g, 7.46 mmol) and potassium *tert*-butoxide (0.91 g, 8.07 mmol) are added to 20 mL of diethyl ether at 0 °C, then the temperature is increased to room temperature, and the mixture is stirred for 2 hrs, followed by dripping 18130 (1.35 g, 6.21 mmol) dissolved in 10 ml of diethyl ether at 0 °C in 15 min, when finishing, the temperature is increased to room temperature again and the mixture continues to react for 16 hrs, then filtered with sillceosearth, washed, rotated to dryness to remove the filtrate, purified by column chromatography (eluant: PE / EA= 100/1), so as to obtain a colorless transparent liquid of 960 mg, that is (2S, 4R)-*tert*-butyl 2-(fluoromethyl)-4-methylenepyrrolidine-1-carboxylate with a yield of 72%.
Spectrum is : ¹H NMR(400 MHz, CDCl₃)*δ*: 5.04(m, 2H), 4.49-4.08(m, 4H), 3.88(m, 1H), 2.78(m, 1H), 2.59(m, 1H), 1.49(s, 9H).

### (4) Preparation of (S)-tert-butyl 7-(fluoromethyl)-2-hydroxy-6-azaspiro[3.4]octan-6-carboxylate.

The synthetic method refers to the method for preparing (R)-*tert*-butyl
2-hydroxy-7-methyl-6-azaspiro[3.4]octan-6-carboxylate of Example 15,wherein (S)-*tert*-butyl 2-(fluoromethyl)-4-methylenepyrrolidine-1-carboxylate is used to replace (R)-*tert*-butyl 2-methyl-4-methylenepyrrolidine-1-carboxylate.
Spectrum is: ¹H-NMR (400 MHz, CDCl₃) *δ*: 4.54(m, 1H), 4.46-4.29(m, 2H), 3.96(m, 1H), 3.48-3.31(m, 2H), 2.45-2.30(m, 2H), 2.04(m, 4H), 1.83(m, 1H), 1.48(s, 9H).

### (5) Preparation of (S)-2-(2-((5-5-cyclopropyl-3-(2,6-dichlorophenyl)isoxazol-4-yl)methoxy)-7-(fluromethyl)-6-azasp iro[3.4]octan-6-yl)benzo[d]thiazole-6-carboxylic acid.

The synthetic method refers to the method of Example 2, wherein (S)-*tert*-butyl
7-(fluoromethyl)-2-hydroxy-6-azaspiro[3.4]octan-6-carboxylate is used to replace *tert*-butyl 2-hydroxy-7-azaspiro[3.5]nonan-7-carboxylate.
Spectrum is: ¹H-NMR (400 MHz, CDCl₃) *δ*: 8.42(s, 1H), 8.09(d, J = 8.8 Hz, 1H), 7.60(m, 1H), 7.48-7.44(m, 2H), 7.40-7.34(m, 1H), 4.97-4.52(m, 2H), 4.32(m, 1H), 4.21(s, 2H), 3.95(m, 1H), 3.56(m, 1H), 3.48(m, 1H), 2.29(m, 1H), 2.21-2.14(m, 4H), 1.95-1.81(m, 2H), 1.29(m, 2H), 1.15(m, 2H).

### Example 17 (R)-2-(2-((5-cyclopropyl-3-(2,6-dichlorophenyl)isoxazol-4-yl)-7-methoxy -6-azaspiro[3.4]octan-6-yl)-4-flurobenzo[d]thiazole-6-carboxylic acid is prepared.

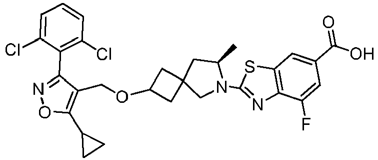

The synthesis method refers to the method of Example 15, wherein methyl
2-chloro-4-fluorobenzo[*d*]thiazole-6-carboxylate is used to replace ethyl
2-chlorobenzo[*d*]thiazole-6-carboxylate.
Spectrum is: ¹H-NMR (400 MHz, CDCl₃) *δ*: 8.19(s, 1H), 7.79(d, J = 11.2 Hz, 1H), 7.48-7.44(m, 2H), 7.41-7.34(m, 1H), 4.20(s, 2H), 4.09(m, 1H), 3.96(m, 1H), 3.60(m, 2H), 2.34-2.12(m, 4H), 1.98-1.73(m, 3H), 1.41(dd, J = 6.4 Hz, 21.2 Hz, 3H), 1.29(m, 2H), 1.16(m, 2H).

### Example 18 (R)-2-(2-((5-cyclopropyl-3-(2,6-dichlorophenyl)isoxazol-4-yl)-7-methoxy -6-azaspiro[3.4]octan-6-yl)-4-methoxybenzo[d]thiazole-6-carboxylic acid is prepared.

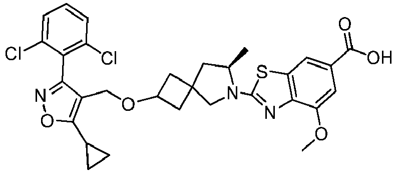

The synthesis method refers to the method of Example 15, and methyl
2-chloro-4-methoxybenzo[*d*]thiazole-6-carboxylate is used to replace ethyl
2-chlorobenzo[*d*]thiazole-6-carboxylate.
Spectrum is: ¹H-NMR (400 MHz, CDCl₃) *δ*: 8.08(s, 1H), 7.57(s, 1H), 7.49-7.44(m, 2H), 7.40-7.33(m, 1H), 4.19(s, 2H), 4.06-3.93(m, 5H), 3.74-3.59(m, 2H), 2.32-2.09 (m, 4H), 1.97-1.71(m, 3H), 1.39(dd, J = 6.4 Hz, 21.6 Hz, 3H), 1.28(m, 2H), 1.15(m, 2H).

### Example 19 (S)-2-(2-((5-cyclopropyl-3-(2,6-dichlorophenyl)isoxazol-4-yl)methoxy)-7-(fluromethyl)-6-azaspir o[3.4]octan-6-yl)-4-flurobenzo[d]thiazole-6-carboxylic acid is prepared.

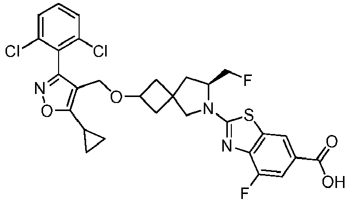

The synthesis method refers to the method of Example 15, wherein methyl
2-chloro-4-fluorobenzo[*d*]thiazole-6-carboxylate is just used to replace ethyl
2-chlorobenzo[*d*]thiazole-6-carboxylate.
Spectrum is: ¹H-NMR (400 MHz, CDCl₃) *δ*: 8.20(s, 1H), 7.80(d, J = 10.8 Hz, 1H), 7.48-7.44(m, 2H), 7.41-7.35(m, 1H), 4.92(m, 1H), 4.67-4.49(m, 1H), 4.35(m, 1H), 4.20(s, 2H), 3.95(m, 1H), 3.59-3.44(m, 2H), 2.29(m, 1H), 2.21-2.14(m, 4H), 1.95-1.80(m, 2H), 1.29(m, 2H), 1.15(m, 2H).

### Example 20 (S)-2-(2-((5-cyclopropyl-3-(2,6-dichlorophenyl)isoxazol-4-yl)methoxy)-7-(fluromethyl)-6-azaspir o[3.4]octan-6-yl)-4-methoxybenzo[d]thiazole-6-carboxylic acid is prepared.

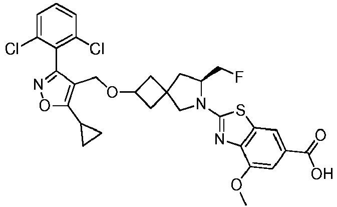

The synthesis method refers to the method of Example 16, wherein methyl
2-chloro-4-fluorobenzo[*d*]thiazole-6-carboxylate is just used to replace ethyl
2-chlorobenzo[*d*]thiazole-6-carboxylate.
Spectrum is: ¹H-NMR (400 MHz, CDCl₃) *δ*: 8.09(m, 1H), 7.58(m, 1H), 7.48-7.44(m, 2H), 7.40-7.34(m, 1H), 4.97-4.49(m, 2H), 4.43-4.26(m, 1H), 4.20(s, 2H), 4.05(s, 3H), 3.94(m, 1H), 3.63-3.47(m, 2H), 2.27(m, 1H), 2.19-2.13(m, 4H), 1.94-1.78(m, 2H), 1.29(m, 2H), 1.15(m, 2H).

### Example 21 (S)-2-(2-((5-cyclopropyl-3-(2,6-dichlorophenyl)isoxazol-4-yl)methoxy)-7-(fluromethyl)-6-azaspir o[3.4]octan-6-yl)benzo[d]oxazole-6-carboxylic acid is prepared.

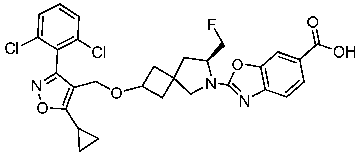

The synthesis method refers to the method of Example 15, wherein methyl
2-chloro-4-fluorobenzo[*d*]oxazole-6-carboxylate is just used to replace ethyl
2-chlorobenzo[*d*]thiazole-6-carboxylate.
Spectrum is: ¹H-NMR (400 MHz, CDCl₃) *δ*: 8.05-8.02(m, 2H), 7.47-7.43(m, 2H), 7.40-7.33(m, 2H), 4.91-4.48(m, 2H), 4.21(m, 3H), 3.95(m, 1H), 3.71(m, 1H), 3.62(m, 1H), 2.27(m, 1H), 2.18-2.13(m, 4H), 1.95-1.78(m, 2H), 1.28(m, 2H), 1.15(m, 2H).

### Example 22 2-(5-((5-cyclopropyl-3-(2,6-dichlorophenyl)isoxazol-4-yl)methoxy)isoindolin-2-yl) benzo[d]thiazole-6-carboxylic acid is prepared.

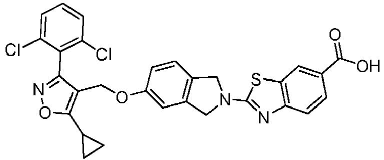

### (1) Preparation of methyl 5-methoxy-2-methylbenzoate.

5-Methoxy-2-methylbenzoic acid (3.95 g, 26.0 mmol) is dissolved in DMF(30 mL), followed by adding Cs₂CO₃ (21.21 g, 65.0 mmol), and dripping CH₃I (4.1 mL, 65.0 mmol) at 0 °C in 10 min, then the temperature is increased to room temperature, and the reaction mixture reacts overnight, then filtered with sillceousearth. Then the filtrate is poured into water (100 mL), the mixture is extracted with ethyl acetate (60 mL × 2), and subsequently the organic layer is combined and washed with saturated salt solution (60 mL × 2), dried with anhydrous Na₂SO₄ and filtered, rotated to dryness, so as to obtain a yellow oily matter of 4.80 g, that is methyl 5-methoxy-2-methylbenzoate, which is directly used for the next reaction.
Spectrum is: ¹H NMR (400 MHz, CDCl₃) *δ*: 7.46(d, *J* =2.8 Hz,1H), 7.16(d, *J* = 8.4 Hz,1H), 6.97(dd, *J* = 8.4 Hz, 2.8 Hz,1H), 3.91(s, 3H), 3.83(s, 3H), 2.53(s, 3H).

### (2) Preparation of methyl 2-(bromomethyl)-5-methoxybenzoate.

Methyl 5-methoxy-2-methylbenzoate(4.8 g, 26.0 mmol) is dissolved in CCl₄ (100 mL), followed by adding NBS (5.1 g, 28.6 mmol), (PhCO₂)₂ (315 mg, 1.30 mmol), and the reaction mixture reacts with a reflux for 1 hr at 80 °C, then the reaction mixture is cooled down to room temperature, and poured into ice water(150 mL), and extracted with dichloromethane (100 mL × 3), the organic layer is combined, dried with anhydrous Na₂SO₄, filtered and rotated to remove the solvent, so as to obtain a yellow oily matter of 7.5 g, that is methyl 2-(bromomethyl)-5-methoxybenzoate, which is used in the following reaction without purification.

### (3) Preparation of 2-benzyl-6-methoxyisoindolin-1-one.

Methyl 2-(bromomethyl)-5-methoxybenzoate (7.5 g, 26.0 mmol) is dissolved in methonal (150 mL), followed by adding benzylamine (3.0 mL, 27.0 mmol), triethylamine (5.6 mL, 40.0 mmol), and the mixture reacts with a reflux overnight at 70 °C. Then the reaction mixture is cooled down to room temperature and is rotated to remove solvent, followed by adding water (100 mL), the mixture is extracted with dichloromethane (100 mL), and then the organic layer is dried with anhydrous Na₂SO₄, filtered, rotated to remove solvent, after that, petroleum ether (100 mL) is added, and the mixture is filtered again, so as to obtain a light yellow solid of 3.1 g, that is 2-benzyl-6-methoxyisoindolin-1-one, wherein an overall yield of the above 3 steps accounts for 49%.
Spectrum is: ¹H NMR (400 MHz, CDCl₃) *δ*: 7.41(d, *J* = 2.4 Hz,1H), 7.33(m, 6H), 7.10(dd, *J* = 8.4 Hz, 2.4 Hz,1H), 4.82(s, 2H), 4.22(s, 2H), 3.90(s, 3H).

### (4) Preparation of 2-benzyl-5-methoxyisoindole.

LiAlH4 (177 mg, 4.66 mmol) is added into anhydrous THF (5 mL) at 0 °C under the argon protection, followed by dripping 2-benzyl-6-methoxyisoindolin-1-one (585 mg, 2.33 mmol) dissolved in anhydrous THF (5 mL) in 15 min, then the temperature is increased to 65 °C, and the reaction mixture reacts with a reflux for 4 hrs. Then the reaction mixture is cooled to room temperature, followed by dripping water (0.8 mL) to quench the reaction, then 10% NaOH solution (0.2 mL) is dripped into the mixture, and the mixture is stirred well, filtered with sillceousearth, rotated to remove solvent and purified by column chromatography (PE : EA = 30 : 1), so as to obtain a brown oily matter of 430 mg, that is 2-benzyl-5-methoxyisoindole with a yield of 78%.
Spectrum is: ¹H NMR (400 MHz, CDCl₃) *δ*: 7.45(d, *J* = 6.8 Hz, 2H), 7.39(t, *J* = 6.8 Hz, 2H), 7.31(m, 1H), 7.11(d, *J* = 8.8 Hz,1H), 3.94(s, 4H), 3.91(s, 2H), 3.81(s, 3H).

### (5) Preparation of 5-methoxyisoindole.

2-benzyl-5-methoxyisoindole (430 mg, 1.81 mmol) is dissolved in methanol (10 mL), and Pd/C (80 mg) is added, then hydrogen gas is led into the mixture, and the mixture reacts overnight at room temperature, then it is filtered with sillceousearth, rotated to remove solvent, so as to obtain a yellow oily matter of 211 mg, that is 5-methoxyindole, with a yield of 78%.

### (6) Preparation of ethyl 2-(5-methoxyisoindole-2-yl)benzo[d]thiazole-6-carboxylate.

5-methoxyindole (211 mg,1.41 mmol) is dissolved in DMF (10 mL), followed by adding ethyl 2-clorobenzo[*d*]thiazole-6-carboxylate (342 mg, 1.41 mmol) and triethylamine (392 µL, 2.82 mmol), the mixture reacts at 100 °C for 1 hr under the nitrogen protection. Then the reaction mixture is cooled to room temperature and filtered, the filter cake is pulped to form slurry and filtered, so as to obtain a white solid of 280 mg, that is ethyl
2-(5-methoxyisoindole-2-yl)benzo[*d*]thiazole-6-carboxylate with a yield of 56%.
Spectrum is: ¹H NMR (400 MHz, CDCl₃) *δ*: 8.38(d, *J* = 1.2 Hz ,1H), 8.05(dd, *J* = 8.6 Hz, 1.8 Hz,1H), 7.64(d, *J* = 8.8 Hz, 1H), 7.26(s, 1H), 6.92(m ,2H), 4.94(s, 2H), 4.90(s, 2H), 4.41(q, *J* = 7.2 Hz, 2H), 3.86(s, 3H), 1.43(t, *J*=7.2 Hz, 3H).

### (7) Preparation of ethyl 2-(5-hydroxyisoindoline-2-yl)benzo[d]thiazole-6-carboxylate.

Ethyl 2-(5-methoxyisoindole-2-yl)benzo[*d*]thiazole-6-carboxylate (280 mg, 0.79 mmol) is dissolved in DCM (10 mL), cooled in an ice bath, followed by dripping boron tribromide (396 mg, 1.58 mmol) dissolved in dichloromethane solution (5 mL), and the reaction mixture continues to react overnight at 0 °C, then dripping methanol (2 mL) into the mixture to quench the reaction, then the mixture is rotated to dryness and purified by column chromatography (dichloromethane : methanol = 50 : 1), so as to obtain a white solid of 120 mg, that is ethyl 2-(5-hydroxyisoindoline -2-yl)benzo[*d*]thiazole-6-carboxylate with a yield of 45%.
Spectrum is: ¹H-NMR (400 MHz, CDCl₃) *δ*: 9.53(s, 1H), 8.45(d, J = 1.6 Hz, 1H), 7.89(dd, J = 1.6 Hz, 8.4 Hz, 1H), 7.55(d, J = 8.4 Hz, 1H), 7.21(d, J = 8.0 Hz, 1H), 6.81(d, J = 1.6 Hz, 1H), 6.75(dd, J = 1.6 Hz, 8.0 Hz, 1H), 4.79(m, 4H), 4.30(q, J = 7.2 Hz, 2H), 1.33(t, J = 7.2 Hz, 3H).

### (8) Preparation of ethyl 2-(5-((5-cyclopropyl-3-(2,6-dichlorophenyl)isoxazol-4-yl)methoxy) isoindoline-2-yl)benzo[d]thiazole-6-carboxylate.

Sodium-hydrogen (28 mg, 0.72 mmol) is suspended on anhydrous THF (5 mL), followed by adding ethyl 2-(5-hydroxyisoindoline-2-yl)benzo[*d*]thiazole-6-carboxylate (80 mg, 0.24 mmol), and the reaction mixture is stirred at room temperature for 20 min, then adding 4-chloromethyl-5-cyclopropyl-3-(2,6-dichlorophenyl)isoxazole (145 mg, 0.48 mmol), tetrabutylammonium iodide (18 mg, 0.048 mmol) and DMF (2 mL), and then the mixture is heated and continues to react at 80 °C for 2 hr. Then the reaction mixture is cooled to room temperature, poured into water (20 mL), and extracted with ethyl acetate (20 mL × 2), then the organic later is washed with saturated salt solution (30 mL × 2), dried with anhydrous Na₂SO₄, rotated to dryness and purified by column chromatography(PE : EA = 5 : 1), so as to obtain a grey white solid of 70 mg, that is ethyl
2-(5-((5-cyclopropyl-3-(2,6-dichlorophenyl)isoxazol-4-yl)methoxy) isoindoline-2-yl) benzo[*d*]thiazole-6-carboxylate with a yield of 49%.
Spectrum is: ¹H-NMR (400 MHz, CDCl₃) *δ*: 8.38(d, J = 1.6 Hz, 1H), 8.05(dd, J = 1.6 Hz, 8.8 Hz, 1H), 7.64(d, J = 8.8Hz, 1H), 7.44-7.42(m, 2H), 7.37-7.33(m, 1H), 7.20(d, J = 8.0 Hz, 1H), 6.82-6.79(m, 2H), 4.89-4.86(m, 6H), 4.41(q, J = 7.2 Hz, 2H), 2.63(m, 1H), 1.43(t, J = 7.2 Hz, 3H), 1.31(m, 2H), 1.17(m, 2H).

### (9) Preparation of 2-(5-((5-cyclopropyl-3-(2,6-dichlorophenyl)isoxazol-4-yl)methoxy)isoindolin-2-yl) benzo[d]thiazole-6-carboxylic acid.

Ethyl 2-(5-((5-cyclopropyl-3-(2,6-dichlorophenyl)isoxazol-4-yl)methoxy) isoindolin-2-yl) benzo[*d*]thiazole-6-carboxylate (65 mg, 0.11 mmol) is dissolved in THF (2 mL), methanol (2 mL) and water (1 mL), followed by adding lithium hydroxide monohydrate (23 mg, 0.55 mmol), and the reaction mixture is stirred at room temperature and reacts overnight, then rotated to dryness, followed by adding water (10 mL) and adjusting pH to approximately 3 with 1N HCl, and then the mixture is filtered and rotated to dryness, so as to obtain a grey white solid of 28 mg, that is 2-(5-((5-cyclopropyl-3-(2,6-dichlorophenyl)isoxazol-4-yl)methoxy)isoindolin-2-yl) benzo[*d*]thiazole-6-carboxylic acid with a yield of 45%.
Spectrum is: ¹H-NMR (400 MHz, CDCl₃) *δ*: 8.42(d, *J* = 1.6 Hz, 1H), 7.88(dd, *J* = 1.6 Hz, 8.4 Hz, 1H), 7.64-7.62(m, 2H), 7.56-7.52(m, 2H), 7.26(d, *J* = 8.4 Hz, 1H), 6.91(d, *J* = 2.0 Hz, 1H), 6.77(dd, *J* = 2.0 Hz, 8.4 Hz, 1H), 4.80(s, 2H), 4.79(m, 4H), 2.44(m, 1H), 1.19-1.13(m, 4H).

### Example 23 2-(6-((5-cyclopropyl-3-(2,6-dichlorophenyl)isoxazol-4-yl)methoxy)-3,4-dihydroisoquinolin-2(1H) -yl)benzo[d]thiazole-6-carboxylic acid

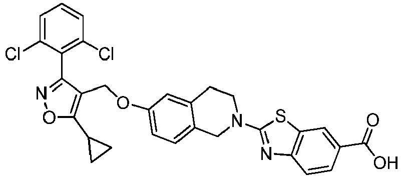

is prepared as the following synthetic scheme:

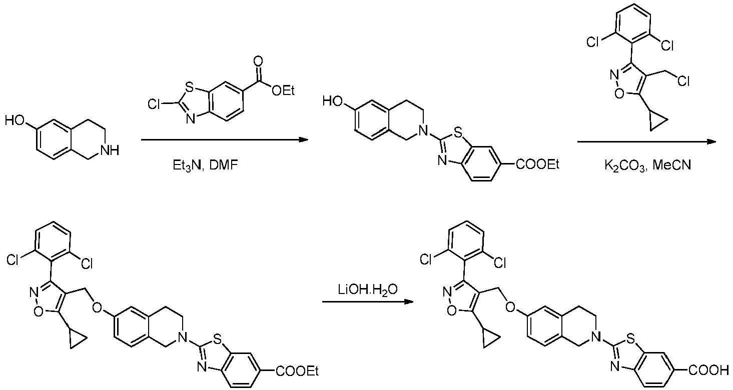

### (1) Preparation of ethyl 2-(6-hydroxy-3,4-dihydroisoquinolin-2(1H)-yl)benzo[d]thiazole-6-carboxylate.

1,2,3,4-tetrahydroisoquinolin-6-ol (100 mg, 0.670 mmol) and ethyl
2-chlorobenzo[*d*]thiazole-6-carboxylate (178 mg, 0.737 mmol) are dissolved in 5 mL of DMF, followed by adding triethylamine (186 µl, 1.34 mmol), and the reaction mixture reacts at 100 °C for 1 hr. Then the reaction mixture is cooled to room temperature, followed by adding 15 mL of water and 10 mL of petroleum ether, the mixture is stirred well and filtered, successively washed with water and petroleum ether, vacuum dried, so as to obtain a light yellow solid of 200 mg, that is ethyl 2-(6-hydroxy-3,4-dihydroisoquinolin-2(1*H*)-yl)benzo[*d*]thiazole-6-carboxylate with a yield of 84%.
Spectrum is: ¹H-NMR (400 MHz, DMSO) *δ*: 9.35(s, 1H), 8.41(d , *J* = 1.6 Hz, 1H), 7.88(dd, *J* = 8.4 Hz, 1.6 Hz, 1H), 7.51(d , *J* = 8.4 Hz , 1H), 7.10(d , *J* = 8.0 Hz, 1H), 6.66(m, 1H), 6.63(m, 1H), 4.69(s , 2H), 4.30(q , *J*=7.2 Hz , 2H), 3.81(t, *J*=5.6 Hz , 2H), 2.90(t, *J* = 5.6 Hz, 2H), 1.32(t, *J*=7.2 Hz, 2H).

### (2) Preparation of ethyl 2-(6-hydroxy((5-cyclopropyl-3-(2,6-dichlorophenyl)isoxazol-4-yl)methoxy)-3,4-dihdroisoquinolin -2(1H)-yl)benzo[d]thiazole-6-carboxylate.

Ethyl 2-(6-hydroxy-3,4-dihydroisoquinolin-2(1*H*)-yl)benzo[*d*]thiazole-6-carboxylate (90 mg, 0.254 mmol) and 4-(chloromethy)-5-cyclopropyl-3-(2,6-dichlorophenyl)isoxazole (90 mg, 0.305 mmol) are dissolved in 5 mL of acetonitrile, followed by adding potassium carbonate (75 mg, 0.508 mmol) and catalytic amount of potassium iodide, and the reaction mixture reacts at 85 °C with a reflux for 16 hrs. Then the reaction mixture is cooled to room temperature, rotated to remove solvent, dissolved in 15 mL of DCM, filtered, rotated to dryness and purified by column chromatography(PE : EA = 5 : 1), so as to obtain a white solid of 130 mg, that is ethyl 2-(6-((5-cyclopropyl-3-(2,6-dichlorophenyl)isoxazol-4-yl)methoxy)-3,4-dihdroisoquinolin-2(1*H*)-yl)benzo[*d*]thiazole-6-carboxylate with a yield of 83%.
Spectrum is: ¹H NMR (400 MHz, CDCl₃) *δ*: 8.35 (d*, J* = 1.6 Hz, 1H), 8.03 (dd, *J*= 8.4 Hz, 1.6 Hz, 1H), 7.58 (d, *J*=8.4 Hz, 1H), 7.42 (m, 2H), 7.33 (m, 1H), 7.09 (d, *J*=8.4 Hz, 1H), 6.66 (dd, *J*=8.4 Hz, 2.4Hz, 1H),6.63 (d, *J*=2.4 Hz, 1H), 4.82 (s, 2H), 4.77 (s, 2H), 4.40 (q, *J*=7.2 Hz, 2H), 3.89 (t, *J*=5.6 Hz, 2H), 2.97 (t, *J*=5.6 Hz, 2H), 2.23-2.16 (m, 1H), 1.42 (t, *J*=7.2 Hz, 3H), 1.31-1.28 (m, 2H), 1.18-1.12 (m, 2H).

### (3) Preparation of 2-(6-((5-cyclopropyl-3-(2,6-dichlorophenyl)isoxazol-4-yl)methoxy)-3,4-dihydroisoquinolin-2(1H) -yl)benzo[d]thiazole-6-carboxylic acid.

Ethyl
2-(6-((5-cyclopropyl-3-(2,6-dichlorophenyl)isoxazol-4-yl)methoxy)-3,4-dihdroisoquinolin-2(1*H*)-yl)benzo[*d*]thiazole-6-carboxylate (125 mg, 0.202 mmol) is dissolved in 2mL of THF and 2mL of methanol, followed by adding lithium hydroxide monohydrate (42 mg, 1.010 mmol) and water (1 mL), and the reaction mixture reacts at room temperature for 24 hrs, then rotated to remove the solvent, followed by adding water (19 mL), adjusting its pH to approximately 3∼4 with 1N HCl, then the mixture is filtered, washed and vacuum to dryness, so as to obtain a white solid of 105 mg, that is
2-(6-((5-cyclopropyl-3-(2,6-dichlorophenyl)isoxazol-4-yl)methoxy)-3,4-dihydroisoquinolin-2(1*H*) -yl)benzo[*d*]thiazole-6-carboxylic acid with a yield of 88%.
Spectrum is: ¹H NMR (400 MHz, DMSO) *δ*: 8.38(d, *J* = 1.6 Hz, 1H), 7.86(dd , *J* = 8.4 Hz, 1.6 Hz, 1H), 7.62-7.60(m, 2H), 7.55-7.51(m, 1H), 7.49(d, *J* = 8.4 Hz, 1H), 7.15(d, *J* = 8.4 Hz, 1H), 6.69-6.65(m, 2H), 4.85(s, 2H), 4.70(s, 2H), 3.80(t, *J* = 5.6Hz, 2H), 2.90(t, *J* = 5.6 Hz, 2H), 2.43(m, 1H), 1.18-1.11(m, 4H).

### Example 24 2-(7-((5-cyclopropyl-3-(2,6-dichlorophenyl)isoxazol-4-yl)methoxy)-3,4-dihydroisoquinolin-2(1H) -yl)benzo[d]thiazole-6-carboxylic acid is prepared.

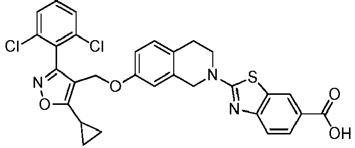

### (1) Preparation of 7-nitro-1,2,3,4-tetrahydroiso-quinoline hydrochloride.

1,2,3,4-tetrahydroisoquinoline (2.5 mL, 2.0 mmol) is dripped to H₂SO₄ (10 mL, 18.0 mmol) at 0 °C in 10 min, after that potassium nitrate (2.16 g, 2.14 mmol) is added in batches, and the temperature is increased to room temperate, then the mixture continues to react for 16 hrs. And then the reaction mixture is poured into ice water (50 mL), washed with ethyl acetate (30 mL × 2), and pH of the aqueous layer is adjusted to approximately 9∼10 with ammonium hydroxide, then the aqueous layer is extracted with ethyl acetate (50 mL × 2), dried with anhydrous Na₂SO₄ and rotated to dryness. The resulted product is dissolved in ethyl acetate (20 mL) and its pH is adjusted to approximately 4∼5 with HCl dissolved in ethyl acetate solution, then the mixture is filtered and washed, dried, to obtain a yellow solid of 1.8 g, that is 7-nitro-1,2,3,4-tetrahydroiso-quinoline hydrochloride with a yield of 42%.
Spectrum is: ¹H NMR (400 MHz, DMSO) *δ*: 9.91(s, 2H), 8.20(d, *J* = 1.6 Hz, 1H), 8.10(dd, *J* = 8.4 Hz, 1.6 Hz, 1H), 7.52(d, *J* = 8.4 Hz, 1H), 4.37(s, 2H), 3.38(t, *J* = 6.0 Hz, 2H), 3.15(t, *J* = 6.0 Hz, 2H).

### (2) Preparation of 7-amino-1,2,3,4-tetrahydroiso-quinoline.

7-nitro-1,2,3,4-tetrahydroiso-quinoline hydrochloride (1.8 g, 8.4 mmol) is dissolved in water(30 mL), followed by adding potassium carbonate (3.5 g, 25.4 mmol), and the reaction mixture is stirred at room temperature for 30 min, then extracted with ethyl acetate (20 mL × 3), dried with anhydrous Na₂SO₄, filtered and rotated to dryness, so as to obtain a brown solid. The solid is dissolved in methanol (20 mL), followed by adding 10% Pd/C (220 mg), and the reaction mixture reacts at room temperature for 16 hrs under the hydrogen protection. Then the resulting substance is filtered with sillceousearth, washed and rotated to remove the solvent, so as to obtain a yellow solid compound of 1.2 g, that is 7-amino-1,2,3,4-tetrahydroiso-quinoline with a yield of 96%. The product is directly used for the next reaction without purification.

### (3) Preparation of 1,2,3,4-tetrahydroiso-quinolin-7-ol.

7-amino-1,2,3,4-tetrahydroiso-quinoline (1.2 g, 8.1 mmol) is dissolved in water (15 mL), followed by adding H₂SO₄ (1.8 mL, 32.4 mmol) at 0 °C, then dripping sodium nitrite (670 mg, 9.7 mmol) in aqueous solution (10 mL) in 15 min, and then the mixture is heated to react with a reflux for 1.5 hr at 120 °C. Then the reaction mixture is cooled to room temperature, and its pH is adjusted to 8 by adding sodium bicarbonate solid in several times. Then, the mixture is filtered, and then the filtrate is extracted with DCM (20 mL × 3), dried with anhydrous Na₂SO₄, filtered, and rotated to obtain a reddish brown oily matter of 185 mg, that is 1,2,3,4-tetrahydroiso-quinolin-7-ol with a yield of 15%. m/z(M+H⁺) of the product is: 150.1.

### (4) Preparation of 2-(7-((5-cyclopropyl-3-(2,6-dichlorophenyl)isoxazol-4-yl)methoxy)-3,4-dihydroisoquinolin-2(1H) -yl)benzo[d]thiazole-6-carboxylic acid.

The synthesis method refers to the method of Example 23, wherein
1,2,3,4-tetrahydroiso-quinolin-7-ol is used to replace 1,2,3,4-tetrahydroiso-quinolin-6-ol. ¹H NMR (400 MHz, DMSO) *δ*: 8.42(m, 1H), 8.10(d, J = 8.8 Hz, 1H), 7.61(d, J = 8.8 Hz, 1H), 7.44-7.42(m, 2H), 7.36-7.32(m, 1H), 7.07(d, J = 8.4 Hz, 1H), 6.71(m, 1H), 6.66(m, 1H), 4.82(s, 2H), 4.79(s, 2H, 3.88(m, 2H), 2.97(m, 2H), 2.20(m, 1H), 1.31(m, 2H), 1.17(m, 2H).

### Example 25 2-(5-((5-cyclopropyl-3-(2,6-dichlorophenyl)isoxazol-4-yl)methoxy)hexahydro-1H-isoindol-2(3H) -yl)benzo[d]thiazole-6-carboxylic acid is prepared.

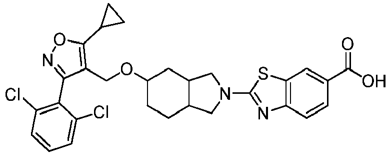

### (1) Preparation of 2,3,3a,4,7,7a-hexahydro-1H-isoindole.

Lithium aluminum hydride (4.0 g, 105 mmol) is added by division to anhydrous THF (100 mL) at 0 °C under argon protection, followed by dripping 3a,4,7,7a-tetrahydro-1H-isoindole-1,3(2*H*)-dione (5.0 g, 33 mmol) dissolved in anhydrous THF in 30 min, then the temperature is increased to 40 °C and the reaction mixture reacts for 16 hrs, and then water (12 mL) is dripped into the mixture in an ice bath to quench the reaction, followed by adding 40 g of anhydrous Na₂SO₄, and the resulting mixture is stirred for 10 min, filtered with sillceousearth, washed with DCM and the filtrate is rotated to dryness, so as to obtain a brown oily matter of 3.2 g, that is 2,3,3a,4,7,7a-hexahydro-1*H*-isoindole with a yield of 80%. The product is directly used for the next reaction without purification.

### (2) Preparation of tert-butyl 3a,4,7,7a-tetrahydro-1H-isoindole-2(3H)-carboxylate.

2,3,3a,4,7,7a-hexahydro-1*H*-isoindole (3.2 g, 26 mmol) is dissolved in THF (80 mL),then triethylamine (5.5 mL, 39 mmol) is added, then Boc anhydride (7.3 mL, 31 mmol) is dripped at 0 °C in 15 min. Then the temperature is naturedly increased to room temperate, and the reaction mixture reacts for 16 hr. Then the reaction mixture is poured into water (150 mL), and extracted with ethyl acetate (150 mL × 2). Subsequently, the organic layer is washed with saturated salt solution (150 mL) and dried with anhydrous Na₂SO₄, then filtered and rotated to dryness, then purified by column chromatography (PE : EA = 10 : 1), so as to obtain a brown oily matter of 4.65 g, that is *tert*-butyl 3a,4,7,7a-tetrahydro-1*H*-isoindole-2(3*H*)-carboxylate with a yield of 80%.
Spectrum is: ¹H NMR (400 MHz, CDCl₃) *δ*: 5.65(s, 2H), 3.48(m, 2H), 3.44-3.37(m, 2H), 3.17(m, 1H), 3.08(m, 1H), 2.34-2.25(m, 2H), 1.91(m, 2H), 1.46(s, 9H).

### (3) Preparation of tert-butyl 1 5-hydroxyhexahydro-1H-isoindole-2(3H)-carboxylate.

*Tert*-butyl 3a,4,7,7a-tetrahydro-1*H*-isoindole-2(3*H*)-carboxylate (500 mg, 2.22 mol) is dissolved in anhydrous THF (5 mL), followed by dripping 1M borane in THF solution (2.22 mL) at 0 °C in 15 min, after that the temperature is increased to room temperature , the mixture continues to react for 16 hrs. Then methanol (1.25 mL), 3M NaOH solution (0.83 mL) and 30% hydrogen peroxide (0.83 mL) are added successively at 0 °C. Subsequently, the temperature is increased to 60 °C and the mixture continues to react for 1.5 hr. Then the reaction mixture is cooled to room temperature, followed by adding ethyl acetate (15 mL) to dilute the solution. Then the mixture is washed with saturated salt solution (15 mL), dried with anhydrous Na₂SO₄ and filtered, subsequently rotated to dryness and purified by column chromatography (PE : EA = 2 : 1), so as to obtain a white solid of 390 mg, which is *tert*-butyl 5-hydroxyhexahydro-1*H*-isoindole-2(3*H*)-carboxylate with a yield of 72%.
Spectrum is: ¹H NMR (400 MHz, CDCl₃) *δ*: 3.90(m, 1H), 3.38(m, 2H), 3.19(m, 2H), 2.14(m, 1H), 1.86(m, 4H), 1.56(m, 2H), 1.48(s, 9H), 1.38(m, 2H).

### (4) Preparation of 2-(5-((5-cyclopropyl-3-(2,6-dichlorophenyl)isoxazol-4-yl)methoxy)hexahydro-1H-isoindol-2(3H) -yl)benzo[d]thiazole-6-carboxylic acid.

The synthesis method refers to the method of Example 2, wherein *tert*-butyl
5-hydroxyhexahydro-1*H*-isoindole-2(3*H*)-carboxylate is used to replace *tert-butyl*
2-hydroxy-7-azaspiro[3.5]nonan-7-carboxylate.
Spectrum is: ¹H NMR (400 MHz, DMSO) *δ*: 8.34(d, *J* = 1.6 Hz, 1H), 7.84(dd, *J* = 1.6 Hz, 8.4 Hz, 1H), 7.67-7.64(m, 2H), 7.59-7.55(m, 1H), 7.43(d, *J* = 8.4 Hz, 1H), 4.26(m, 2H), 3.58-3.36 (m, 5H), 2.33(m, 1H), 2.22(m, 2H), 1.56(m, 2H), 1.47(m, 2H), 1.23(m, 2H), 1.16(m, 2H), 1.10(m, 2H).

### Example 26 2-(5-((5-cyclopropyl-3-(2,6-dichlorophenyl)isoxazol-4-yl)methoxy)hexahydrocyclopenta[c]pyrrol -2(1H)-yl)benzo[d]thiazole-6-carboxylic acid is prepared.

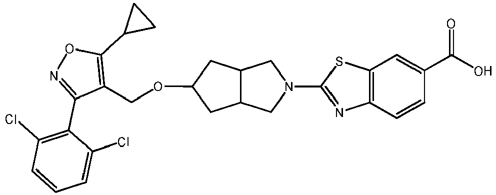

### (1) Preparation of 2,2'-(1-(tert-butoxy)pyrrolidine-3,4-diyl)diacetic acid.

Potassium permanganate (1.49 g, 9.39 mmol) is dissolved in water(15 mL) and the solution is cooled to 5 °C, followed by slowly dripping the solution of *tert*-butyl
3a,4,7,7a-tetrahydro-1*H*-isoindole-2(3*H*)-carboxylate (700 mg, 3.13 mmol) dissolved in acetone (5 mL). After that the reaction continues to react at 5 °C for 3 hrs, then saturated sodium bisulfate solution is added to react with excess potassium permanganate, then pH is adjusted to 2 with IN HCl. Then the mixture is extracted with dichloromethane (30 mL × 3), washed with saturated salt solution (30 mL × 3), rotated to dryness, so as to obtain a white foamed solid, that is 2,2'-(1-(*tert*-butoxy)pyrrolidine-3,4-diyl)diacetic acid, which is used for the next step without purification.

### (2) Preparation of tert-butyl 5-oxo-hexahydrocyclopenta[c]pyrrole-2(1H)-carboxylate.

2,2'-(1-(*tert*-butoxy) pyrrolidine-3,4-diyl)diacetic acid (900 mg, 3.13 mmol) is dissolved in acetic anhydride (15 mL), followed by adding sodium acetate (231 mg, 2.82 mmol), and the reaction mixture reacts at 120 °C for 2 hr. Then the reaction solution is cooled to room temperature, filtered, rotated to dryness and purified by column chromatography (PE : EA = 5 : 1), so as to obtain a light yellow oily matter of 390 mg, that is *tert*-butyl
5-oxo-hexahydrocyclopenta[*c*]pyrrole-2(1*H*)-carboxylate. An overall yield of the two steps is 55%.
Spectrum is: ¹H NMR (400 MHz, CDCl₃) *δ*: 3.67(m, 2H), 3.23(m, 2H), 2.94(m, 2H), 2.50(m, 2H), 2.17(m, 2H), 1.48(s, 9H).

### (3) Preparation of tert-butyl 5-hydroxy-hexahydrocyclopenta[c]pyrrole-2(1H)-carboxylate.

*Tert*-butyl 5-oxo-hexahydrocyclopenta[*c*]pyrrole-2(1*H*)-carboxylate (390 mg, 1.73 mmol) is dissolved in methanol(10 mL) and cooled to 0 °C, followed by adding sodium borohydride (132 mg, 3.46 mmol) in batches, and then the mixture reacts at room temperature for 2 hrs, then rotated to dryness. Then water (20 mL) is added, the mixture is then extracted with ethyl acetate (20 mL × 2), washed with saturated salt solution (20 mL × 2), then dried with anhydrous Na₂SO₄, rotated to dryness and purified by column chromatography (PE:EA = 2:1), so as to obtain a light yellow slurry of 336 mg, that is *tert*-butyl 5-hydroxy-hexahydrocyclopenta[*c*]pyrrole-2(1*H*)-carboxylate with a yield of 85%.
Spectrum is: ¹H NMR (400 MHz, CDCl₃) *δ*: 4.32(m, 1H), 3.51(m, 2H), 3.36(m, 2H), 2.62(m, 2H), 2.19(m, 2H), 1.76(d, J = 4.8 Hz, 1H), 1.56-1.47(m, 11H).

### (4) Preparation of 2-(5-((5-cyclopropyl-3-(2,6-dichlorophenyl)isoxazol-4-yl)methoxy)hexahydrocyclopenta[c]pyrrol -2(1H)-yl)benzo[d]thiazole-6-carboxylic acid.

The synthesis method refers to the method of Example 2, and *tert*-butyl
5-hydroxyhexadrocyclopenta[*c*]pyrrole-2(1*H*)-carboxylate is used to replace *tert-butyl*
2-hydroxy-7- azaspiro [3.5]nonan-7-carboxylate.
Spectrum is: ¹H-NMR (400 MHz, CDCl₃) *δ*: 8.40(m, 1H), 8.09(d, J = 8.4 Hz, 1H), 7.61(d, J = 8.8 Hz, 1H), 7.38-7.36(m, 2H), 7.26(m, 1H), 4.23(s, 2H), 3.96(m, 1H), 3.78(m, 2H), 3.47(m, 2H), 2.79(m, 2H), 2.08(m, 3H), 1.48(m, 2H), 1.22(m, 2H), 1.07(m, 2H).

### Example 27 2-(5-((5-cyclopropyl-3-(2,6-dichlorophenyl)isoxazol-4-yl)methoxy)hexahydrocyclopenta[c]pyrrol -2(1H)-yl)benzo[d]thiazole-6-carboxylic acid is prepared.

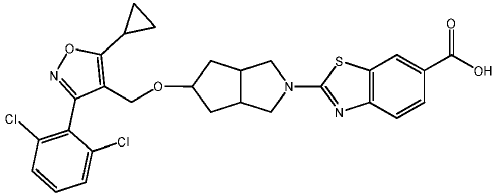

The synthesis method refers to the method of Example 26, and methyl
2-chloro-benzo[*d*]oxazole-6-carboxylate is used to replace ethyl
2-chlorobenzo[*d*]thiazole-6-carboxylate.
Spectrum is: ¹H-NMR (400 MHz, CDCl₃) *δ*: 8.05-8.00(m, 2H), 7.41-7.36(m, 3H), 7.28-7.24(m, 1H), 4.24(s, 2H), 3.96(m, 1H), 3.85(m, 2H), 3.53(m, 2H), 2.76(m, 2H), 2.07(m, 3H), 1.50(m, 2H), 1.23(m, 2H), 1.08(m, 2H).

### Example 28 2-(9-((5-cyclopropyl-3-(2,6-dichlorophenyl)isoxazol-4-yl)methoxy)-3-azazbicyclo[3.3.1]nonan-3-yl)benzo[d]thiazole-6-carboxylic acid is prepared

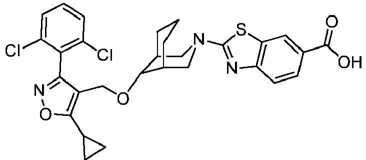

### (1) Preparation of 3-benzyl-3-azabicyclo[3.3.1]nonan-9-one.

Benzylamine (2.14 mL, 19.6 mmol) is dissolved in tertiary butanol (5 mL), followed by successively adding paraformaldehyde (1.18 g, 39.2 mmol), HCl (1.8 mL, 21.6 mmol) and cyclohexanone (5.26 mL, 50.9 mmol) at 0 °C.Then the mixture is heated to 80 °C to react with a reflux for 3 hrs, the mixture is then rotated to remove the solvent, then water (50 mL) and ethyl acetate(50 mL) is added to form layering, and pH of the aqueous layer is adjusted to approximately 9∼10 with 4M NaOH, then the mixture is extracted with ethyl acetate (30 mL × 2) and the organic layer is washed with saturated salt solution(20 mL), dried with anhydrous Na₂SO₄, then filtered and purified by column chromatography(PE : EA = 100 : 1), so as to obtain a yellow oily substance of 1.28 g, that is 3-benzyl-3-azabicyclo[3.3.1]nonan-9-one with a yield of 28%. Product m/z[M+H]⁺ is: 230.2.

### (2) Preparation of 3-benzyl-3-azabicyclo[3.3.1]nonan-9-ol.

3-benzyl-3-azabicyclo[3.3.1]nonan-9-one (1.28 g, 5.5 mmol) is dissolved in methanol (15 mL), sodium borohydride(0.42 g, 11.0 mmol) is added in batches in an ice bath. Then the temperature is increased to room temperature, and the mixture reacts for 1 hr and rotated to remove the solvent, and then water (15 mL) is added. Subsequently, the resulting mixture is extracted with ethyl acetate (15 mL × 2), and the organic layer is dried with anhydrous Na₂SO₄, then filtered, rotated to dryness and purified by column chromatography (PE : EA = 10 : 1), so as to obtain a white solid of 900 mg, that is 3-benzyl-3-azabicyclo[3.3.1]nonan-9-ol.

### (3) Preparation of 3-azabicyclo[3.3.1]nonan-9-ol

3-benzyl-3-azabicyclo[3.3.1]nonan-9-ol (900 mg, 3.85 mmol) is dissolved in methanol (15 mL), followed by adding Pd/C (180 mg), and the reaction mixture reacts at room temperature for 16 hrs under the hydrogen protection, then the mixture is filtered with sillceousearth, washed and rotated to dryness, so as to obtain a white solid of 640 mg, that is 3-azabicyclo[3.3.1]nonan-9-ol, which is directly used in the next reaction without any purification.

### (4) Preparation of tert-butyl 9-hydroxy-3- azabicyclo[3.3.1]nonan-3-carboxylate.

3-azabicyclo[3.3.1]nonan-9-ol (640 mg, 4.5 mmol) is dissolved in dichloromethane (20 mL), followed by adding Boc anhydride (1.24 mL, 5.4 mmol) and triethylamine (0.94 mL, 6.75 mmol), and the reaction mixture reacts at room temperature for 1 hr, then washed with water(20 mL) to form layering, and the organic layer is dried with anhydrous Na₂SO₄, subsequently filtered and rotated to dryness, then purified by column chromatography (PE : EA = 5 : 1), so as to obtain a white solid product, that is *tert*-butyl 9-hydroxy-3- azabicyclo[3.3.1]nonan-3-carboxylate with a yield of 44%. The m/z[M+H]⁺ of the product is: 242.2.

### (5) Preparation of 2-(9-((5-cyclopropyl-3-(2,6-dichlorophenyl)isoxazol-4-yl)methoxy)-3-azazbicyclo[3.3.1]nonan-3-yl)benzo[d]thiazole-6-carboxylic acid.

The synthetic method refers to the method of Example 2, and *tert*-butyl 9-hydroxy-3-azabicyclo[3.3.1]nonan-3-carboxylate is used to replace *tert-butyl*
2-hydroxy-7-azaspiro[3.5]nonan-7-carboxylate.
Spectrum is: ¹H NMR (400 MHz, DMSO) *δ*: 8.33(d, J = 1.2 Hz, 1H), 7.84(dd, J = 1.2 Hz, 8.0 Hz, 1H), 7.65-7.63(m, 2H), 7.58-7.54(m, 1H), 7.43(m, *J* = 8.4 Hz, 1H), 4.33(s, 2H), 4.02(m, 1H), 3.56(m, 4H), 2.36(m, 1H), 1.94(m, 2H), 1.47(m, 3H), 1.35(m, 2H), 1.23(m, 1H), 1.17-1.15(m, 4H).

### Example 29 2-(5-((5-cyclopropyl-3-(2,6-dichlorophenyl)isoxazol-4-yl)methoxy)-6-azaspiro[3.4]octan-6-yl) benzofuran-2-carboxylic acid is prepared.

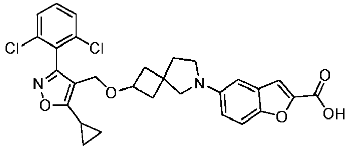

The synthetic method refers to the method of Example 10, and ethyl
5-bromobenzofuran-2-carboxylate is used to replace methyl 3-bromobenzoate.
Spectrum is: ¹H-NMR (400 MHz, CDCl₃) *δ*: 7.54(s, 1H), 7.47-7.43(m, 3H), 7.38-7.33(m, 1H), 6.77(m, 1H), 6.64(m, 1H), 4.22(s, 2H), 3.97(m, 1H), 3.30(m, 2H), 3.22(m, 2H), 2.17(m, 3H), 1.95(m, 2H), 1.82(m, 2H), 1.29(m, 2H), 1.15(m, 2H).

### Example 30 6-(2-((5-cyclopropyl-3-(2,6-dichlorophenyl)isoxazol-4-yl)methoxy)-6-azaspiro[3.4]octan-6-yl) benzothiophene-2-carboxylic acid is prepared.

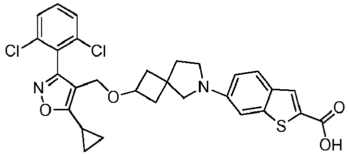

The synthetic method refers to the method of Example 10, and ethyl
6-bromobenzothiophene-2-carboxylate is used to replace methyl 3-bromobenzoate.
Spectrum is: ¹H-NMR (400 MHz, CDCl₃) *δ*: 8.01(s, 1H), 7.70(d, *J* = 8.8 Hz, 1H), 7.46-7.44(m, 2H), 7.39-7.33(m, 1H), 6.83(m, 1H), 6.70(m, 1H), 4.22(s, 2H), 3.99(m, 1H), 3.37-3.26(m, 4H), 2.17(m, 3H), 1.96(m, 2H), 1.81(m, 2H), 1.29(m, 2H), 1.15(m, 2H).

### Example 31 6-(2-((5-cyclopropyl-3-(2,6-dichlorophenyl)isoxazol-4-yl)methoxy)-6-azaspiro[3.4]octan-6-yl) benzo[d]isothiazole-2-carboxylic acid is prepared.

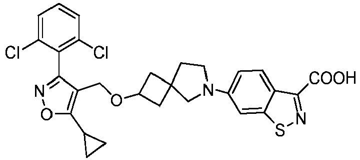

The synthetic method refers to the method of Example 10, and methyl
6-bromobenzo[*d*]isothiazole-2-carboxylate is used to replace methyl 3-bromobenzoate.
Spectrum is: ¹H-NMR (400 MHz, CDCl₃) *δ*: 8.64(s, 1H), 7.39(m, 3H), 6.60(m, 2H), 4.18(s, 2H), 3.93(m, 1H), 3.18(m, 4H), 2.14(m, 3H), 1.88(m, 2H), 1.74(m, 2H), 1.28(m, 2H), 1.14(m, 2H).

### Example 32 5-(2-((5-cyclopropyl-3-(2,6-dichlorophenyl)isoxazol-4-yl)methoxy)-6-azaspiro[3.4]octan-6-yl)-1-methyl-1H-indole-2-carboxylic acid is prepared.

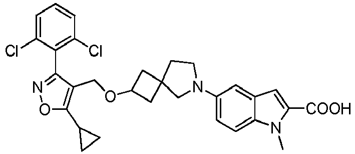

### (1) Preparation of methyl 5-(2-((5-cyclopropyl-3-(2,6-dichlorophenyl)isoxazol-4-yl)methoxy)-6-azaspiro[3.4]octan-6-yl)-1-methyl-1H-indole-2-carboxylate.

4-((6-azaspiro[3.4]octan-2-oxy)methyl)-5-cyclopropyl-3-(2,6-dichlorophenyl)isoxazole (100 mg, 0.24 mmol) is dissolved in methylbenzene (5 mL), followed by adding methyl 1-methyl-6-bromo-1*H*-indole-3-carboxylate (80 mg, 0.28 mmol), Pd₂(dba)₃ (12 mg, 0.012 mmol), 2-dicyclohexylphosphino-2,4,6-triisopropylbiphenyl (12 mg, 0.024 mmol) and cesium carbonate (298 mg, 0.91 mmol), and the reaction mixture reacts overnight at 100 °C under the protection of argon gas. Then the reaction solution is cooled to room temperature, it is filtered with sillceousearth, rotated to dryness and purified by column chromatography (petroleum ether : ethyl acetate = 4 : 1), so as to obtain a yellow oily matter of 65 mg, that is methyl 5-(2-((5-cyclopropyl-3-(2,6-dichlorophenyl)isoxazol-4-yl)methoxy)-6-azaspiro[3.4]octan-6-yl)-1-methyl-1*H*-indole-2-carboxylate with a yield of 47%.
Spectrum is: ¹H NMR (400 MHz, CDCl₃) *δ*: 7.46-7.43(m, 2H), 7.38-7.33(m, 1H), 7.26(m, 1H), 7.13(s, 1H), 6.81(m, 1H), 6.65(m, 1H), 4.21(s, 2H), 4.04(s, 3H), 3.98(m, 1H), 3.91(s, 3H), 3.31(m, 2H), 3.22(m, 2H), 2.17(m, 3H), 1.94(m, 2H), 1.80(m, 2H), 1.29(m, 2H), 1.15(m, 2H).

### (2) Preparation of 5-(2-((5-cyclopropyl-3-(2,6-dichlorophenyl)isoxazol-4-yl)methoxy)-6-azaspiro[3.4]octan-6-yl)-1-methyl-1H-indole-2-carboxylic acid.

Methyl
6-(2-((5-cyclopropyl-3-(2,6-dichlorophenyl)isoxazol-4-yl)methoxy)-6-azaspiro[3.4]octan-6-yl)-1-methyl-1H-indole-2-carboxylate (65 mg, 0.11 mmol) is dissolved in THF (2 mL), methanol (2 mL) and water (1 mL), followed by adding lithium hydroxide monohydrate (23 mg, 0.55 mmol), and the reaction mixture reacts overnight at room temperature, then rotated to dryness. Then water (15 mL) is added, pH of the mixture is adjusted to approximately 3 with IN HCl, then the mixture is filtered and dried, so as to obtain a white solid, that is
5-(2-((5-cyclopropyl-3-(2,6-dichlorophenyl)isoxazol-4-yl)methoxy)-6-azaspiro[3.4]octan-6-yl)-1-methyl-1*H*-indole-2-carboxylic acid with a yield of 81%.
Spectrum is: ¹H-NMR (400 MHz, CDCl₃) *δ*: 7.46-7.43(m, 2H), 7.38-7.34(m, 1H), 7.29(m, 2H), 6.87(m, 1H), 6.69(m, 1H), 4.22(s, 2H), 4.05(s, 3H), 3.97(m, 1H), 3.32(m, 2H), 3.25(m, 2H), 2.17(m, 3H), 1.95(m, 2H), 1.82(m, 2H), 1.29(m, 2H), 1.15(m, 2H).

### Example 33 6-(2-((5-cyclopropyl-3-(2,6-dichlorophenyl)isoxazol-4-yl)methoxy)-6-azaspiro[3.4]octan-6-yl)-1-methyl-1H-indole-3-carboxylic acid is prepared.

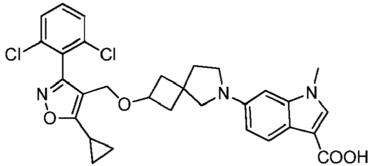

The synthetic method refers to the method of Example 32, and methyl
1-methyl-bromo-1*H*-indole-3-carboxylate is used to replace methyl
1-methyl-5-bromo-1*H*-indole-2-carboxylate.
Spectrum is: ¹H-NMR (400 MHz, CDCl₃) *δ*: 8.02(d, *J* = 8.8 Hz, 1H), 7.69(s, 1H), 7.46-7.43(m, 2H), 7.38-7.33(m, 1H), 6.64 (m, 1H), 6.30(s, 1H), 4.22(s, 2H), 3.99(m, 1H), 3.77(s, 3H), 3.35(m, 2H), 3.26(m, 2H), 2.18(m, 3H), 1.95(m, 2H), 1.82(m, 2H), 1.29(m, 2H), 1.15(m, 2H).

### Example 34 2-(2-((5-cyclopropyl-3-(2,6-dichlorophenyl)isoxazol-4-yl)methoxy)-6-azaspiro[3.4]octan-6-yl)qui nolin-6-carboxylic acid is prepared.

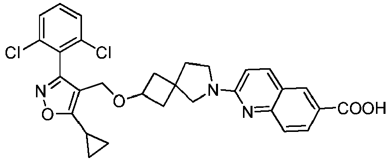

The synthetic method refers to the method of Example 10, and methyl 2-chloroquinolin-6-carboxylate is used to replace methyl 3-bromobenzoate.
Spectrum is: ¹H NMR (400 MHz, CDCl₃) *δ*: 9.12(m, 1H), 8.55(m, 2H), 8.28(m, 1H), 7.40(m, 2H), 7.37(m, 1H), 6.43(m, 1H), 4.16(s, 2H), 3.96-3.76(m, 5H), 2.20-2.02(m, 5H), 1.79(m, 2H), 1.23(m, 2H), 1.12(m, 2H).

### Example 35 5-(2-((5-cyclopropyl-3-(2,6-dichlorophenyl)isoxazol-4-yl)methoxy)-6-azaspiro[3.4]octan-6-yl)pyr azine-2-carboxylic acid is prepared.

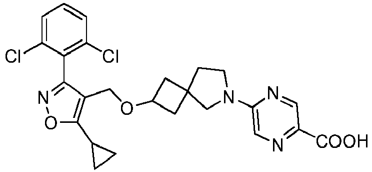

### (1) Preparation of methyl 5-(2-((5-cyclopropyl-3-(2,6-dichlorophenyl)isoxazol-4-yl)methoxy)-6-azaspiro[3.4]octan-6-yl)pyr azine-2-carboxylate.

4-((7-azaspiro[3.4]nonan-2-oxy)methyl)-5-cyclopropyl-3-(2,6-dichlorophenyl)isoxazole (100 mg, 0.24 mmol) is dissolved in DMF (5 mL), followed by adding methyl 5-chloropyrazine -2-carboxylate (45 mg, 0.26 mmol) and potassium carbonate (81 mg, 0.59 mmol), and the mixture is heated to 80 °C and continues to react for 2 hr. Then the reaction mixture is cooled down to room temperature, poured into water (20 mL), and extracted with ethyl acetate (15 mL × 2) and the organic layer is washed with saturated salt solution (20 mL × 2), then dried with anhydrous Na₂SO₄, filtered, rotated to dryness and purified by column chromatography (PE : EA = 1.5 : 1), so as to obtain a brown oily matter of 100 mg, that is methyl
5-(2-((5-cyclopropyl-3-(2,6-dichlorophenyl)isoxazol-4-yl)methoxy)-6-azaspiro[3.4]octan-6-yl)pyr azine-2-carboxylate with a yield of 81%.
Spectrum is: ¹H NMR (400 MHz, CDCl₃) *δ*: 8.81(m, 1H), 7.86(m, 1H), 7.46-7.44(m, 2H), 7.39-7.34(m, 1H), 4.21(s, 2H), 3.97(m, 4H), 3.58-3.44(m, 4H), 2.16(m, 3H), 1.98(m, 2H), 1.83(m, 2H), 1.29(m, 2H), 1.15(m, 2H).

### (2) Preparation of 5-(2-((5-cyclopropyl-3-(2,6-dichlorophenyl)isoxazol-4-yl)methoxy)-6-azaspiro[3.4]octan-6-yl)pyr azine-2-carboxylic acid.

Methyl
5-(2-((5-cyclopropyl-3-(2,6-dichlorophenyl)isoxazol-4-yl)methoxy)-6-azaspiro[3.4]octan-6-yl)pyr azine-2-carboxylate (100 mg, 0.19 mmol) is dissolved in THF (2 mL), methanol (2 mL) and water (1 mL), followed by adding lithium hydroxide monohydrate (40 mg, 0.95 mmol), and the reaction mixture reacts overnight at room temperature, then it is rotated to dryness. Then water(15 mL) is added, and subsequently pH of the mixture is adjusted to approximately 2 with IN HCl, and the mixture is then filtered and dried, so as to obtain a white solid of 73 mg, that is
5-(2-((5-cyclopropyl-3-(2,6-dichlorophenyl)isoxazol-4-yl)methoxy)-6-azaspiro[3.4]octan-6-yl)pyr azine-2-carboxylic acid with a yield of 75%.
Spectrum is: ¹H NMR (400 MHz, CDCl₃) *δ*: 8.86(s, 1H), 7.80(s, 1H), 7.46-7.43(m, 2H), 7.39-7.34(m, 1H), 4.21(s, 2H), 3.97(m, 1H), 3.51(m, 4H), 2.16(m, 3H), 1.97(m, 2H), 1.82(m, 2H), 1.28(m, 2H), 1.15(m, 2H).

### Example 36 6-(2-((5-cyclopropyl-3-(2,6-dichlorophenyl)isoxazol-4-yl)methoxy)-6-azaspiro[3.4]octan-6-yl)pyr idine-2-carboxylic acid is prepared.

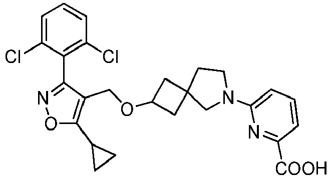

The synthetic method refers to the method of Example 35, and methyl
6-chloropyridine-2-carboxylate is used to replace methyl 5-chloropyrazine-2-carboxylate. ¹H NMR(400 MHz, CDCl₃)*δ*: 7.62(m, 1H), 7.46-7.37(m, 4H), 6.56(m, 1H), 4.22(s, 2H), 3.98(m, 1H), 3.49-3.35(m, 4H), 2.17(m, 3H), 1.96(m, 2H), 1.82(m, 2H), 1.27(m, 2H), 1.15(m, 2H)

### Example 37 4-(2-((5-cyclopropyl-3-(2,6-dichlorophenyl)isoxazol-4-yl)methoxy)-6-azaspiro[3.4]octan-6-yl)-3-f luorobenzoic acid is prepared.

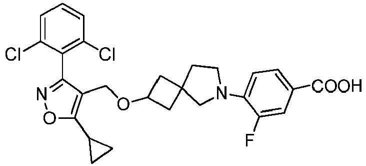

The synthetic method refers to the method of Example 10, and methyl 3-fluoro-4-bromobenzoate is just used to replace methyl 3-bromobenzoate.
Spectrum is: ¹H NMR (400 MHz, CDCl₃) *δ*: ¹H NMR (400 MHz, CDCl₃) *δ*: 7.75(m, 1H), 7.68(m, 1H), 7.45-7.43(m, 2H), 7.38-7.33(m, 1H), 6.51(m, 1H), 4.21(s, 2H), 3.96(m, 1H), 3.50-3.38(m, 4H), 2.16(m, 3H), 1.87(m, 2H), 1.78(m, 2H), 1.28(m, 2H), 1.15(m, 2H).

### Example 38 2-(2-((5-cyclopropyl-3-(2,6-dichlorophenyl)isoxazol-4-yl)methoxy)-6-azaspiro[3.4]octan-6-yl) benzo[d]thiazole-6-carboxylic acid is prepared.

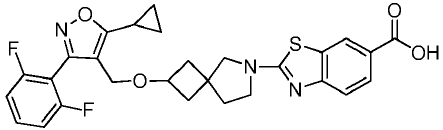

The synthetic method refers to the method of Example 8, and 2,6-difluorobenzaldehyde is used to replace 2,6-dichlorobenzaldehyde.
Spectrum is: ¹H NMR (400 MHz, CDCl₃) *δ*: 8.39(s, 1H), 8.08(d, *J* = 8.4 Hz, 1H), 7.60(d, *J* = 8.4 Hz, 1H), 7.46(m, 1H), 7.08-7.03(m, 2H), 4.29(s, 2H), 3.97(m, 1H), 3.59(m, 4H), 2.27-2.16(m, 3H), 2.02(m, 2H), 1.90(m, 2H), 1.28(m, 2H), 1.15(m, 2H).

### Example 39 2-(2-((5-cyclopropyl-3-(2,6-difluorophenyl)isoxazol-4-yl)methoxy)-6-azaspiro[3.4]octan-6-yl) benzo[d]oxazole-6-carboxylic acid is prepared.

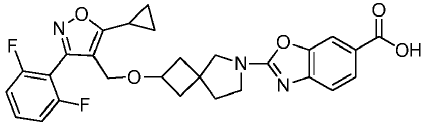

The synthetic method refers to the method of Example 14, and 2,6-difluorobenzaldehyde is used to replace 2,6-dichlorobenzaldehyde .
Spectrum is: ¹H NMR (400 MHz, CDCl₃) *δ*: 8.03(d, *J* = 8.4 Hz, 1H), 8.00(s, 1H), 7.46(m, 1H), 7.39(d, *J* = 8.4 Hz, 1H), 7.06(m, 2H), 4.29(s, 2H), 3.98(m, 1H), 3.66(m, 2H), 3.58(d, J = 11.2 Hz, 2H), 2.27-2.15(m, 3H), 1.98(m, 2H), 1.88(m, 2H), 1.27(m, 2H), 1.15(m, 2H).

### Example 40 2-(6-((5-cyclopropyl-3-(2,6-difluorophenyl)isoxazol-4-yl)methoxy)-3,4-dihydroisoquinolin-2(1H) -yl)benzo[d]thiazole-6-carboxylic acid is prepared.

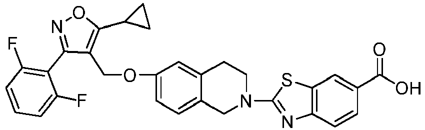

The synthetic method refers to the method of Example 23, and 2,6-difluorobenzaldehyde is used to replace 2,6-dichlorobenzaldehyde.
Spectrum is: ¹H NMR (400 MHz, CDCl₃) *δ*: 8.42(s, 1H), 8.10(m, 1H), 7.62(d, *J* = 8.4 Hz, 1H), 7.44(m, 1H), 7.10(d, *J* = 8.4 Hz, 1H), 7.03(m, 2H), 6.73(m, 1H), 6.64(m, 1H), 4.90(s, 2H), 4.78(s, 2H), 3.90(t, *J* = 5.6 Hz, 2H), 2.97(t, *J* = 5.6 Hz, 2H), 2.19(m, 1H), 1.28(t, m, 2H), 1.14(m, 2H).

### Example 41 2-(2-((5-cyclopropyl-3-(2-trifluoromethylphenyl)isoxazol-4-yl)methoxy)-6-azaspiro[3.4]octan-6-y l)benzo[d]thiazole-6-carboxylic acid is prepared.

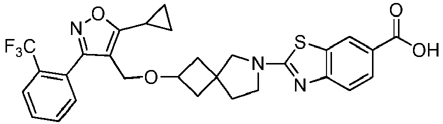

The synthetic method refers to the method of Example 8, and 2-(trifluoromethyl)benzaldehyde is used to replace 2,6-dichlorobenzaldehyde.
Spectrum is: ¹H NMR (400 MHz, CDCl₃) *δ*: 8.39(s, 1H), 8.08 (d, *J* = 8.4 Hz, 1H), 7.82(d, *J* = 7.6 Hz, 1H), 7.69-7.59(m, 3H), 7.49(m, 1H), 4.15(s, 2H), 3.96(m, 1H), 3.56(m, 4H), 2.24(m, 2H), 2.13(m, 1H), 2.03(m, 2H), 1.86(m, 2H), 1.27(m, 2H), 1.15(m, 2H).

### Example 42 2-(2-((5-cyclopropyl-3-(2-trifluoromethylphenyl)isoxazol-4-yl)methoxy)-6-azaspiro[3.4]octan-6-y l)benzo[d]oxazole-6-carboxylic acid is prepared.

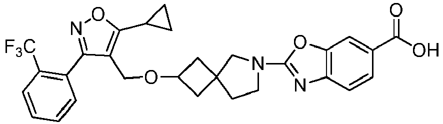

The synthetic method refers to the method of Example 14, and 2-(trifluoromethyl)benzaldehyde is used to replace 2,6-dichlorobenzaldehyde.
Spectrum is: ¹H NMR (400 MHz, CDCl₃) *δ*: 8.02(m, 2H), 7.82(d, *J* = 7.6 Hz, 1H), 7.64(m, 2H), 7.49(m, 1H), 7.39(m, 1H), 4.15(s, 2H), 3.96(m, 1H), 3.68(m, 2H), 3.58(d, *J* = 11.2 Hz, 2H), 2.23(m, 2H), 2.13(m, 1H), 1.99(m, 2H), 1.86(m, 2H), 1.28(m, 2H), 1.14(m, 2H).

### Example 43 2-(6-((5-cyclopropyl-3-(2-trifluoromethylphenyl)isoxazol-4-yl)methoxy)-3,4-dihydroisoquinolin-2(1H)-yl)benzo[d]thiazole-6-carboxylic acid is prepared.

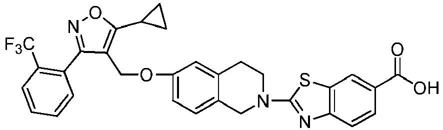

The synthetic method refers to the method of Example 23, and 2-(trifluoromethyl)benzaldehyde is used to replace 2,6-dichlorobenzaldehyde.
Spectrum is: ¹H NMR (400 MHz, CDCl₃) *δ*: 8.41(s, 1H), 8.09(d, *J* = 8.4 Hz, 1H), 7.81(m, 1H), 7.60(m, 3H), 7.48(m, 1H), 7.10(d, *J* = 8.4 Hz, 1H), 6.73(m, 1H), 6.66(m, 1H), 4.78(s, 2H), 4.75(s, 2H), 3.89(m, 2H), 2.98(m, 2H), 2.16(m, 1H), 1.29(t, m, 2H), 1.15(m, 2H).

### Example 44 6-(2-((5-cyclopropyl-3-(2,6-dichlorophenyl)isoxazol-4-yl)methoxy)-6-azaspiro[3.4]octan-6-yl) pyridine-3-carboxylic acid is prepared.

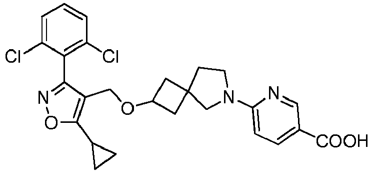

The synthetic method refers to the method of Example 35, and methyl
6-chloropyridine-3-carboxylate is just used to replace methyl 5-chloropyrazine-2-carboxylate.
Spectrum is: ¹H NMR (400 MHz, CDCl₃) *δ*: 8.86(s, 1H), 8.03(m, 1H), 7.43-7.34(m, 3H), 6.27(m, 1H), 4.20(s, 2H), 3.96(m, 1H), 3.45(m, 4H), 2.15(m, 3H), 1.92(m, 2H), 1.79(m, 2H), 1.28(m, 2H), 1.14(m, 2H).

### Example 45 5-(2-((5-cyclopropyl-3-(2,6-dichlorophenyl)isoxazol-4-yl)methoxy)-6-azaspiro[3.4]octan-6-yl) pyridine-3-carboxylic acid is prepared.

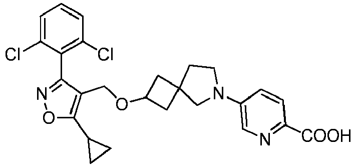

The synthetic method refers to the method of Example 32, and methyl 5-bromopyridine-2-carboxylate is used to replace methyl
1-methyl-6-bromo-1*H*-indole-3-carboxylate.
Spectrum is: ¹H NMR (400 MHz, CDCl₃) *δ*: 8.02(m, 1H), 7.83(s, 1H), 7.45-7.35(m, 3H), 6.81(m, 1H), 4.21(s, 2H), 3.99(m, 1H), 3.36-3.25(m, 4H), 2.16(m, 3H), 1.98(m, 2H), 1.82(m, 2H), 1.28(m, 2H), 1.15(m, 2H).

### Example 46 6-(2-((5-cyclopropyl-3-(2,6-dichlorophenyl)isoxazol-4-yl)methoxy)-6-azaspiro[3.4]octan-6-yl) pyridazine-3-carboxylic acid is prepared.

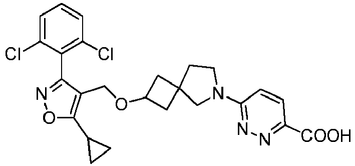

The synthetic method refers to the method of Example 35, and methyl 6-chloropyridazine-3-carboxylate is used to replace methyl 5-chloropyrazine-2-carboxylate.
Spectrum is: ¹H NMR (400 MHz, CDCl₃) *δ*: 7.97(d, J = 10.4 Hz, 1H), 7.43(s, 2H), 7.38(m, 1H), 6.67(m, 1H), 4.20(s, 2H), 3.97(m, 1H), 3.52(m, 4H), 2.15(m, 3H), 1.98(m, 2H), 1.80(m, 2H), 1.28(m, 2H), 1.15(m, 2H).

### Example 47 2-(2-((5-cyclopropyl-3-(2,6-dichlorophenyl)isoxazol-4-yl)methoxy)-6-azaspiro[3.4]octan-6-yl)-N-(methylsulfonyl)benzo[d]thiazole-6-carboxamide is prepared.

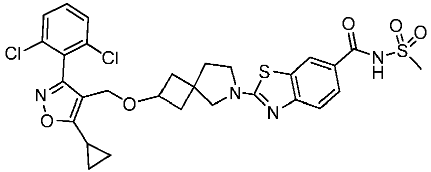

2-(2-((5-cyclopropyl-3-(2,6-dichlorophenyl)isoxazol-4-yl)methoxy)-6-azaspiro[3.4]octan-6-yl) benzo[*d*]thiazole-6-carboxylic acid (50 mg, 0.088 mmol) is dissolved in dichloromethane (5 mL), followed by adding DMAP (19.5 mg, 0.16 mmol) and EDC.HCl (31 mg, 0.16 mmol), and the reaction mixture is stirred overnight at room temperature. Subsequently, the reaction mixture is poured into water (20 mL), and then extracted with dichloromethane (20 mL x 2) and washed with saturated salt solution (20 mL x 2), then dried with anhydrous Na₂SO₄, rotated to dryness and purified by column chromatography (dichloromethane : methanol = 30 : 1), so as to obtain a grey white solid of 34 mg, with a yield of 60%.
Spectrum is: ¹H NMR (400 MHz, CDCl₃) *δ*: 8.19(s, 1H), 7.77(d, J= 8.4 Hz, 1H), 7.55(d, *J=* 8.4 Hz, 1H), 7.47-7.41(m, 2H), 7.38-7.33(m, 1H), 4.18(s, 1H), 3.95(m, 1H), 3.53(m, 4H), 3.40(s, 4H), 2.20-2.14(m, 3H), 1.99(m, 2H), 1.80(m, 2H), 1.26(m, 2H), 1.16(m, 2H).

### Example 48 2-(4-((5-cyclopropyl-3-(2,6-dichlorophenyl)isoxazol-4-yl)methoxy)methyl)piperidin-1-yl)benzo[d ]thiazole-6-carboxylic acid is prepared.

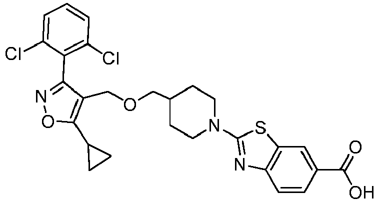

The synthetic method refers to the method of Example 2, and *tert*-butyl 4-(hydroxymethyl) piperidine-1-carboxylate is used to replace *tert-butyl*
2-hydroxy-7-azaspiro[3.5]nonan-7-carboxylate.
Spectrum is: ¹H NMR (400 MHz, CDCl₃) *δ*: 8.37(d, *J* = 1.6 Hz, 1H), 8.06(dd, *J* = 1.6 Hz, 8.4 Hz, 1H), 7.59(d, *J* = 8.8 Hz, 1H), 7.46-7.44(m, 2H), 7.38-7.34(m, 1H), 4.19(s, 2H), 3.33(m, 2H), 2.98(m, 4H), 2.15(m, 1H), 1.85(m, 1H), 1.43(m, 4H), 1.28(m, 2H), 1.15(m, 2H).

Experimental cases FXR agonist activity in vitro for the chemicals prepared in the above examples is measured.

AlphaScreen-based assays are performed with a hexahistidine detection kit manufactured by Perkins-Elmer, for measuring the effect of chemical compounds on the binding between human farnesol receptor protein and its coactivator (SRC1-2), the method is shown as follows:
1) Preparing a mixture comprising human farnesol receptor protein hFXR (100nM, 15 µL/well, peptide bSRC1-2 (50 nM, 15 µL/well),10 × AlphaScreen buffer solution (15µL/well and DI water (60 µL/well), and then transferring 105 µL/well of the mixture to a 96-well plate;
2) Diluting the chemicals prepared in the above examples by twice gradient 12x, with 15 µL/well:
3) Adding donor beads and acceptor beads, 30 µL/well in total; (attention: the beads are light-sensitive)
4) Incubating in dark at room temperature for 2 hr;
5) Transferring 40 µL/well of the mixture to a 384-well plate with 3 replicates, and then centrifuging at 1000 rpm for 1min, to make liquid mixed well underneath the wells.(attention: photopathic operation);
6) In a dark room, reading the plate (by Homogeneous luminescent immunoassay system) to obtain EC₅₀ with a GraphPad Prism5 software.

The results are shown as table 1:

**Table 1 the results of FXR agonist activity**

| Example number | EC₅₀ (nM) |
|---|---|
| Example 1 | C |
| Example 2 | A |
| Example 3 | C |
| Example 4 | A |
| Example 5 | C |
| Example 6 | B |
| Example 7 | A |
| Example 8 | A |
| Example 9 | B |
| Example 10 | B |
| Example 11 | A |
| Example 12 | B |
| Example 13 | B |
| Example 14 | A |
| Example 15 | B |
| Example 16 | B |
| Example 17 | B |
| Example 18 | B |
| Example 19 | B |
| Example 20 | B |
| Example 21 | B |
| Example 22 | A |
| Example 23 | A |
| Example 24 | B |
| Example 25 | B |
| Example 26 | A |
| Example 27 | C |
| Example 28 | A |
| Example 29 | A |
| Example 30 | B |
| Example 31 | A |
| Example 32 | B |
| Example 33 | B |
| Example 34 | B |
| Example 35 | A |
| Example 36 | B |
| Example 37 | B |
| Example 38 | B |
| Example 39 | B |
| Example 40 | A |
| Example 41 | B |
| Example 42 | B |
| Example 43 | A |
| Example 44 | A |
| Example 45 | B |
| Example 46 | A |
| Example 47 | B |
| Example 48 | B |
| GW4064 | B |
| CDCA chenodesoxycholic acid) | C |

Wherein, nM is n mol/L, A represents EC₅₀ < 100 nM, B represents 100 nM < EC₅₀ < 1000 nM, C represents EC₅₀ > 1000 nM, GW4064 is a positive control compound, and its structure is shown as follow:

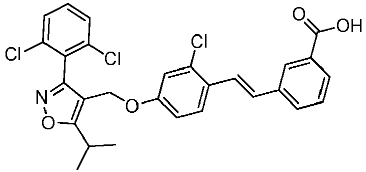

It can be seen from the above results that the chemical compounds prepared in the present disclosure have a better FXR agonist activity, particularly, for some of the compounds, their EC₅₀ for FXR agonist activity reach below 100nM, which show perfect FXR agonist activity.

The technical features of the above described examples may be combined to any combination. For the sake of brevity of description, not all available combinations of the technical features in the above examples are described. However, these technical features should be considered to fall within the scope of the description as long as there is no contradiction between the combinations of the technical features.

The above described examples merely illustrate several examples of the present disclosure, and the description thereof is more specific and detailed, but it cannot be considered as the limiting scope of the disclosure. It should be noted, for one skilled in that art, that a number of variations and modifications may be made without departing from the spirit and scope of the disclosure.

Therefore, the scope of the present disclosure should be determined by the appended claims.

## Claims

1. A modulator of FXR receptor having a structural formula I or a pharmaceutically acceptable salt, stereoisomer, solvate or prodrug thereof: 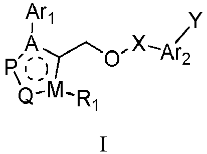 wherein
A is selected from C or N;
P is selected from C, N or O;
Q is selected from C, N or O;
M is selected from C or N;
R₁ is selected from hydrogen, 1 to 5 R₂ substituted or unsubstituted C1∼C6 alkyl, 1 to 5 R₂ substituted or unsubstituted C₂∼C₆ alkenyl, 1 to 5 R₂ substituted or unsubstituted C₁∼C₆ alkynyl, 1 to 5 R₂ substituted or unsubstituted C₃∼C₆ cycloalkyl, 1 to 5 R₂ substituted or unsubstituted heterocyclyl, 1 to 5 R₂ substituted or unsubstituted aryl, or 1 to 5 R₂ substituted or unsubstituted heteroaryl;
Ar₁ is 1 to 5 R₆ substituted or unsubstituted C₅∼C₁₀ aryl, or 1 to 5 R₆ substituted or unsubstituted C₅∼C₁₀ heteroaryl;
X is selected from one of the following groups: 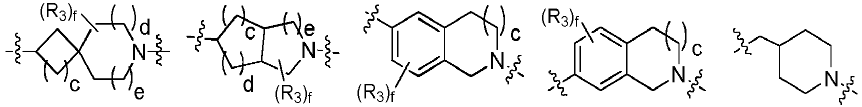 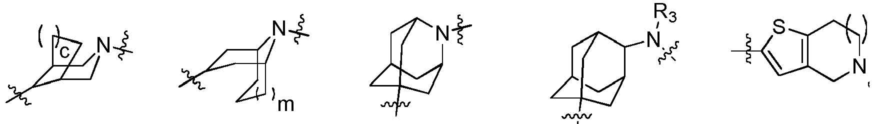 wherein,
c is 0, 1, 2, or 3;
d is 0, 1, 2, or 3;
e is 0, 1, 2, or 3;
f is 0, 1, 2, or 3;
m is 1 or 2;
each R₃ is independently selected from hydrogen, halogen, 1 to 5 R₇ substituted or unsubstituted C₁∼C₃ alkyl, 1 to 5 R₇ substituted or unsubstituted C₁∼C₃ alkoxy, or 1 to 5 R₇ substituted or unsubstituted C3∼C6 cycloalkyl; and two independent substituents R₃ together with the atoms to which they attached can form a 3 to 6-membered carbocyclic, aryl, heteroaryl or heterocyclic ring; Ar₂ is selected from 1 to 3 R₈ substituted or unsubstituted phenyl, 1 to 3 R₈ substituted or unsubstituted naphthyl, or 1 to 3 R₈ substituted or unsubstituted monocyclic or bicyclic C₅∼C₁₀ heteroaryl;
R₈ is selected from hydrogen, halogen, C₁∼C₄ alkyl, C₁∼C₄ alkoxy, C₃∼C₅ cycloalkyl, C₂∼C₄ alkenyl, C₂∼C₄ alkynyl, acylamino, sulfonylamino, ester group, cyano, -OCH₂F, -OCHF₂, -OCF₃, -SCF₃, or -N(CH₃)₂;
Y is selected from COOR₄, CONR₄R₅, C(O)NHSO₂R₄, SO₂NHC(O)R₄, or tetrazole attached to Ar₂ via carbon atom;
each of R₄ and R₅ is independently selected from hydrogen, a metal ion, 1 to 5 R₉ substituted or unsubstituted C₁∼C₆ alkyl, 1 to 5 R₉ substituted or unsubstituted C₂∼C₆ alkenyl, 1 to 5 R₉ substituted or unsubstituted C₂∼C₆ alkynyl, or 1 to 5 R₉ substituted or unsubstituted C₃∼C₆ cycloalkyl; and
each of R₂, R₆, R₇, and R₉ is independently selected from hydrogen, halogen, cyano, acylamino, sulfonylamino, amino, ester group, nitro, C₁∼C₃ alkyl, monohalogenated or polyhalogenated C₁∼C₃ alkyl, C₁∼C₃ alkoxy, monohalogenated or polyhalogenated C₁∼C₃ alkoxy, or 1 to 3 fluorine atoms substituted or unsubstituted C₃∼C₅ cycloalkyl.

2. The modulator of FXR receptor or a pharmaceutically acceptable salt, stereoisomer, solvate or prodrug thereof according to claim 1, wherein
A is C;
P is N;
Q is O; and
M is C.

3. The modulator of FXR receptor or a pharmaceutically acceptable salt, stereoisomer, solvate or prodrug thereof according to claim 1, wherein
Ar₂ is selected from one of the following groups: 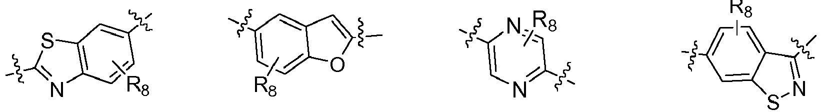 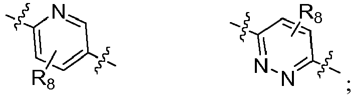 R₈ is selected from hydrogen, halogen, C₁∼C₄ alkyl, or C₁∼C₄ alkoxy.

4. The modulator of FXR receptor or a pharmaceutically acceptable salt, stereoisomer, solvate or prodrug thereof according to any of claims 1-3, wherein X is selected from one of the following groups: 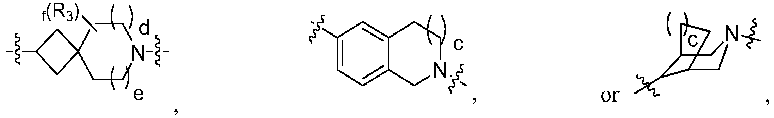 wherein c, d, e, f, and R₃ are as defined in claim 1.

5. The modulator of FXR receptor or a pharmaceutically acceptable salt, stereoisomer, solvate or prodrug thereof according to claim 1, wherein the modulator of FXR receptor is selected from a compound having a structural formula II: 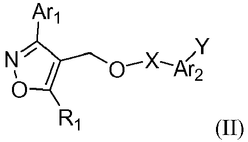 wherein,
R₁ is selected from 1 to 5 R₂ substituted or unsubstituted C₃∼C₆ cycloalkyl;
Ar₁ is selected from 1 to 5 R₆ substituted or unsubstituted C₅∼C₁₀ aryl, and preferably selected from 1 to 3 R₆ substituted or unsubstituted phenyl or 1 to 3 R₆ substituted or unsubstituted pyridyl;
X is selected from one of the following groups: 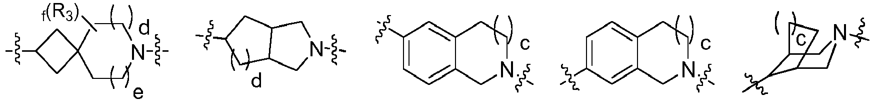 wherein,
c is 0, 1, 2, or 3;
d is 0, 1 or 2;
e is 0 or 1;
f is 0 or 1;
R₃ is selected from hydrogen, halogen, or 1 to 5 R₇ substituted or unsubstituted C₁∼C₃ alkyl;
Ar₂ is selected from one of the following groups: 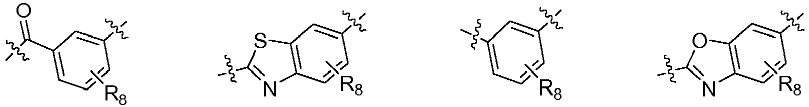 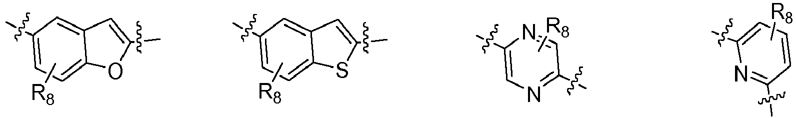 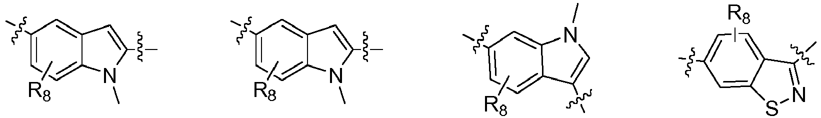 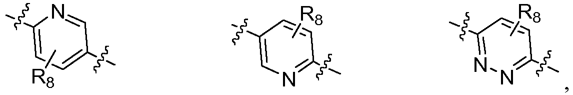 wherein,
R₈ is selected from hydrogen, halogen, C₁∼C₄ alkyl, or C₁∼C₄ alkoxy;
Y is selected from COOR₄ or C(O)NHSO₂R₄;
R₄ is selected from hydrogen, a metal ion, 1 to 5 R₉ substituted or unsubstituted C₁∼C₆ alkyl; and each of R₂, R₆, R₇, and R₉ is independently selected from hydrogen, halogen, cyano, nitro, C₁∼C₃ alkyl, monohalogenated or polyhalogenated C₁∼C₃ alkyl, C₁∼C₃ alkoxy, or monohalogenated or polyhalogenated C₁∼C₃ alkoxy.

6. The modulator of FXR receptor or a pharmaceutically acceptable salt, stereoisomer, solvate or prodrug thereof according to claim 1, wherein
A is C;
P is N;
Q is O;
M is C;
Ar₁ is selected from 1 to 3 R₆ substituted or unsubstituted phenyl;
R₆ is selected from halogen, 1 to 3 fluorine atoms substituted or unsubstituted C₁∼C₃ alkyl, or 1 to 3 fluorine atoms substituted or unsubstituted C₃∼C₅ cycloalkyl;
R₁ is cyclopropyl;
X is selected from: 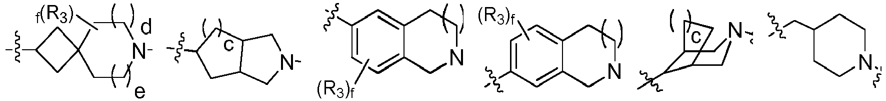 wherein,
c is 0, 1 or 2;
d is 0, 1 or 2;
e is 0, 1 or 2; and
f is 0, 1 or 2.

7. The modulator of FXR receptor or a pharmaceutically acceptable salt, stereoisomer, solvate or prodrug thereof according to claim 1, wherein the modulator is selected from one of the following compounds

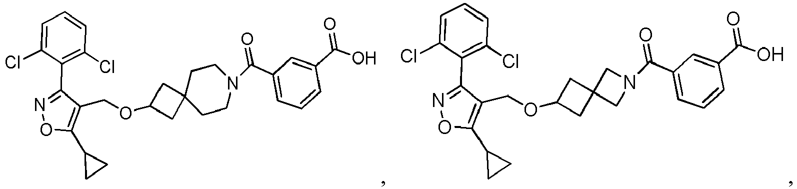

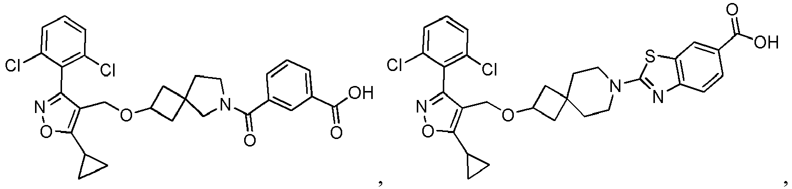

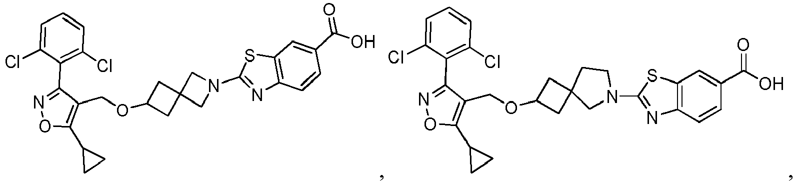

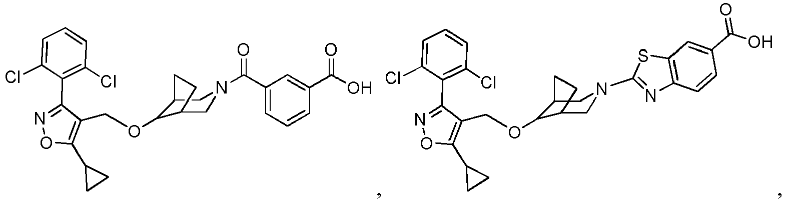

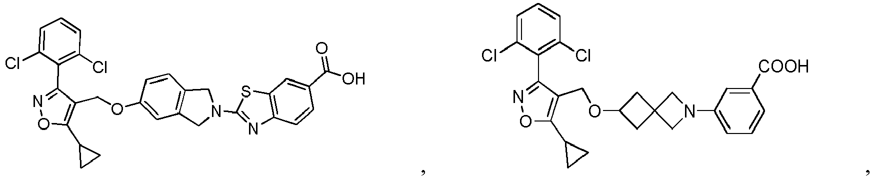

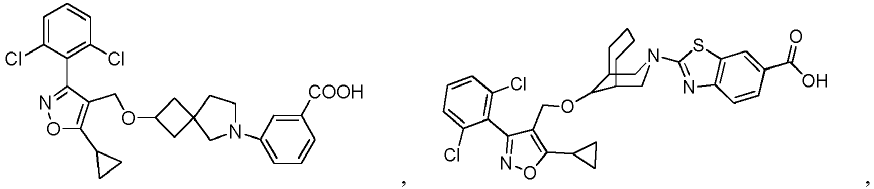

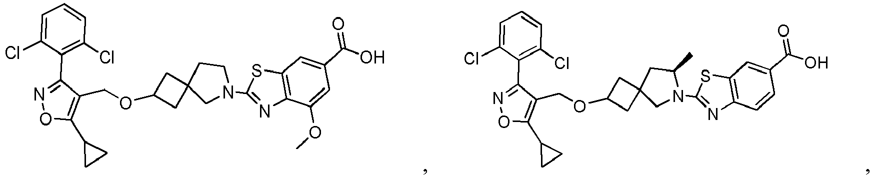

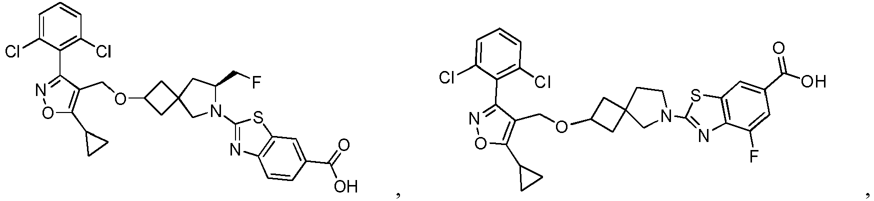

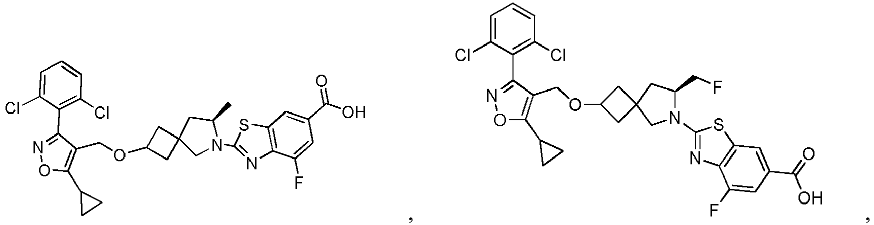

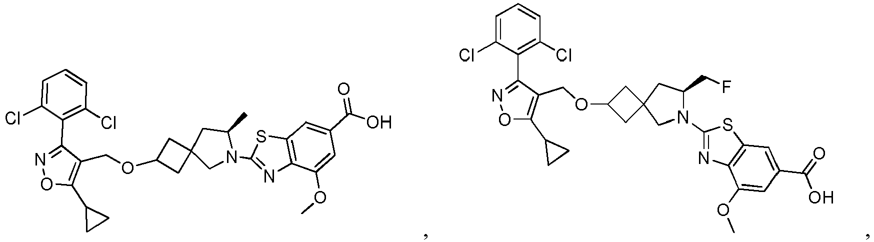

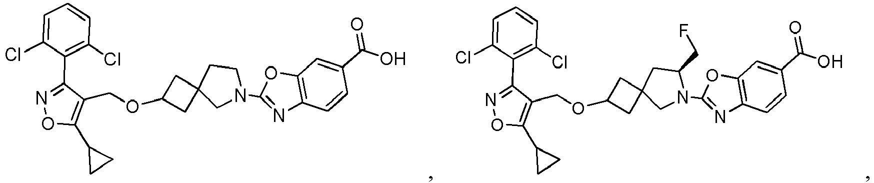

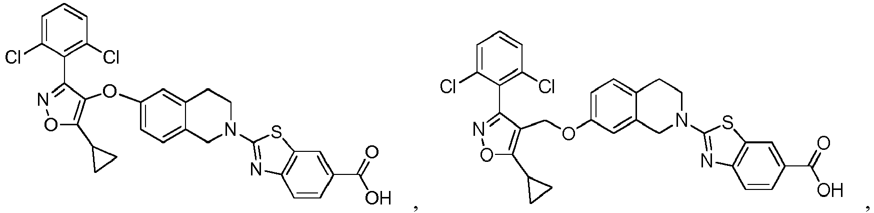

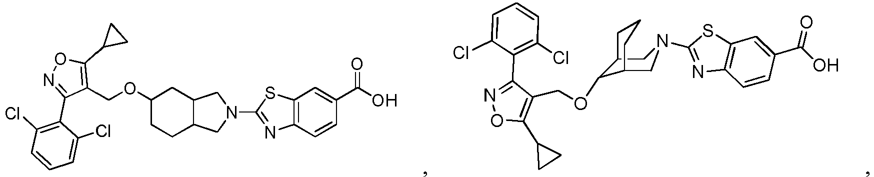

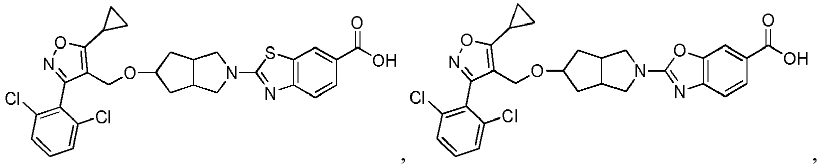

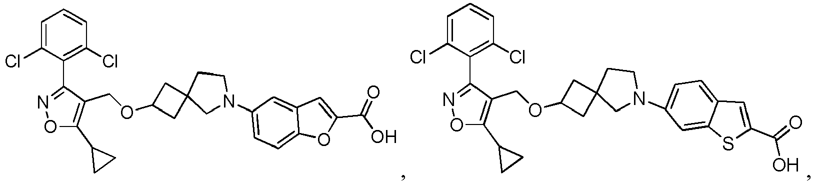

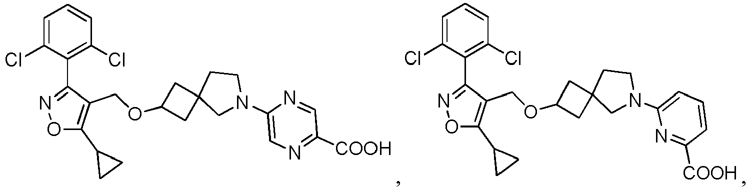

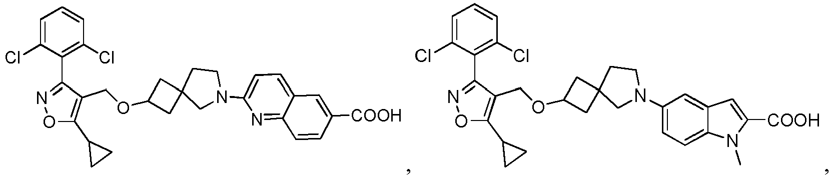

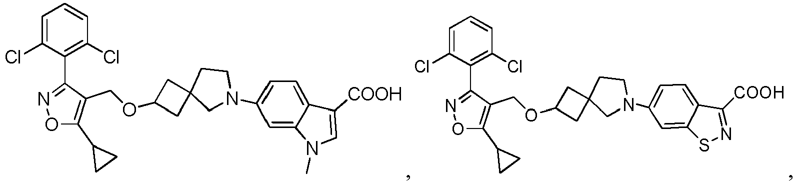

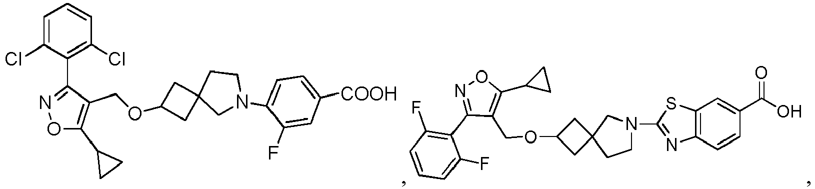

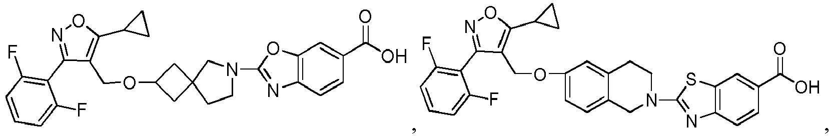

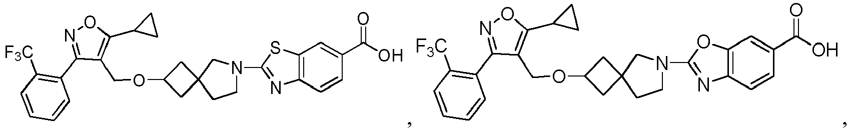

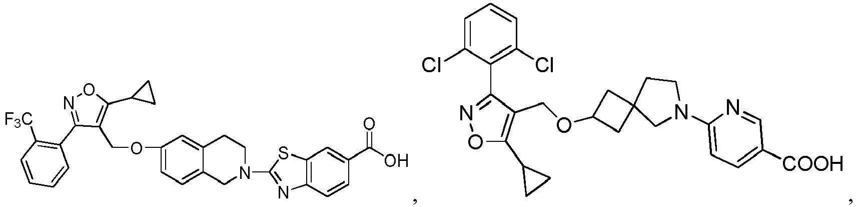

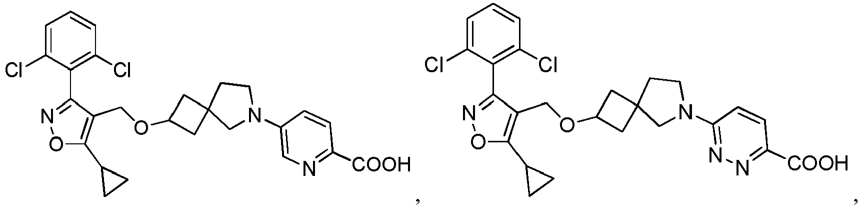

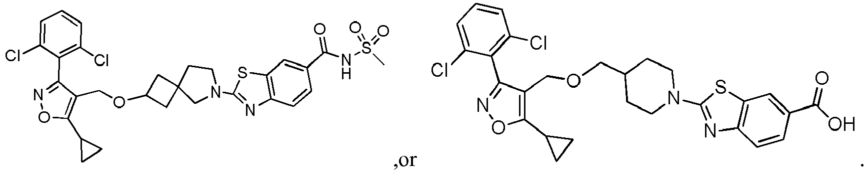

8. A method for preparing the modulator of FXR receptor according to any one of claims 1-7, comprising one of the following synthetic approaches: 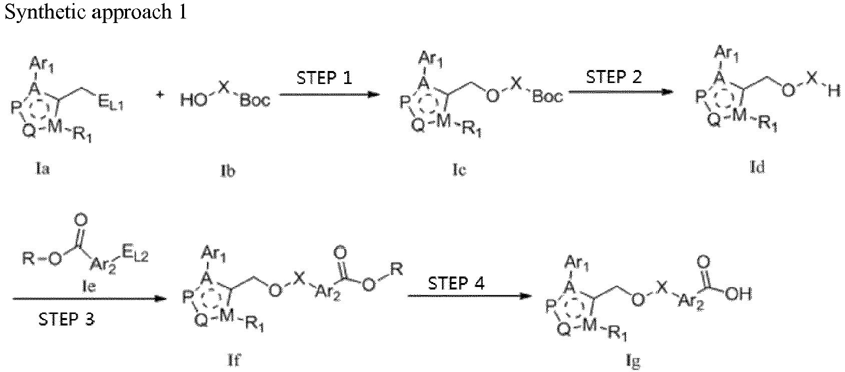 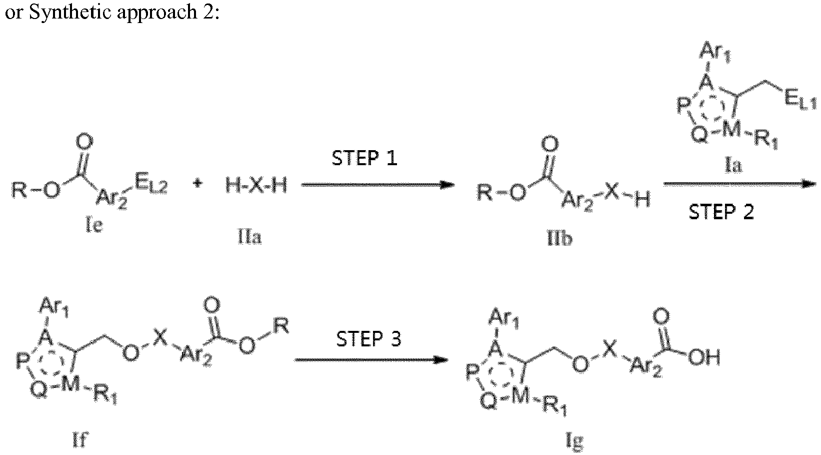 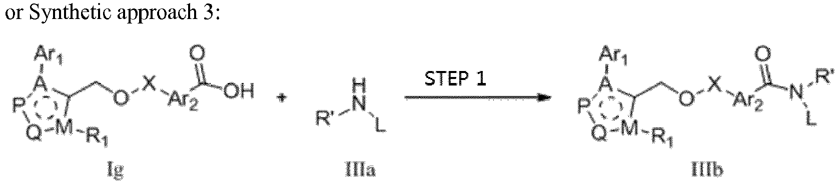 wherein A, M, P, Q, X, R₁, Ar₁ and Ar₂ are as defined in claim 1;
E_{L1} is selected from halogen, alkylsulfonyloxy or arylsulfonyloxy;
E_{L2} is selected from halogen;
R is selected from C₁∼C₆ alkyl;
R' is selected from hydrogen or C₁∼C₆ alkyl;
L is selected from C₁∼C₆ alkyl, C₂∼C₆ alkenyl, C₂∼C₆ alkynyl, C₃∼C₆ cycloalkyl, C(O)R₆ or SO₂R₆; and
R₆ is selected from C₁∼C₃ alkyl, aryl or heteroaryl.

9. Use of the modulator of FXR receptor or a pharmaceutically acceptable salt, stereoisomer, solvate or prodrug thereof according to any one of claims 1-7 in the preparation of a medicament for preventing and treating a disease mediated by FXR.

10. The use according to claim 8, wherein the disease mediated by FXR comprises non-alcoholic fatty liver disease, non-alcoholic steatohepatitis, chronic intrahepatic or extrahepatic cholestasis , hepatic fibrosis caused by chronic cholestasis or acute intrahepatic cholestasis, chronic hepatitis B, gallstone, hepatic carcinoma, colon cancer, or intestinal inflammatory disease.

11. A pharmaceutical composition, comprising the modulator of FXR receptor or a pharmaceutically acceptable salt, stereoisomer, solvate or prodrug thereof according to any claims 1-7, and a pharmaceutically acceptable excipient or carrier.
